# EUROPÄISCHE PATENTSCHRIFT

(11) **EP 1 028 949 B1**
(45) Veröffentlichungstag und Bekanntmachung des Hinweises auf die Patenterteilung: **23.03.2005**
(21) Anmeldenummer: 98955485.2
(22) Anmeldetag: 20.10.1998
(51) Int. Cl.: C07D 239/52, A01N 47/36, C07D 239/47, C07D 239/42, C07D 239/46, C07D 251/46, C07D 251/16, C07D 251/22, C07D 251/52, C07D 239/56, C07D 275/06, C07C 311/15, C07C 311/54, C07C 309/86

(54) **CARBAMOYLPHENYLSULFONYLHARNSTOFFE, VERFAHREN ZU IHRER HERSTELLUNG UND IHRE VERWENDUNG ALS HERBIZIDE UND PFLANZENWACHSTUMSREGULATOREN**
CARBAMOYLPHENYLSULFONYL UREAS, METHOD FOR THEIR PRODUCTION AND THEIR APPLICATION AS HERBICIDES AND PLANT GROWTH REGULATORS
UREES CARBAMOYLPHENYLSULFONIQUES, LEUR PROCEDE DE PRODUCTION ET LEUR UTILISATION COMME HERBICIDES ET REGULATEURS DE LA CROISSANCE DES PLANTES

(30) Priorität: 03.11.1997 DE 19748470
(43) Veröffentlichungstag der Anmeldung: 23.08.2000
(73) Patentinhaber: Bayer CropScience GmbH, 65929 Frankfurt/Main (DE)
(72) Erfinder: KEHNE, Heinz, D-65719 Hofheim (DE); WILLMS, Lothar, D-65719 Hofheim (DE); WALDRAFF, Christian, D-60318 Frankfurt (DE); DIETRICH, Hansjörg, D-65830 Kriftel (DE); BIERINGER, Hermann, D-65817 Eppstein (DE); ROSINGER, Christopher, D-65719 Hofheim (DE); AULER, Thomas, 65812 Bad Soden (DE)
(86) Internationale Anmeldenummer: PCT/EP1998/006623
(87) Internationale Veröffentlichungsnummer: WO 1999/023079

(56) Entgegenhaltungen:
- WO-A-95/20963
- US-A- 4 302 241
- KATOGHI TETRAHEDRON Bd. 55, 1999, Seiten 14885 - 14900

## Beschreibung

Es ist bekannt, daß Phenylsulfonylharnstoffe, die mit Carbamoylgruppen substituiert sind, herbizide Eigenschaften besitzen. Dabei handelt es sich um symmetrische Derivate der Isophthalsäure (allgemeine Formel A; US-PS 4 302 241).

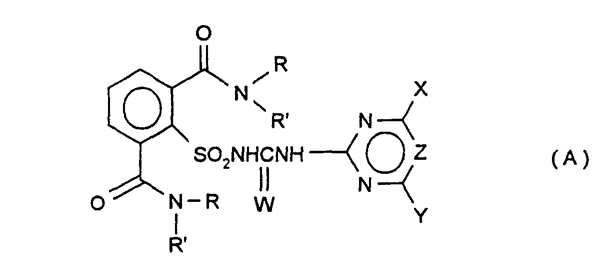

Überraschenderweise wurden nun Terephthalsäureesteramide bzw. -diamide gefunden, die sich besonders gut als Herbizide oder Pflanzenwachstumsregulatoren eignen.

Gegenstand der vorliegenden Erfindung sind Verbindungen der Formel (I) oder deren Salze, 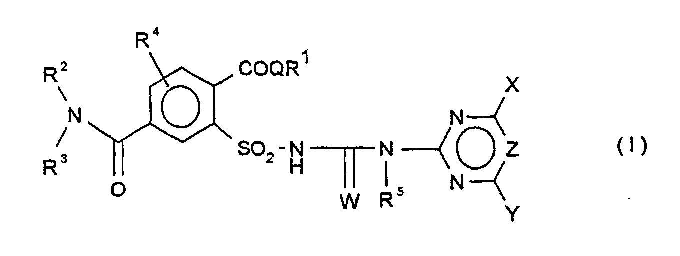 worin
- R¹: ein Wasserstoffatom, einen Kohlenwasserstoffrest oder einen Heterocyclylrest, wobei jeder der letztgenannten beiden Reste unsubstituiert oder substituiert ist und inklusive Substituenten 1 bis 30 C-Atome, vorzugsweise 1 bis 20 C-Atome aufweist,
- R²: eine Gruppe der Formel R⁰-Q⁰-, worin
R⁰ ein Wasserstoffatom, einen Kohlenwasserstoffrest oder einen Heterocyclylrest, wobei jeder der letztgenannten beiden Reste unsubstituiert oder substituiert ist und inklusive Substituenten 1 bis 30 C-Atome, vorzugsweise 1 bis 20 C-Atome aufweist, bedeutet und
Q⁰ eine direkte Bindung oder eine divalente Gruppe der Formel -O-, -SO₂-, -NH-, -N[(C₁-C₆)Alkyl]-, -CO-, -CO-NH- oder -O-CO-NH- bedeutet,
- R³: ein Wasserstoffatom, einen Kohlenwasserstoffrest oder einen Heterocyclylrest, wobei jeder der letztgenannten beiden Reste unsubstituiert oder substituiert ist und inklusive Substituenten 1 bis 30 C-Atome, vorzugsweise 1 bis 20 C-Atome aufweist,
- R² und R³: gemeinsam mit dem N-Atom einen Heterocyclus von 3-6 Ringatomen, der gesättigt oder ungesättigt ist, zusätzlich zum N-Atom ein oder zwei Atome aus der Gruppe N, O und S enthalten kann und unsubstituiert oder durch einen oder mehrere Reste aus der Gruppe (C₁-C₆)Alkyl, (C₁-C₆)Alkoxy, Halogen, [(C₁-C₆)Alkoxy]carbonyl, (C₁-C₆)Haloalkyl und Oxo substituiert ist,
- R⁴: H, Halogen, NO₂, CN, (C₁-C₄)Alkyl, (C₁-C₄)Alkoxy, [(C₁-C₄)Alkyl]-carbonyl oder [(C₁-C₄)Alkoxy]carbonyl, wobei jeder der letztgenannten vier Reste unsubstituiert oder im Alkylteil durch ein oder mehrere Halogenatome substituiert ist,
- R⁵: H oder (C₁-C₄)Alkyl, vorzugsweise H oder CH₃,
- Q: O oder NR*,
- R*: H, (C₁-C₄)Alkyl, (C₃-C₄)Alkenyl oder (C₃-C₄)Alkinyl, wobei jeder der drei letztgenannten Reste unsubstituiert oder durch einen oder mehrere Reste aus der Gruppe Halogen, (C₁-C₄)Alkoxy und (C₁-C₄)Alkylthio substituiert ist,
- W ein: Sauerstoff- oder Schwefelatom,
- X,Y: unabhängig voneinander H, Halogen, (C₁-C₄)Alkyl, (C₁-C₄)Alkoxy, (C₁-C₄)-Alkylthio, wobei jeder der letztgenannten 3 Reste unsubstituiert oder durch einen oder mehrere Reste aus der Gruppe Halogen, (C₁-C₄)Alkoxy und (C₁-C₄)Alkylthio substituiert ist, oder Mono- oder Di[((C₁-C₄)alkyl]-amino, (C₃-C₄)-Cycloalkyl, (C₂-C₅)Alkenyl, (C₂-C₅)Alkinyl, (C₂-C₅)Alkenyloxy oder (C₂-C₅)-Alkinyloxy und
- Z: CH oder N bedeuten.

Die Verbindungen der Formel (I) können Salze bilden, bei denen der Wasserstoff der -SO₂-NH-Gruppe durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze oder Salze mit organischen Aminen. Ebenso kann Salzbildung durch Anlagerung einer Säure an basischen Gruppen, wie z.B. Amino und Alkylamino, erfolgen. Geeignete Säuren hierfür sind starke anorganische und organische Säuren, beispielsweise HCl, HBr, H₂SO₄ oder HNO₃.

In Formel (I) und allen nachfolgenden Formeln können die kohlenstoffhaltigen Reste wie Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Alkenyl in der Form "(C₃-C₄)Alkenyl" oder "(C₃-C₆)Alkenyl" bedeutet vorzugsweise einen Alkenylrest mit 3 bis 4 bzw. 3 bis 6 C-Atomen, bei dem die Doppelbindung nicht an dem C-Atom liegt, das mit dem übrigen Molekülteil der Verbindung (I) verbunden ist ("yl"-Position). Entsprechendes gilt für (C₃-C₄)Alkinyl usw.

Cycloalkyl bedeutet ein carbocyclisches, gesättigtes Ringsystem mit 3-8 C-Atomen, z.B. Cyclopropyl, Cyclobutyl, Cyclopentyl oder Cyclohexyl.

Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein Kohlenwasserstoffrest ist ein geradkettiger, verzweigter oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl; Aryl bedeutet dabei ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl; vorzugsweise bedeutet ein Kohlenwasserstoffrest Alkyl, Alkenyl oder Alkinyl mit bis zu 12 C-Atomen oder Cycloalkyl mit 3, 4, 5, 6 oder 7 Ringatomen oder Phenyl.

Ein heterocyclischer Rest oder Ring (Heterocyclyl) kann gesättigt, ungesättigt oder heteroaromatisch sein; er enthält vorzugsweise ein oder mehrere Heteroatome im Ring, vorzugsweise aus der Gruppe N, O und S; vorzugsweise ist er ein aliphatischer Heterocyclylrest mit 3 bis 7 Ringatomen oder ein heteroaromatischer Rest mit 5 oder 6 Ringatomen und enthält 1, 2 oder 3 Heteroatome. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Triazinyl, Thienyl, Thiazolyl, Oxazolyl, Furyl, Pyrrolyl, Pyrazolyl und Imidazolyl, oder ist ein partiell oder vollständig hydrierter Rest wie Oxiranyl, Oxetanyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substituiertes Alkyl, Alkenyl, Alkinyl, Aryl, Phenyl und Benzyl, oder substituiertes Heterocyclyl oder Heteroaryl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy und Chlor. Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl. Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Ein Acylrest bedeutet den Rest einer organischen Säure, z.B. den Rest einer Carbonsäure und Reste davon abgeleiteter Säuren wie der Thiocarbonsäure, gegebenenfalls N-substituierten Iminocarbonsäuren oder den Rest von Kohlensäuremonoestern, gegebenenfalls N-substituierter Carbaminsäure, Sulfonsäuren, Sulfinsäuren, Phosphonsäuren, Phosphinsäuren. Acyl bedeutet beispielsweise Formyl, Alkylcarbonyl wie (C₁-C₄-Alkyl)-carbonyl, Phenylcarbonyl, wobei der Phenylring substituiert sein kann, z.B. wie oben für Phenyl gezeigt, oder Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die vorstehenden Beispiele für Reste oder Restebereiche, die unter die allgemeinen Begriffe wie "Alkyl", "Acyl", "substituierten Reste" etc., fallen, bedeuten keine vollständige Aufzählung. Die allgemeinen Begriffe umfassen auch die weiter unten angeführten Definitionen für Restebereiche in Gruppen bevorzugter Verbindungen, insbesondere Restebereiche, welche spezifische Reste aus den Tabellenbeispielen umfassen.

Vor allem aus Gründen der höheren herbiziden Wirkung, besseren Selektivität und/oder besseren Herstellbarkeit sind erfindungsgemäße Verbindungen der Formel (I) oder deren Salze von besonderem Interesse, worin
- R¹: H, (C₁-C₆)Alkyl, (C₃-C₆)Alkenyl, (C₃-C₆)Alkinyl, wobei jeder der drei letztgenannten Reste unsubstituiert oder durch einen oder mehrere Reste aus der Gruppe Halogen, unsubstituiertes und substituiertes Phenyl, unsubstituiertes und substituiertes Heterocyclyl mit 3 bis 6 Ringatomen, unsubstituiertes und substituiertes (C₃-C₆)Cycloalkyl, (C₁-C₄)Alkoxy, (C₁-C₄)Alkylthio, [(C₁-C₄)Alkoxy]-carbonyl und [(C₁-C₄)Haloalkoxy]-carbonyl, substituiert ist, oder unsubstituiertes oder substituiertes (C₃-C₆)Cycloalkyl, unsubstituiertes oder substituiertes Phenyl, unsubstituiertes oder substituiertes Heterocyclyl mit 3 bis 6 Ringatomen und/oder
- R²: eine Gruppe der Formel R⁰-Q⁰-,
worin R⁰ ein Wasserstoffatom, (C₁-C₁₂)Alkyl, (C₃-C₁₂)Alkenyl oder (C₃-C₁₂)Alkinyl, wobei jeder der letztgenannten 3 Reste unsubstituiert oder durch einen oder mehrere Reste aus der Gruppe Halogen, (C₁-C₆)Alkoxy, (C₁-C₆)Haloalkoxy, (C₁-C₆)Alkylthio, (C₁-C₆)-Haloalkylthio, (C₁-C₆)Alkylsulfinyl, (C₁-C₆)Haloalkylsulfinyl, (C₁-C₆)Alkylsulfonyl, (C₁-C₆)Haloalkylsulfonyl, [(C₁-C₆)Alkoxy]carbonyl, [(C₁-C₆)Haloalkoxy]carbonyl, CONR⁶R⁷, SO₂NR⁶R⁷, CN, OH, (C₃-C₆)Cycloalkyl, NR⁸R⁹, unsubstituiertes Phenyl, substituiertes Phenyl, unsubstituiertes Heterocyclyl und substituiertes Heterocyclyl substituiert ist, oder
unsubstituiertes oder substituiertes (C₃-C₆)Cycloalkyl, unsubstituiertes oder substituiertes (C₃-C₆)Cycloalkenyl, unsubstituiertes oder substituiertes Heterocyclyl oder unsubstituiertes oder substituiertes Phenyl bedeutet und worin Q⁰ eine direkte Bindung oder eine divalente Gruppe der Formel -O-, -- SO₂-, -NH-, -N[(C₁-C₆)Alkyl]-, -CO-, -CO-NH- oder -O-CO-NH- bedeutet, und
R³ unabhängig voneinander wie R⁰ im Rest R² definiert ist, oder
R² undR³ gemeinsam mit dem N-Atom einen Heterocyclus von 3-6 Ringatomen, der gesättigt oder ungesättigt ist, zusätzlich zum N-Atom ein oder zwei Atome aus der Gruppe N, O und S enthalten kann und unsubstituiert oder durch einen oder mehrere Reste aus der Gruppe (C₁-C₆)Alkyl, (C₁-C₆)Alkoxy, Halogen, [(C₁-C₆)Alkoxy]carbonyl, (C₁-C₆)Haloalkyl und Oxo substituiert ist, und/oder
R⁶ und R⁷ unabhängig voneinander H, (C₁-C₆)Alkyl, (C₃-C₆)Alkenyl, (C₃-C₆)Alkinyl oder unsubstituiertes oder substituiertes Phenyl oder
R⁶ und R⁷ gemeinsam mit dem N-Atom einen heterocyclischen Ring mit 5 oder 6 Ringgliedern, der gegebenenfalls weitere Heteroatome aus der Gruppe N, O und S enthalten kann und unsubstituiert oder ein- oder mehrfach durch Reste aus der Gruppe (C₁-C₄)Alkyl und Oxo substituiert ist, und/oder
R⁸ und R⁹ unabhängig voneinander und unabhängig von R⁶ und R⁷ wie unter R⁶ und R⁷ definiert oder (C₁-C₄)Alkylcarbonyl, (C₁-C₄)Haloalkylcarbonyl, (C₁-C₄)-Alkoxycarbonyl oder (C₁-C₄)Alkylsulfonyl,
Q O oder NR*, wobei R* wie oben definiert ist, und/oder
X und Y unabhängig voneinander H, Halogen, (C₁-C₄)Alkyl, (C₁-C₄)Alkoxy, (C₁-C₄)Alkylthio, wobei jeder der drei letztgenannten Reste unsubstituiert oder durch einen oder mehrere Reste aus der Gruppe Halogen, (C₁-C₃)Alkoxy und (C₁-C₄)Alkylthio substituiert ist, Mono- oder Di[(C₁-C₄)alkyl]amino, (C₃-C₆)-Cycloalkyl, (C₃-C₅)Alkenyl, (C₃-C₅)Alkenyloxy oder (C₃-C₅)Alkinyloxy und
Z CH oder N bedeuten, wobei
substituiertes Phenyl, substituiertes Heterocyclyl, substituiertes Cycloalkyl oder substituiertes Cycloalkenyl als Substituenten vorzugsweise einen oder mehrere Reste aus der Gruppe Halogen, (C₁-C₄)Alkyl, (C₁-C₄)Haloalkyl, (C₁-C₄)Alkoxy-(C₁-C₄)Alkyl, Di-[(C₁-C₄)Alkoxy]-(C₁-C₄)Alkyl, (C₁-C₄)Haloalkoxy, (C₁-C₄)Alkylthio, (C₁-C₄)Alkylsulfinyl, (C₁-C₄)Haloalkylsulfinyl, (C₁-C₄)Alkylsulfonyl, (C₁-C₄)Haloalkylsulfonyl, NR⁸R⁹, [(C₁-C₄)Alkoxy]carbonyl, [(C₁-C₄)Haloalkoxy]carbonyl, [(C₁-C₄)Alkyl]carbonyl, OH, Phenyl, CN und NO₂ - trägt und
wobei jeder der Reste R¹, R² und R³ inklusive Substituenten 1 bis 20 C-Atome, vorzugsweise 1 bis 16 C-Atome aufweist.

Von besonderem Interesse sind erfindungsgemäße Verbindungen der Formel (I),
worin
- R¹: H, (C₁-C₆)Alkyl, (C₃-C₆)Alkenyl oder (C₃-C₆)Alkinyl, wobei jeder der drei letztgenannten Reste unsubstituiert oder durch einen oder mehrere Reste aus der Gruppe Halogen, Phenyl, (C₁-C₄)Alkoxy, (C₁-C₄)Alkylthio und [(C₁-C₄)Alkoxy]-carbonyl substituiert ist, oder (C₃-C₆)Cycloalkyl, (C₃-C₆)Cycloalkyl(C₁-C₃)alkyl, Heterocyclyl mit 3 bis 6 Ringatomen oder Heterocyclyl-(C₁-C₃)alkyl mit 3 bis 6 Ringatomen, wobei jeder der letztgenannten 4 Reste unsubstituiert oder durch einen oder mehrere Reste aus der Gruppe Halogen, (C₁-C₄)Alkyl und (C₁-C₄)Alkoxy substituiert ist, und/oder
- R²: eine Gruppe der Formel R⁰-Q⁰-, worin
R⁰ ein Wasserstoffatom, (C₁-C₈)Alkyl, (C₃-C₈)Alkenyl oder (C₃-C₈)Alkinyl, wobei jeder der letztgenannten 3 Reste unsubstituiert oder durch einen oder mehrere Reste aus der Gruppe Halogen (C₁-C₄)Alkoxy, (C₁-C₄)Haloalkoxy, (C₁-C₄)Alkylthio, (C₁-C₄)-Haloalkylthio, (C₁-C₄)Alkylsulfinyl, (C₁-C₄)Haloalkylsulfinyl, (C₁-C₄)Alkylsulfonyl, (C₁-C₄)Haloalkylsulfonyl, [(C₁-C₆)Alkoxy]carbonyl, CONR⁶R⁷, SO₂NR⁶R⁷, CN, OH, (C₃-C₆)Cycloalkyl, NR⁸R⁹, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsufonyl, NR⁸R⁹, [(C₁-C₄)Alkoxy]carbonyl, [(C₁-C₄)Alkyl]carbonyl, Phenyl, [(C₁-C₄)Alkyl]-carbonyl, CN und NO₂ substituiert ist, und Heterocyclyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsufonyl, NR⁸R⁹, [(C₁-C₄)Alkoxy]carbonyl, [(C₁-C₄)Alkyl]carbonyl, Phenyl, [(C₁-C₄)Alkyl]-carbonyl, CN und NO₂ substituiert ist, substituiert ist, oder
(C₃-C₆)Cycloalkyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)-Haloalkoxy, [(C₁-C₄)Alkoxy]carbonyl, CN, OH und Phenyl substituiert ist, oder (C₃-C₆)Cycloalkenyl, das unsubstituiert oder durch einen oder mehrere Reste aus der Gruppe Halogen, (C₁-C₄)Alkyl, (C₁-C₄)Haloalkyl, (C₁-C₄)Alkoxy und [(C₁-C₄)Alkoxy]carbonyl substituiert ist, oder
Heterocyclyl oder Phenyl, wobei jeder der letztgenannten 2 Reste unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsufonyl, NR⁸R⁹, [(C₁-C₄)Alkoxy]carbonyl, [(C₁-C₄)Alkyl]carbonyl, Phenyl, [(C₁-C₄)Alkyl]-carbonyl, CN und NO₂ substituiert ist, und
Q⁰ eine direkte Bindung oder eine divalente Gruppe der Formel -O-, -SO₂-, -NH-, -CO-NH- oder -O-CO-NH-, vorzugsweise eine direkte Bindung oder -O-, -SO₂- oder -NH- bedeutet, und
- R³: unabhängig voneinander wie R⁰ im Rest R² definiert ist, oder
- R² und R³: gemeinsam mit dem N-Atom einen Heterocyclus von 3-6 Ringatomen, der gesättigt oder ungesättigt ist und zusätzlich zum N-Atom ein oder zwei Heteroatome aus der Gruppe N, O und S enthalten kann und unsubstituiert oder durch einen oder mehrere Reste aus der Gruppe (C₁-C₃)Alkyl, (C₁-C₃)Alkoxy, Halogen, [(C₁-C₃)Alkoxy]carbonyl, (C₁-C₃)Haloalkyl und Oxo substituiert ist, und/oder
- R⁶ und R⁷: unabhängig voneinander H, (C₁-C₄)Alkyl, (C₃-C₄)Alkenyl, (C₃-C₄)Alkinyl oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfonyl, [(C₁-C₄)Alkoxy]carbonyl, CN und NO₂ substituiert ist, vorzugsweise H oder (C₁-C₄)Alkyl, oder
- R⁶ und R⁷: gemeinsam mit dem N-Atom einen heterocyclischen Ring mit 5 oder 6 Ringgliedern, der gegebenenfalls weitere Heteroatome aus der Gruppe N, O und S enthalten kann und unsubstituiert oder ein- oder mehrfach durch Reste aus der Gruppe (C₁-C₄)Alkyl und Oxo substituiert ist,
vorzugsweise gemeinsam mit dem N-Atom einen heterocyclischen Ring mit 5 oder 6 Ringgliedern, der gegebenenfalls ein weiteres Heteroatom aus der Gruppe N und O enthalten kann und unsubstituiert oder ein- oder mehrfach durch Reste aus der Gruppe (C₁-C₄)Alkyl und Oxo substituiert ist, und/oder
- R⁸ und R⁹: unabhängig voneinander unabhängig von R⁶ und R⁷ wie unter R⁶ und R⁷ definiert ist oder (C₁-C₄)Alkylcarbonyl, (C₁-C₄)Haloalkylcarbonyl, (C₁-C₄)-Alkoxycarbonyl oder (C₁-C₄)Alkylsulfonyl, und
- Q: O oder NR*, wobei R* wie weiter oben definiert ist, und/oder
- X und Y: unabhängig voneinander H, Halogen, (C₁-C₄)Alkyl, (C₁-C₄)Alkoxy, (C₁-C₄)Alkylthio, wobei jeder der drei letztgenannten Reste unsubstituiert oder durch einen oder mehrere Reste aus der Gruppe Halogen, (C₁-C₃)Alkoxy und (C₁-C₄)Alkylthio substituiert ist, Mono- oder Di[(C₁-C₄)alkyl]amino, (C₃-C₆)Cycloalkyl, (C₃-C₅)Alkenyl, (C₃-C₅)Alkenyloxy oder (C₃-C₅)Alkinyloxy und
- Z: CH oder N bedeuten.

Von besonderem Interesse sind auch erfindungsgemäße Verbindungen der Formel (I) und deren Salze, worin
- R¹: (C₁-C₆)Alkyl, das unsubstituiert oder durch einen oder mehrere Reste aus der Gruppe Halogen und (C₁-C₄)Alkoxy substituiert ist, oder 3-Oxetanyl, (C₃-C₄)Alkenyl oder (C₃-C₄)Alkinyl und/oder
- R²: H, (C₁-C₆)Alkyl, (C₃-C₆)Alkenyl, (C₃-C₆)Alkinyl, wobei jeder der letztgenannten drei Reste unsubstituiert oder durch einen oder mehrere Reste aus der Gruppe Halogen, (C₁-C₄)Alkoxy, (C₁-C₄)Alkylthio, (C₁-C₄)Alkylsulfonyl,
[(C₁-C₄)Alkoxy]carbonyl, (C₃-C₆)Cycloalkyl, CN und OH substituiert ist, oder (C₃-C₆)Cycloalkyl, das unsubstituiert oder durch einen oder mehrere Reste aus der Gruppe Halogen, (C₁-C₄)Alkyl, (C₁-C₄)Alkoxy, [(C₁-C₄)Alkoxy]carbonyl, CN und OH substituiert ist, oder (C₃-C₆)Cycloalkenyl, oder (C₁-C₄)Alkoxy, (C₁-C₄)Alkenyloxy, (C₁-C₄)Alkylsulfonyl, (C₁-C₄)Alkylamino oder Di[(C₁-C₄)Alkyl]amino und
- R³-: H, (C₁-C₆)Alkyl, (C₃-C₆)Alkenyl, (C₃-C₆)Alkinyl, wobei jeder der letztgenannten drei Reste unsubstituiert oder durch einen oder mehrere Reste aus der Gruppe Halogen, (C₁-C₄)Alkoxy, (C₁-C₄)Alkylthio, (C₁-C₄)Alkylsulfonyl, [(C₁-C₄)Alkoxy]carbonyl, (C₃-C₆)Cycloalkyl, CN und OH substituiert ist, oder (C₃-C₆)Cycloalkyl, das unsubstituiert oder durch einen oder mehrere Reste aus der Gruppe Halogen, (C₁-C₄)Alkyl, (C₁-C₄)Alkoxy, [(C₁-C₄)Alkoxy]carbonyl, CN und OH substituiert ist, oder (C₃-C₆)Cycloalkenyl oder
- R² und R³: gemeinsam mit dem N-Atom einen Heterocyclus von 3-6 Ringatomen, der gesättigt oder ungesättigt ist, zusätzlich zu dem N-Atom ein oder zwei Atome aus der Gruppe N, O und S enthalten kann und unsubstituiert oder durch einen oder mehrere Reste aus der Gruppe Halogen, (C₁-C₃)Alkyl, (C₁-C₃)Alkoxy, Oxo und [(C₁-C₃)Alkoxy]carbonyl substituiert ist, und/oder
- R⁴: H, (C₁-C₄)Alkyl, (C₁-C₄)Haloalkyl, (C₁-C₄)Alkoxy oder Halogen und/oder
- R⁵: H oder Methyl und/oder
- R*: H oder (C₁-C₄)Alkyl und/oder
- X und Y: unabhängig voneinander (C₁-C₄)Alkyl, (C₁-C₄)Alkoxy, wobei jeder der letztgenannten zwei Reste unsubstituiert oder durch einen oder mehrere Halogenatome substituiert ist, oder (C₁-C₄)Alkylthio, Halogen oder Mono- oder Di[(C₁-C₂)alkyl]amino und/oder
- W: ein Sauerstoffatom bedeuten.

Bevorzugte erfindungsgemäße Verbindungen der Formel (I) oder deren Salze sind solche, worin
- R² und R³: unabhängig voneinander H, (C₁-C₄)Alkyl, (C₁-C₃)Alkenyl, (C₁-C₃)Alkinyl, (C₁-C₃)Cycloalkyl oder (C₃-C₆)Cycloalkenyl und/oder
- R⁴: H, (C₁-C₃)Alkyl oder Halogen und/oder
- R*: (C₁-C₃)Alkyl, und/oder
- X: (C₁-C₂)Alkyl, (C₁-C₂)Alkoxy, (C₁-C₂)Alkylthio, (C₁-C₂)Haloalkyl oder (C₁-C₂)-Haloalkoxy und
- Y: (C₁-C₂)Alkyl, (C₁-C₂)Alkoxy, Halogen, NHCH₃ oder N(CH₃)₂ bedeuten.

Besonders bevorzugte erfindungsgemäße Verbindungen der Formel (I) oder deren Salze sind solche, worin
- R¹: (C₁-C₃)Alkyl, Allyl oder Propargyl und/oder
- R⁴: H und/oder
- Q: ein Sauerstoffatom bedeuten.

Besonders bevorzugt sind auch erfindungsgemäße Verbindungen der Formel (I) und deren Salze, welche eine Kombination von Resten aus den obengenannten Verbindungen von besonderem Interesse bzw. den bevorzugten Verbindungen enthalten, sowie solche, welche einzelne oder mehrere Reste aus den in den Tabellen 1 und 2 (s. u.) aufgeführten Verbindungen enthalten.

Gegenstand der vorliegenden Erfindung sind auch Verfahren zur Herstellung der erfindungsgemäßen Verbindungen der allgemeinen Formel (I) oder deren Salze, dadurch gekennzeichnet, daß man
a) eine Verbindung der Formel (II) 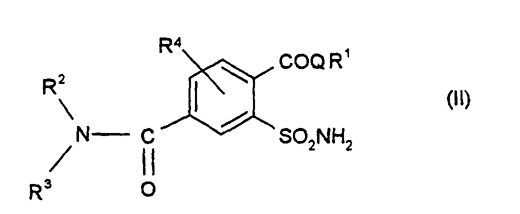 mit einem heterocyclischen Carbamat der Formel (III), 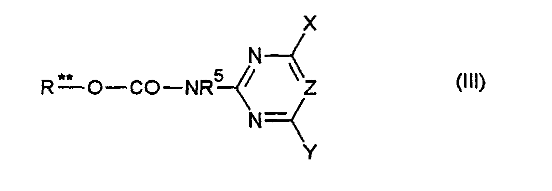 worin R** gegebenenfalls substituiertes Aryl oder einen aliphatischen Rest, vorzugsweise Phenyl oder (C₁-C₄)Alkyl bedeutet, umsetzt oder
b) ein Sulfonylcarbamat der Formel (IV), 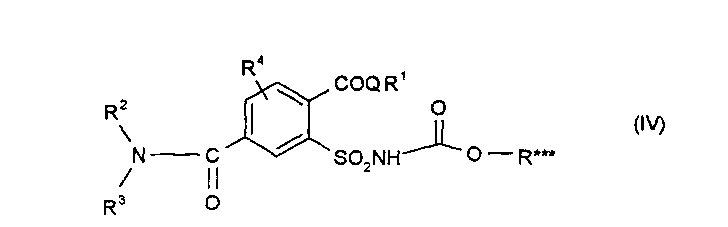 worin R*** gebenenfalls substituiertes Phenyl oder (C₁-C₄)Alkyl bedeutet, mit einem Aminoheterocyclus der Formel (V) 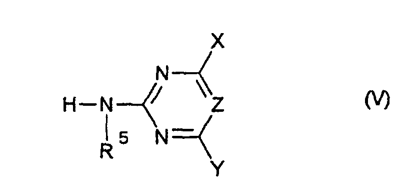 umsetzt oder
c) ein Sulfonylisocyanat der Formel (VI) 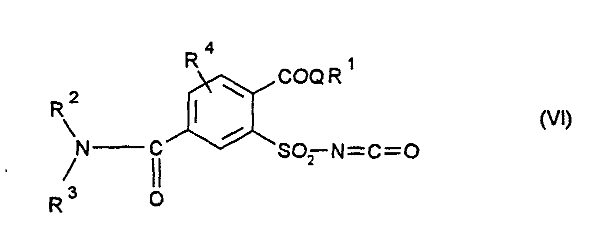 mit einem Aminoheterocyclus der Formel (V) umsetzt oder
d) ein Sulfonamid der Formel (II) mit einem (Thio)-Isocyanat der Formel (VII) 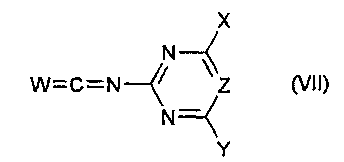 in Gegenwart einer Base umsetzt oder
e) einen Aminoheterocyclus der Formel (V) zunächst basenkatalysiert mit einem Kohlensäureester, z.B. Diphenylcarbonat, umsetzt und das gebildete Intermediat in einer Eintopfreaktion mit einem Sulfonamid der Formel (II) (siehe Variante a) umsetzt,
wobei in den Formeln (II)-(VII) die Reste bzw. Gruppen R¹-R⁵, W, X, Y und Z wie in Formel (I) definiert sind und in Verfahrensvarianten a) bis c) und e) zunächst Verbindungen (I) mit W = O erhalten werden.

Die Umsetzung der Verbindungen der Formeln (II) und (III) erfolgt vorzugsweise basenkatalysiert in einem inerten organischen Lösungsmittel, wie z.B. Dichlormethan, Acetonitril, Dioxan oder THF bei Temperaturen zwischen 0°C, vorzugsweise 20°C, und dem Siedepunkt des Lösungsmittels. Als Base werden dabei beispielsweise organische Aminbasen, wie 1,8-Diazabicyclo[5.4.0]undec-7-en (DBU) oder Alkalimetallhydroxide, wie z.B. NaOH, insbesondere bei R° = (subst.) Phenyl (vgl. EP-A-44807), oder Trimethylaluminium oder Triethylaluminium, letztere insbesondere bei R° = Alkyl (vgl. EP-A-166 516) verwendet. Die jeweilige Base wird dabei beispielsweise im Bereich von 1 bis 3 Moläquivalenten, bezogen auf die Verbindung der Formel (II) eingesetzt.

Die Sulfonamide (II) und damit strukturell verwandte Verbindungen der Formeln (IV) und (VI) sind neue Verbindungen. Sie und ihre Herstellung sind ebenfalls Gegenstand dieser Erfindung.

Man erhält die Verbindungen der Formel (II) z.B. ausgehend von Verbindungen der Formel (VIII) 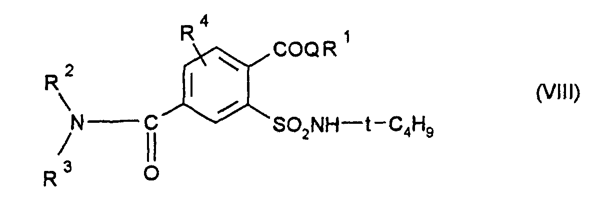 worin R¹-R⁴ wie in Formel (I) definiert sind, durch Umsetzung mit einer starken Säure (vgl. hierzu WO 89/10921). Als starke Säuren kommen z.B. Mineralsäuren, wie H₂SO₄ oder HCl, oder starke organische Säuren, wie Trifluoressigsäure in Frage. Die Abspaltung der tert.-Butyl-Schutzgruppe erfolgt beispielsweise bei Temperaturen von -20°C bis zur jeweiligen Rückflußtemperatur des Reaktionsgemisches, vorzugsweise bei 0°C bis 40°C. Die Umsetzung kann in Substanz oder auch in einem inerten Solvens, wie z.B. Dichlormethan oder Trichlormethan, durchgeführt werden.

Alternativ sind die Sulfonamide der Formel (II) mit Q=O auch ausgehend von den Saccharinderivaten der Formel (IX) durch Umsetzung mit gasförmigem Chlorwasserstoff in Gegenwart eines Alkohols der Formel R¹OH zugänglich (R¹-R⁴ sind definiert wie in Formel (I))

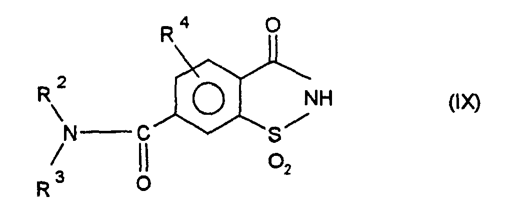

Man führt die Reaktion beispielsweise bei Temperaturen zwischen 0°C und dem Siedepunkt des Alkohols R¹OH durch, wobei der Alkohol R¹OH gleichzeitig als Lösungsmittel dienen kann (vgl. hierzu: US-A-4 566 898; J. Pharmaceutical Sciences 56, 134 (1967)). Eine weitere Synthesemöglichkeit für die Herstellung der Verbindungen der Formel (II) ist gegeben durch die Umsetzung der Sulfochloride der Formel (X) mit Ammoniak, 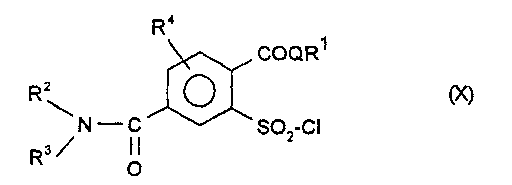 wobei die Reste R¹ bis R⁴ sowie Q wie in Formel (I) definiert sind. Die Reaktion wird beispielsweise in inerten Lösungsmitteln wie z.B. Dichlormethan, Tetrahydrofuran (THF), Dioxan, Toluol oder Dimethylformamid (DMF) bei Temperaturen von -70°C bis zum Siedepunkt des Lösungsmittels, vorzugsweise bis 25°C durchgeführt. Dabei kommt bevorzugt eine Ammoniakmenge von 1,5-2,5 Äquivalenten bezogen auf Sulfochlorid zum Einsatz.
Die Zwischenprodukte der Formel (VIII) erhält man beispielsweise gemäß Schema 1:

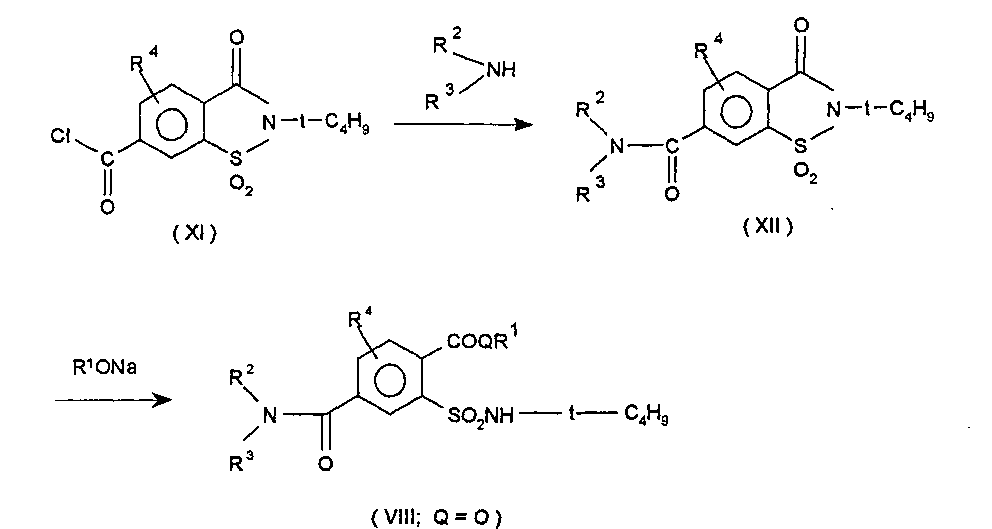

Die Verbindungen der Formel (XI) sind in WO 96/05182 beschrieben. Ihre Umsetzung mit Aminen zu den Amiden der Formel (XII) erfolgt nach im Prinzip bekannten Methoden (vgl. Houben Weyl "Methoden der Organischen Chemie", 4. Aufl. Bd. 8, S. 655 ff, Thieme Verlag, Stuttgart, 1952).
Eine basenkatalysierte Öffnung des Saccharinringes in (XII) führt schließlich zu den Verbindungen der Formel (VIII) mit Q = O, wobei als Lösungsmittel der jeweilige Alkohol R¹OH dient und als Base das entsprechende Alkoholat, bevorzugt das Natriumalkoholat, verwendet wird. Die Reaktion wird vorzugsweise bei Temperaturen zwischen -20°C und der Siedetemperatur des Alkohols durchgeführt. Aus den Verbindungen (VIII) mit Q=O erhält man analog zu bekannten Methoden die Verbindungen (VIII) mit Q = NR* durch Reaktion mit den Aminen HNR¹R*, wobei R¹ und R* wie in Formel (I) definiert sind (vgl. Houben-Weyl, "Methoden der Organischen Chemie", 4. Aufl., Bd. 8, S. 658 ff, Thieme Verlag, Stuttgart, 1952).

Eine weitere Methode zur Synthese von Verbindungen der Formel (VIII) besteht in der Öffnung des Saccharinringes in (XII) mit einem Äquivalent Kaliumhydroxid. Die Reaktion wird beispielsweise in wässrigem Ethanol, Aceton, Acetonitril oder Pyridin bei Temperaturen von 25°C bis zum Siedepunkt des Lösungsmittelgemisches durchgeführt. Das so erhaltene Kaliumcarboxylat der Formel (VIII), worin Q = O und R¹ = K bedeutet, kann nachfolgend mit 1 bis 5 Äquivalenten Alkylhalogenid R-Hal, vorzugsweise in Gegenwart von 1,4,7,10,13,16-Hexacyclooctan (18-Crown-6), z.B. von 0,1-Äquivalenten 18-Crown-6 unter Phasentransferbedingungen zur Verbindung (VIII; Q = O, R¹=R) alkyliert. Man führt die Reaktion beispielsweise in Acetonitril oder Benzol bei Temperaturen zwischen 25°C und der Siedetemperatur des Lösungsmittels durch (vgl. hierzu Tetrahedron Lett. 28, 2417-2420 (1974)).

Außer auf dem in WO 96/05182 beschriebenen Syntheseweg erhält man die Säurechloride der Formel (XI) vorteilhaft gemäß Schema 2.

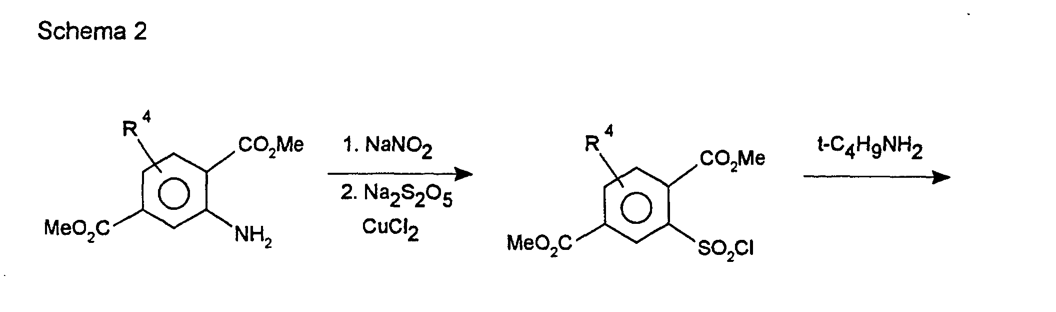

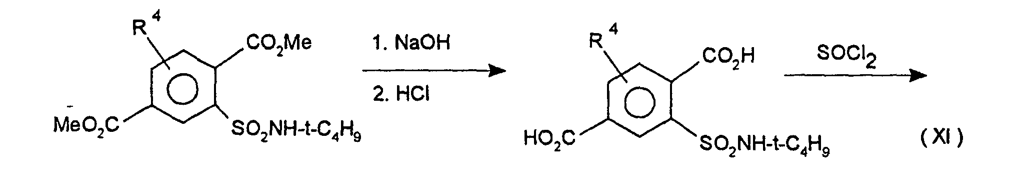

Das Edukt Aminoterephthalatsäuredimethylester beispielsweise ist kommerziell verfügbar; alle Reaktionsschritte können analog zu literaturbekannten Methoden durchgeführt werden.

Die Zwischenprodukte (IX) können gemäß Schema 3 erhalten werden. 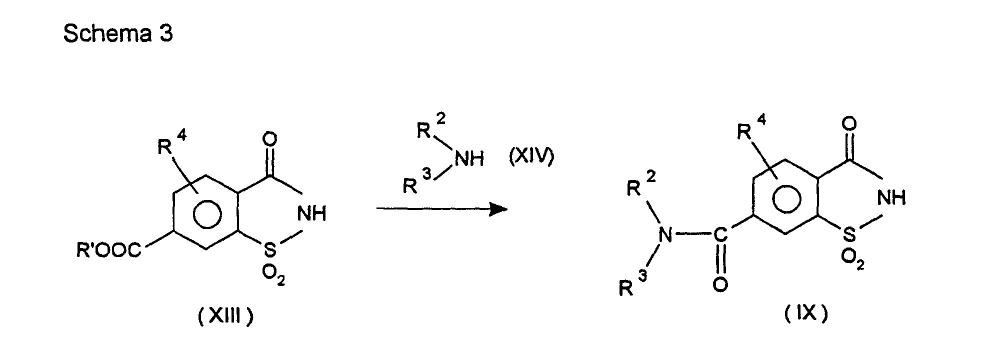 In den Verbindungen der Formeln der Schemata 1 bis 3, insbesondere den Formeln (VIII) und (IX), (XI), (XII), (XIII) und (XIV), sind die Reste R¹-R⁴ wie in Formel (I) definiert sind.
R' in Formel (XIII), Schema 3 bedeutet H oder einen gegebenenfalls substituierten Kohlenwasserstoffrest wie (C₁-C₄)Alkyl, (C₁-C₄)Haloalkyl oder Phenyl. Für den Fall, daß R' = H ist, muß die Carboxylfunktion zunächst durch ein geeignetes Agens aktiviert werden. Es eignen sich insbesondere Chlorameisensäurealkylester wie z.B. Chlorameisensäureisobutylester, Carbonyldiimidazol oder Dicyclohexylcarbodiimid. Die Reaktion wird beispielsweise in einem inerten Lösungsmittel bei Temperaturen zwischen -20°C und dem Siedepunkt des Lösungsmittels durchgeführt. Das Edukt (XIII) mit R' = R⁴ = H ist bekannt (vgl. hierzu Chem. Ber. 115, 1740 (1982)). Die Ester der Formel (XIII) mit R' = (C₁-C₆)Alkyl sind beschrieben in WO 96/05184. Ihre Umsetzung mit den Aminen (XIV) erfolgt bevorzugt in einem inerten Lösungsmittel bei Temperaturen zwischen 20°C und der Siedetemperatur des Lösungsmittels.

Sekundäre Alkyl- oder Allylamine der allgemeinen Formel (XIV) können durch Umsetzung primärer Amine mit Halogenverbindungen nach im Prinzip bekannten Methoden hergestellt werden (vgl. hierzu Ind. J. Chem. 15B, 135 (1977); J. Am. Chem. Soc. 81, 719, 722, 727 (1959); Bull. Chim. Soc. France II, 9-10, 395 (1984); Tetrahedron 29, 4118 (1973)).

Viele der Amine (XIV) sind kommerziell erhältlich oder können analog dem Fachmann allgemein bekannten Methoden hergestellt werden.

Gegenstand der Erfindung sind auch die Saccharinderivate der allgemeinen Formel (IX)' und deren Herstellung, 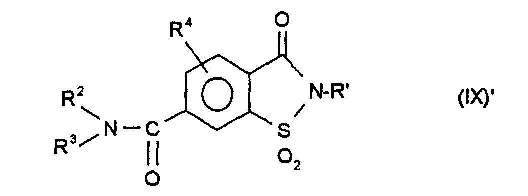 worin R² bis R⁴ wie in Formel (I) nach Anspruch 1 definiert ist und R' eine Alkyl gruppe, insbesondere tert.-Butyl bedeutet, und zu denen vorzugsweise die Verbindung (XII) gehört.

Die Zwischenprodukte der Formel (X) können gemäß Schema 4 erhalten werden. 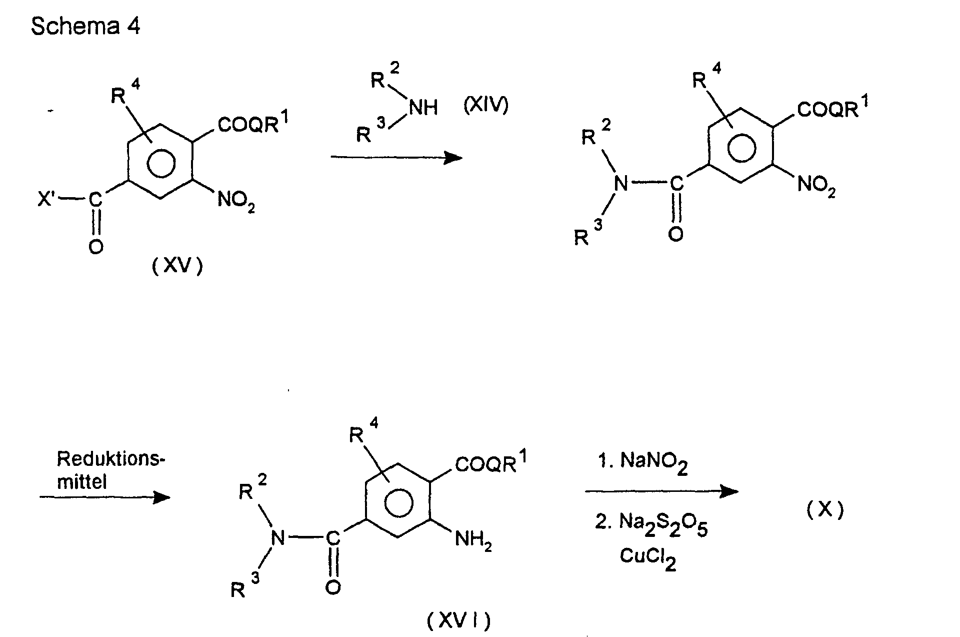 In den Formeln nach Schema 4 sind die Reste R¹-R⁴ und Q wie in Formel (I) definiert und X' bedeutet OH oder Cl. Für den Fall X' = OH ist z.B. die Verbindung mit R¹ = Methyl, R⁴ = H und Q = O kommerziell verfügbar. Andere Verbindungen des Typs (XV) sind auf bekanntem Weg zugänglich (vgl. hierzu Monatsh. Chem. 23, 406, 410, 412 (1902)). Zur Umsetzung der Verbindungen (XV) mit X'=OH mit den Aminen (XIV) muß zunächst die Carboxylfunktion mit einem geeigneten Agens aktiviert werden. Es eignen sich insbesondere Chlorameisensäurealkylester wie z.B. Chlorameisensäureisobutylester, Carbonyldiimidazol oder Dicyclohexylcarbodiimid. Die Reaktion wird beispielsweise in einem inerten Lösungsmittel bei Temperaturen zwischen -20°C und der Siedetemperatur des Lösungsmittels durchgeführt. Für den Fall X'=Cl sind die Verbindungen (XV) teils bekannt oder können analog zu bekannten Methoden hergestellt werden (vgl. hierzu J. Chem. Soc. 113, 66 (1918)). Ihre Umsetzung zu Amiden erfolgt ebenfalls analog zu bekannten Methoden (vgl. hierzu Houben-Weyl, "Methoden der organischen Chemie", 4. Aufl. Bd. 8, S. 655 ff, Thieme Verlag Stuttgart, 1952).
Die weiteren Umsetzungen zu den Anilinderivaten (XVI) bzw. zu den Sulfochloriden (X) können analog zu bekannten Methoden durchgeführt werden; vgl. Houben-Weyl, "Methoden der organischen Chemie", 4. Aufl. Bd. 11/1, S. 360 ff, Thieme Verlag Stuttgart, 1957 bzw. ebenda Bd. 9, S. 579 ff, 1955.

Die Carbamate der Formel (III) können nach Methoden hergestellt werden, die in den südafrikanischen Patentanmeldungen 82/5671 und 82/5045 bzw. EP-A 70804 (US-A-4 480 101) oder RD 275056 beschrieben sind.

Die Umsetzung der Verbindungen (IV) mit den Aminoheterocyclen (V) führt man vorzugsweise in inerten, aprotischen Lösungsmitteln wie z.B. Dioxan, Acetonitril oder Tetrahydrofuran bei Temperaturen zwischen 0 °C und der Siedetemperatur des Lösungsmittels durch. Die benötigten Ausgangsmaterialien (V) sind literaturbekannt oder können nach literaturbekannten Verfahren hergestellt werden. Die Phenylsulfonylcarbamate der Formel (IV) erhält man analog US-A-4 684 393 oder US-A-4 743 290.

Die Phenylsulfonylisocyanate der Formel (VI) lassen sich analog US-A-4 481 029 herstellen und mit den Aminoheterocyclen (V) umsetzen.

Die (Thio-)Isocyanate der Formel (VII) sind nach literaturbekannten Verfahren erhältlich (EP-A-232067, EP-A-166516). Die Umsetzung der (Thio)-Isocyanate (VII) mit Verbindungen (II) erfolgt beispielsweise bei -10 °C bis 100 °C, vorzugsweise 20°C bis 100 °C, in einem inerten aprotischen Lösungsmittel, wie z.B. Aceton oder Acetonitril, in Gegenwart einer geeigneten Base, z.B. N(C₂H₅)₃ oder K₂CO₃.

Unter Umgehung der Isolierung von Zwischenprodukten wie z.B. der Isocyanate der Formel (VI) lassen sich die Verbindungen der Formel (I) auch direkt aus den Sulfochloriden (X) und den Aminoheterocyclen (V) in Gegenwart eines Alkali- oder Ammoniumcyanats und Pyridin herstellen (vgl. hierzu US-A-5157119).

Die Umsetzung eines Aminoheterocyclus der Formel (V) mit Diphenylcarbonat und einem Sulfonamid der Formel (II) in einer Eintopfreaktion kann gemäß EP-A-562 575 durchgeführt werden.

Die genannten Verbindungen der Formel (II), (IV), (VI), (VIII) und (X) sind strukturverwandte neue Zwischenprodukte der allgemeinen Formel (II)* 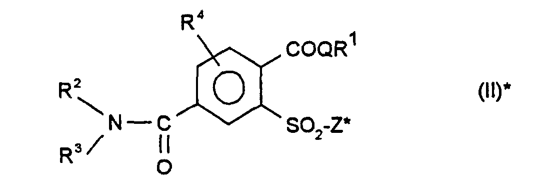 worin Z*= NH₂, NHCOOR***, NCO, NH-tert.-Butyl oder Cl bedeutet und R¹-R⁴, R*** und Q wie in Formel (I) bzw. Formel (IV) definiert sind.

Die Salze der Verbindungen der Formel (I) werden vorzugsweise in inerten polaren Lösungsmitteln wie z.B. Wasser, Methanol oder Aceton bei Temperaturen von 0°C bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, oder Alkalialkoholate, wie Natriummethanolat oder Natrium-tert.-butanolat, oder Ammoniak oder Ethanolamin.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) und deren Salze, im folgenden zusammen als (erfindungsgemäße) Verbindungen der Formel (I) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.
Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.
Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft.
Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen oder in Zierpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel (I) oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.
Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Herbizide aus der Gruppe der Sulfonylharnstoffe, Glufosinate-ammonium oder Glyphosate-isopropylammonium und analoge Wirkstoffe resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasserin-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streuund Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emutsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z:B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B.
G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie z.B. aus Weed Research 26, 441-445 (1986) oder "The Pesticide Manual", 11th edition, The British Crop Protection Council and the Royal Soc. of Chemistry, 1997 und dort zitierter Literatur beschrieben sind. Als bekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d.h. Ammoniumsulfamat; anilofos; asulam; atrazin; azafenidin; azimsulfuron (DPX-A8947); aziprotryn; barban; BAS 516 H, d.h. 5-Fluor-2-phenyl-4H-3,1-benzoxazin-4-on; BAS 620 H; BAS 65400H; BAY FOE 5043; benazolin; benfluralin; benfuresate; bensulfuron-methyl; bensulide; bentazone; benzofenap; benzofluor; benzoylprop-ethyl; benzthiazuron; bialaphos; bifenox; bispyribac-Na; bromacil; bromobutide; bromofenoxim; bromoxynil; bromuron; buminafos; busoxinone; butachlor; butamifos; butenachlor; buthidazole; butralin; butroxydim; butylate; cafenstrole (CH-900); caloxydim; carbetamide; carfentrazone-ethyl; CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafoppropargyl); clomazone; clomeprop; cloproxydim; clopyralid; cloransulam-methyl; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diclosulam, d.h. N-(2,6-Dichlorphenyl)-5-ethoxy-7-fluor[1,2,4]triazolo[1,5-c]pyrimidin-2-sulfonamid; diethatyl; difenoxuron; difenzoquat; diflufenican; diflufenzopyr (BAS 654 00H), dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1Hpyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); flupyrsulfuron-methyl-sodium; fluridone; flurochloridone; fluroxypyr; flurtamone; fluthiacet-methyl; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazamethabenz-methyl; imazamox; imazapyr; imazaquin und Salze wie das Ammoniumsalz; imazethamethapyr; imazethapyr; imazosulfuron; indanofan (MK-243), ioxynil; isocarbamid; isopropalin; isoproturon; isouron; isoxaben; isoxaflutole; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metobenzuron; metobromuron; metolachlor; metosulam (XRD 511); metoxuron; metribuzin; metsulfuron-methyl; MH; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxasulfuron; oxaziclomefone (MY-100); oxyfluorfen; paraquat; pebulate; pendimethalin; pentoxazone (KPP-314); perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyroflufen-ethyl; pyrazolinate; pyrazon; pyrazosulfuron-ethyl; pyrazoxyfen; pyribenzoxim (LGC-40836); pyributicarb; pyridate; pyriminobac-methyl; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; sulcotrione; sulfentrazon (FMC-97285, F-6285); sulfazuron; sulfometuron-methyl; sulfosate (ICI-A0224); sulfosulfuron; TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiazopyr (Mon-13200); thidiazimin (SN-24085); thifensulfuron-methyl; thiobencarb; tiocarbazil; tralkoxydim; tri-allate; triasulfuron; triaziflam, triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1H-tetrazol; JTC-101; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

### A. Chemische Beispiele

### Beispiel A1

### Chlorsulfonylterephthalsäuredimethylester

Zu einer Lösung von 104,6 g (0,5 mol) Aminoterephthalsäuredimethylester in 500 ml Eisessig und 165 ml konz. Salzsäure gab man bei 10°C innerhalb von ca. 20 min eine Lösung von 36,2 g (0,52 mol) Natriumnitrit in 100 ml Wasser. Man rührt 10 min bei dieser Temperatur nach, filtrierte von wenig ungelöstem Material ab und tropfte das Filtrat innerhalb von 35 min bei 15-20°C zu einer Lösung, die wie folgt hergestellt wurde: Zu 360 ml konz. Salzsäure tropfte man bei Raumtemperatur 8,5 g (0,1 mol) CuCl₂ in 100 ml Wasser und kühlte auf 5°C ab. Hierzu tropfte man eine Lösung aus 118,8 g (0,625 mol) Na₂S₂O₅ in 180 ml Wasser.
Nach Zugabe der Diazoniumsalz-Lösung rührte man 30 min bei 15-20°C nach und goß anschließend in 2 l Eiswasser. Nach Extraktion mit Diethylether, Waschen der Etherphase mit Wasser, Trocknen und Eindampfen erhielt man 118,3 g (81 % d.Th.) Chlorsulfonylterephthalsäuredimethylester als braunes Öl, das ohne Reinigung in die folgende Reaktion eingesetzt wurde.

### Beispiel A2

### tert.-Butylsulfamoylterephthalsäuredimethylester

Zu einer Lösung von 118,3 g (0,4 mol) rohem Chlorsulfonylterephthalsäuredimethylester (aus Beispiel A1) in 500 ml Dichlormethan tropfte man bei 5°C 59,1 g (0,81 mol) tert-Butylamin und rührte bei Raumtemperatur 1 h nach. Die Reaktionslösung wurde mit Wasser gewaschen, getrocknet und eingedampft. Nach Verreiben des Rohprodukts mit Heptan erhielt man 106,7 g (81 % d.Th.) tert.-Butylsulfamoylterephthalsäuredimethylester vom Schmp. 132-134°C.

### Beispiel A3

### tert.-Butylsulfamoylterephthalsäure

Zu einer Lösung von 106,0 g (0,32mol) tert.-Butylsulfamoylterephthalsäuredimethylester in 1600 ml Methanol tropfte man bei 50°C 45,0 g (1,13 mol) Natriumhydroxid in 600 ml Wasser. Man rührte 2h bei Rückflußtemperatur, dampfte ein, nahm den Rückstand in ca. 500 ml Wasser auf und brachte mit konz. Salzsäure auf pH 1. Der ausgefallene Feststoff wurde abgesaugt und getrocknet. Man erhielt 87,4 g (90 % d.Th.) tert.-Butylsulfamoylterephthalsäure vom Schmp. 210-213°C.

### Beispiel A4

### 2-tert.-Butylsaccharin-6-carbonsäurechlorid

15,0 g (0,05 mol) tert.-Butylsulfamoylterephthalsäure wurden in 95 ml Thionylchlorid 8h am Rückfluß erhitzt. Man dampfte ein und verrieb den Rückstand mit Essigester. Es hinterblieben 14,6 g (97 % d.Th.) 2-tert.-Butylsaccharin-6-carbonsäurechlorid vom Schmp. 185-186°C.

### Beispiel A5

### 2-tert.-Butylsaccharin-6-carbonsäureisopropylamid

Zu 6,0 g (0,02 mol) 2-tert.-Butylsaccharin-6-carbonsäurechlorid in 30 ml THF tropfte man bei 10°C 2,6 g (0,044 mol) Isopropylamin und rührte 3h bei 10-15°C nach. Man goß in Wasser, extrahierte mit Dichlormethan,wusch die organische Phase mit Wasser, trocknete und dampfte ein. Es hinterblieben 5,6 g (86 % d.Th.) 2-tert.-Butylsaccharin-6-carbonsäureisopropylamid vom Schmp. 152-154°C.

### Beispiel A6

### 2-tert.-Butylsulfamoyl-4-isopropylcarbamoylbenzoesäuremethylester

Man gab 22,0 g (0,068 mol) 2-tert.-Butylsaccharin-6-carbonsäureisopropylamid zu einer Lösung von 1,56 g (0,068 mol) Natrium in 250 ml abs. Methanol und rührte 5h bei Raumtemperatur. Man dampfte ein, nahm in Dichlormethan auf, wusch mit 2N HCl und Wasser, trocknete und dampfte ein. Es hinterblieben 20,8 g (86 % d.Th.) 2-tert.-Butylsulfamoyl-4-isopropylcarbamoylbenzoesäuremethylester vom Schmp. 159 bis 160°C.

### Beispiel A7

### 4-Isopropylcarbamoyl-2-sulfamoylbenzoesäuremethylester

19,6 g (0,055 mol) 2-tert.-Butylsulfamoyl-4-isopropylcarbamoylbenzoesäuremethylester wurden in 200 ml Trifluoressigsäure 3h bei Raumtemperatur gerührt. Man dampfte ein, verrieb den Rückstand mit Diethylether und filtrierte ab. Man erhielt 15,6 g (95 % d.Th.) 4-Isopropylcarbamoyl-2-sulfamoylenzoesäuremethylester vom Schmp. 203-205°C.

### Beispiel A8

### 2-[3-(4,6-Dimethoxypyrimidin-2-yl)ureidosulfonyl]-4-isopropylcarbamoylbenzoesäuremethylester

2,6 g (8,6 mmol) 4-Isopropylcarbamoyl-2-sulfamoylbenzoesäuremethylester und 2,8 g (10,3 mmol) N-(4,6-dimethoxypyrimidin-2-yl)carbaminsäurephenylester wurden in 50 ml Acetonitril vorgelegt. Bei Raumtemperatur tropfte man 2,9 g (19 mmol) 1,8-Diazabicyclo[5.4.0]undec-7-en (DBU) zu und rührte 3h bei dieser Temperatur. Man goß in Eiswasser und stellt den pH-Wert mit 2N Salzsäure auf 1 ein. Man saugte den ausgefallenen Feststoff ab und wusch mit Wasser nach. Nach Trocknen erhielt man 3,9 g (94 % d.Th.) 2-[3-(4,6-Dimethoxypyrimidin-2-yl)ureidosulfonyl]-4-isopropylcarbamoylbenzoesäuremethylester vom Schmp. 149-151°C (Zers.).

### Beispiel A9

### 2-[3-(4,6-Dimethoxypyrimidin-2-yl)ureidosulfonyl]-4-pyrrolidinocarbonylbenzoesäuremethylester, Natriumsalz

Analog den Beispielen A5 bis A8 wurde unter Verwendung von Pyrrolidin als Amin in Beispiel A5 zunächst 2-[3-(4,6-Dimethoxypyrimidin-2-yl)ureidosulfonyl]-4-pyrrolidinocarbonylbenzoesäuremethylester hergestellt. Anschließend ließ man zu 0,5 g (1,0 mmol) dieser Verbindung in 15 ml Methanol bei Raumtemperatur 0,2 g (1,1 mmol) einer 30 %igen Natriummethylat-Lösung in Methanol zutropfen. Man rührte 2h bei Raumtemperatur, saugte ab, wusch mit Methanol und trocknete. Man erhielt 0,5 g (96 % d.Th.) 2-[3-(4,6-Dimethoxypyrimidin-2-yl)-ureidosulfonyl]-4-pyrrolidinocarbonylbenzoesäuremethylester, Natriumsalz vom Schmp. 233-235°C (Zers. )

### Beispiel A10

### 2-tert.-Butylsulfamoyl-4-carbamoylbenzoesäuredimethylamid

Analog den Beispielen A5 bis A6 wurde unter Verwendung von Ammoniak als basisches Reagens in Beispiel A5 wurde zunächst 2-tert.-Butylsulfamoyl-4-carbamoylbenzoesäuremethylester hergestellt. Anschließend gab man bei Raumtemperatur 3,0 g (0,01 mol) dieser Verbindung zu einer Lösung von 45 g (1,0 mol) Dimethylamin in 60 ml Methanol und ließ das Reaktionsgemisch 7h bei Raumtemperatur stehen. Man dampfte ein und isolierte das gewünschte Produkt durch Säulenchromatographie an Kieselgel (Laufmittel: Essigester; R_{f} ≈ 0,2). Man erhielt 1,0 g (31 % d.Th.) 2-tert.-Butylsulfamoyl-4-carbamoylbenzoesäuredimethylamid von glasartiger Konsistenz.

### Beispiel A11

### 4-Carbamoyl-2-sulfamoylbenzoesäuredimethylamid

1,0 g (3,1 mmol) 2-tert.-Butylsulfamoyl-4-carbamoylbenzoesäuredimethylamid wurden in 10 ml Trifluoressigsäure 2,5 h bei Raumtemperatur gerührt. Man dampfte ein, verrieb den Rückstand mit Diethylether, saugte ab und trocknete. Man erhielt so 0,53 g (63 % d.Th.) 4-Carbamoyl-2-sulfamoylbenzoesäuredimethylamid vom Schmp. 202-205°C.

### Beispiel A12

### 2-[3-(4,6-Dimethoxypyrimidin-2-yl)ureidosulfonyl]-4-carbamoylbenzoesäuredimethylamid

0,53 g (2,0 mmol) 4-Carbamoyl-2-sulfamoylbenzoesäuredimethylamid und 0,59 g (2,2 mmol) N-(2,4-dimethoxypyrimidin-2-yl)carbaminsäurephenylester wurden in 15 ml Acetonitril vorgelegt. Bei Raumtemperatur tropfte man 0,39 g (2,5 mmol) 1,8-Diazabicyclo[5.4.0]undec-7-en (DBU) zu und rührte 3h bei dieser Temperatur. Man goß in Wasser und stellte den pH-Wert mit 2N Salzsäure auf ca. 2 ein. Die Wasserphase wurde dreimal mit CH₂Cl₂ extrahiert. Nach Waschen der organischen Phase mit 2N Salzsäure und Wasser trocknete man und dampft ein. Der Rückstand wurde mit Diisopropylether verrieben. Nach Absaugen und Trocknen erhielt man 0,60 g (67 % d.Th.) 2-[3-(4,6-Dimethoxypyrimidin-2-yl)ureidosulfonyl]-4-carbamoylbenzoesäuredimethylamid vom Schmp. 194-196°C (Zers.).

### Beispiel A13

### N-Allylisopropylamin

Zu einer Lösung von 8,0 g (0,2 mol) Natriumhydroxid in 25 ml Wasser gab man tropfenweise 20,7 g (0,35 mol) Isopropylamin. Anschließend versetzt man binnen 1 h tropfenweise mit 15,3 g (0,2 mol) Allylchlorid, wobei die Temperatur bei 35-40°C gehalten wurde. Man erwärmte für weitere 2,5 h auf 60°C und ließ dann abkühlen. Zur Aufarbeitung wurden die Phasen getrennt und die wässrige Phase mit Ether extrahiert. Die organischen Phasen wurden vereinigt, über Natriumhydroxid getrocknet, abdekantiert und destilliert. Man erhielt so 9,6 g (48 % d.Th.) farbloses N-Allylisopropylamin vom Sdp. 96-98°C.

### Beispiel A14

### 2-tert.-Butylsulfamoyl-4-dimethylcarbamoylbenzoesäure, Kaliumsalz

Zu einer Lösung von 19,7 g (63,5 mmol) 2-tert.-Butylsaccharin-6-dimethylamid in 235 ml Ethanol ließ man bei 25°C unter Rühren 254 ml einer 0,25 M wässrigen Lösung von Kaliumhydroxid einfließen. Anschließend erhitzte man 6 h unter Rückfluß, ließ dann abkühlen und evaporierte zur Trockne. Es hinterblieben 23,3 g (100 % d.Th.) 2-tert.-Butylsulfamoyl-4-dimethylcarbamoylbenzoesäure, Kaliumsalz vom Schmp. >260°C als farbloser Feststoff.

### Beispiel A15

### 2-tert.-Butylsulfamoyl-4-dimethylcarbamoylbenzoesäureethylester

Zu einer Suspension von 3,0 g (8,2 mmol) 2-tert.-Butylsulfamoyl-4-dimethylcarbamoylbenzoesäure, Kaliumsalz und 0,21 g (0,79 mmol) 1,4,7,10,13,16-Hexaoxacyclooctan in 25 ml Acetonitril gab man nach halbstündigem Rühren bei Raumtemperatur 890 mg (8,2 mmol) Bromethan. Anschließend erhitzte man für 6 h unter Rückfluß und ließ dann auf Raumtemperatur abkühlen. Zur Aufarbeitung verdünnte man das Reaktionsgemisch mit Essigsäureethylester und extrahierte mit 1 M wässriger Salzsäurelösung, gesättigter wässriger Natriumhydrogencarbonatlösung und Wasser. Die organische Phase wurde über Natriumsulfat getrocknet, abfiltriert und eingeengt. Es hinterblieben 2,31 g (79 % d.Th.) 2-tert.-Butylsulfamoyl-4-dimethylcarbamoylbenzoesäureethylester als farbloser Feststoff vom Schmp. 101 bis 102,5°C.

Die in den nachfolgenden Tabellen 1 und 2 beschriebenen Verbindungen werden nach bzw. analog den obigen Beispielen A1 - A15 erhalten.

Abkürzungen in den Tabellen 1 und 2:
Smp. = Fp. = Festpunkt, Schmelzpunkt in °C
(Z) = Schmelzpunkt unter Zersetzung
Bu = n-Butyl; entsprechend Pentyl = n-Pentyl, Hexyl = n-Hexyl)
Et = Ethyl
Me = Methyl
Ph = Phenyl
Pr, i-Pr, c- Pr = n-Propyl, Isopropyl bzw. Cyclopropyl
Ein Diradikal wie Butylen der Formel

in den Spalten für R², R³ heißt, daß R² und R³ zusammen die Diradikalbrücke bedeuten und mit dem N-Atom der Gruppe R²R³N ein cyclisches Amin bilden.

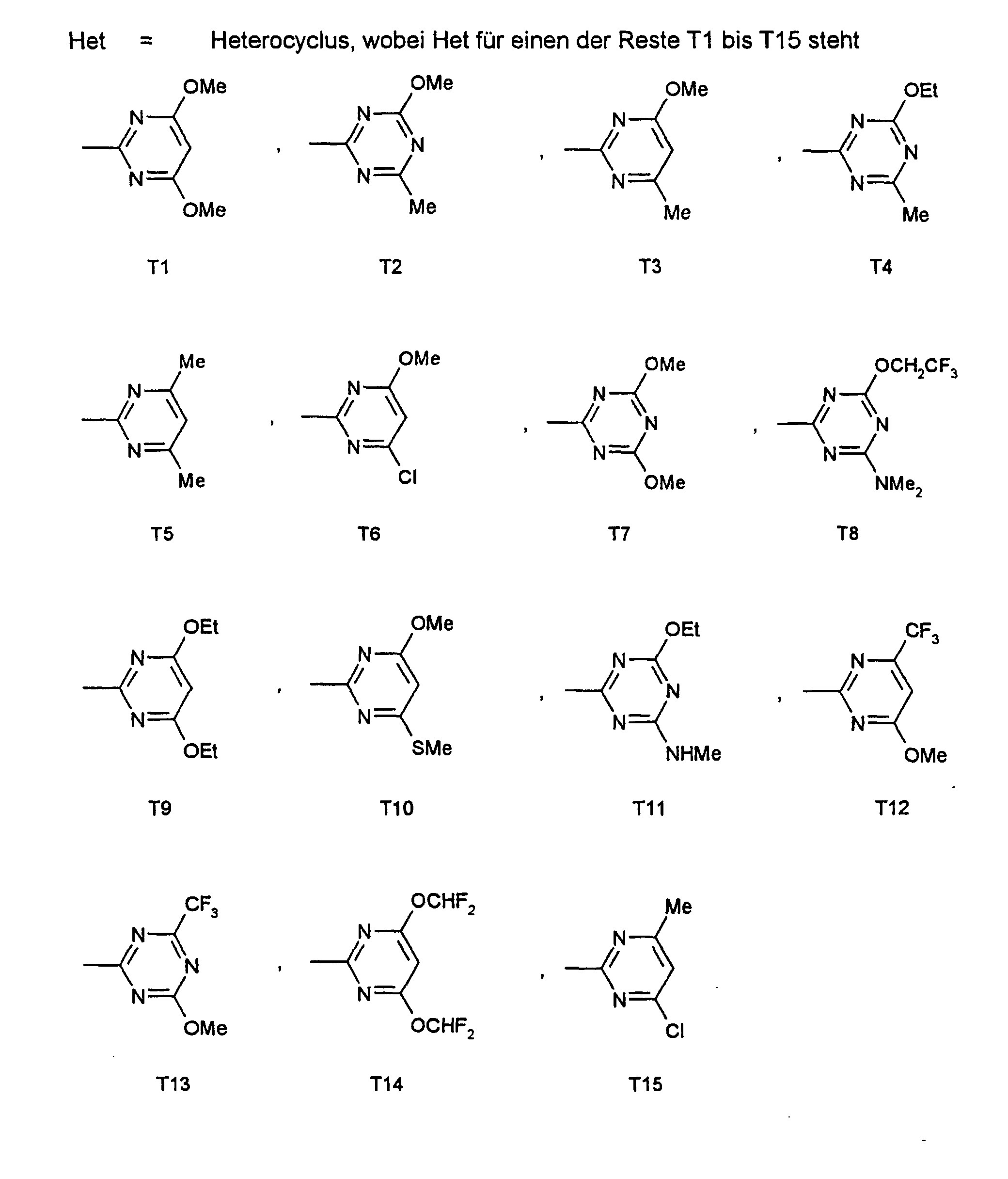

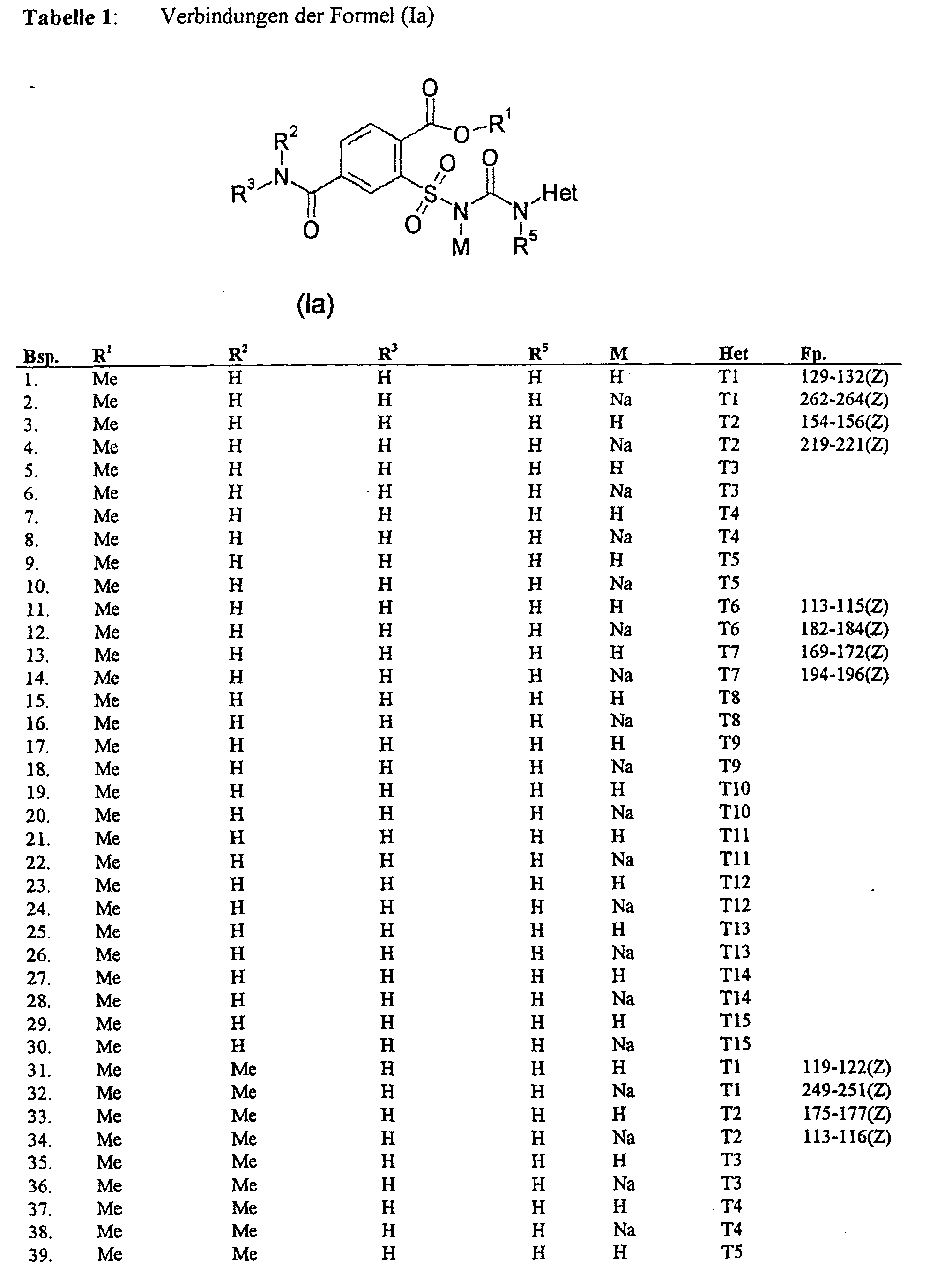

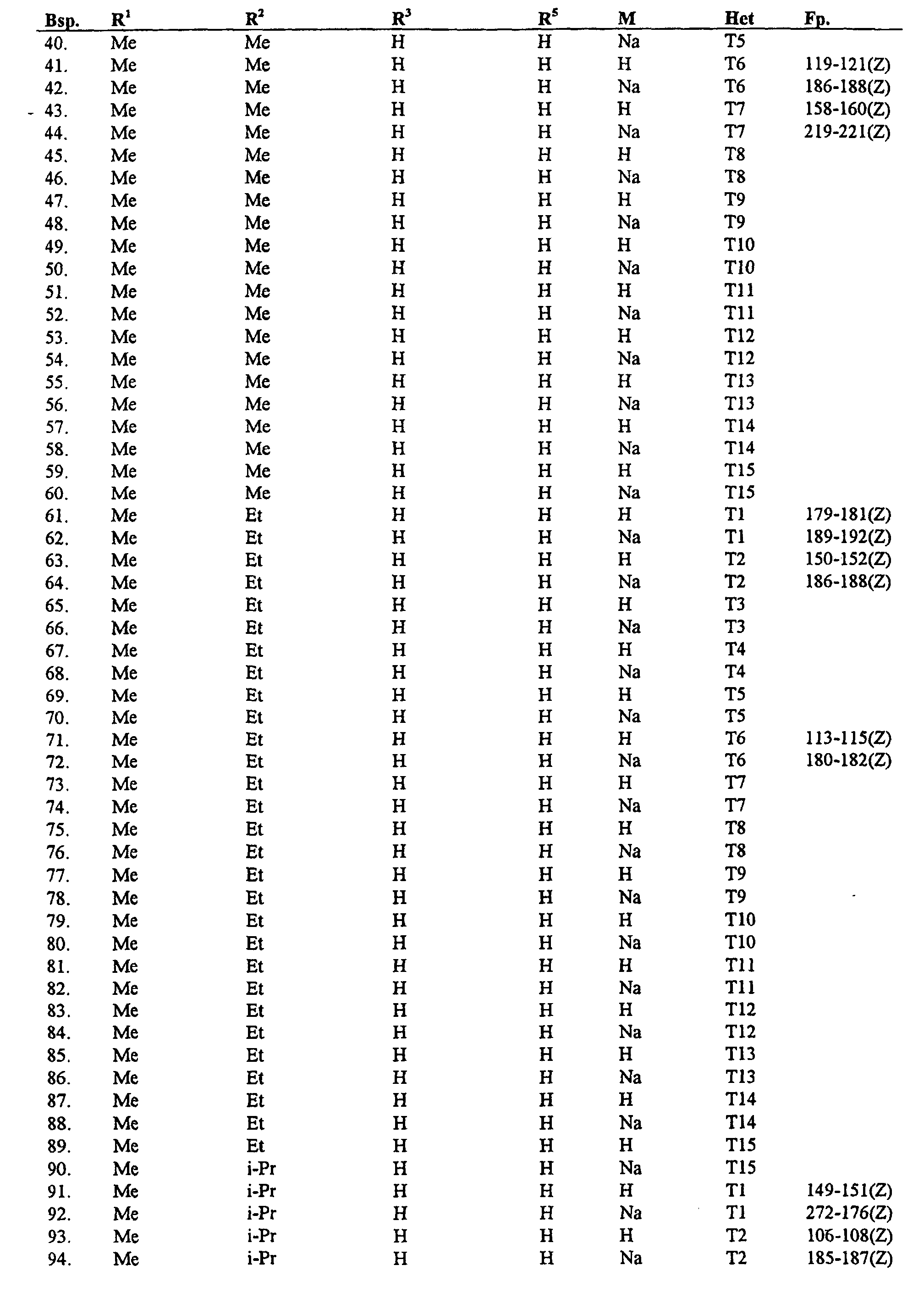

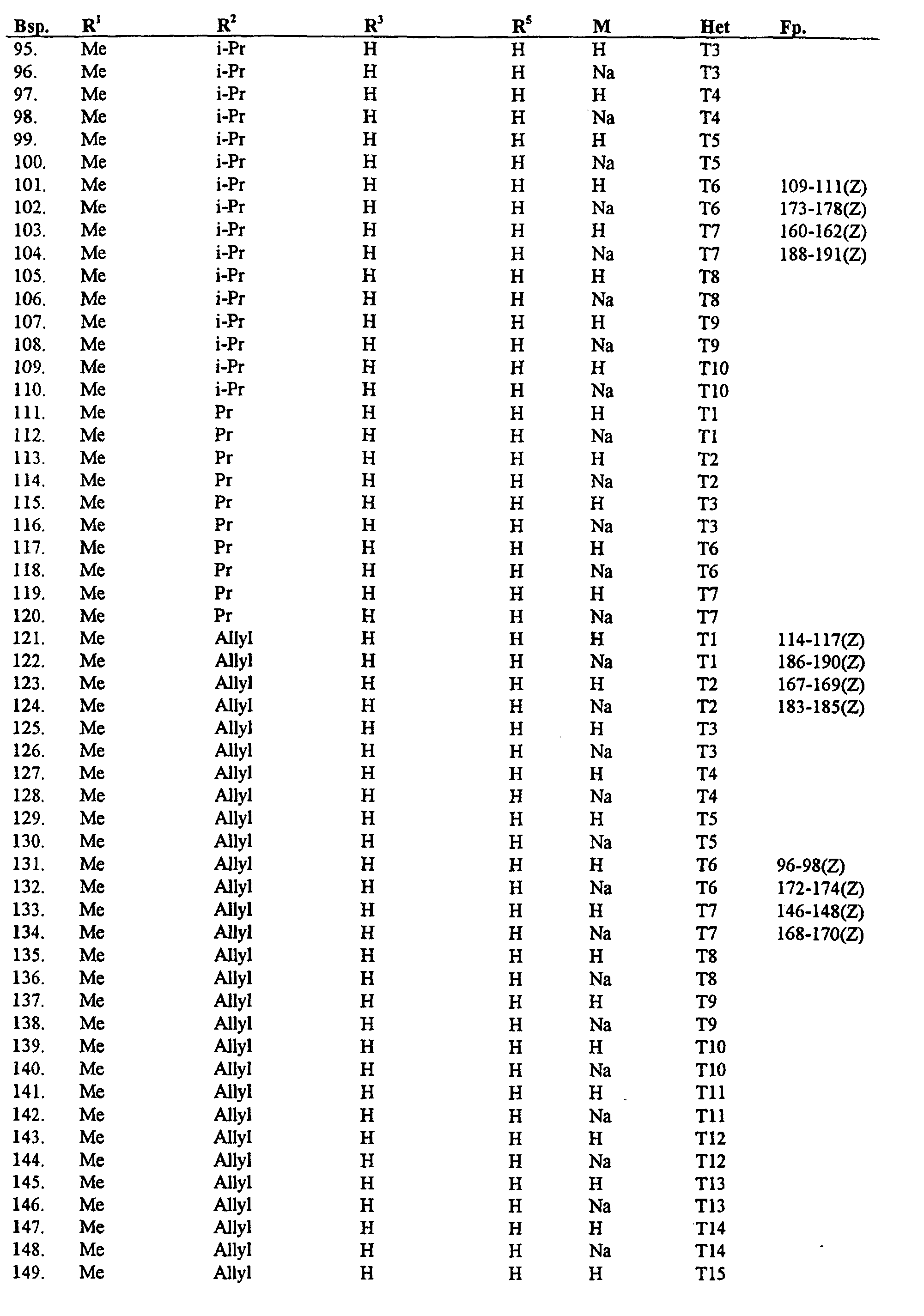

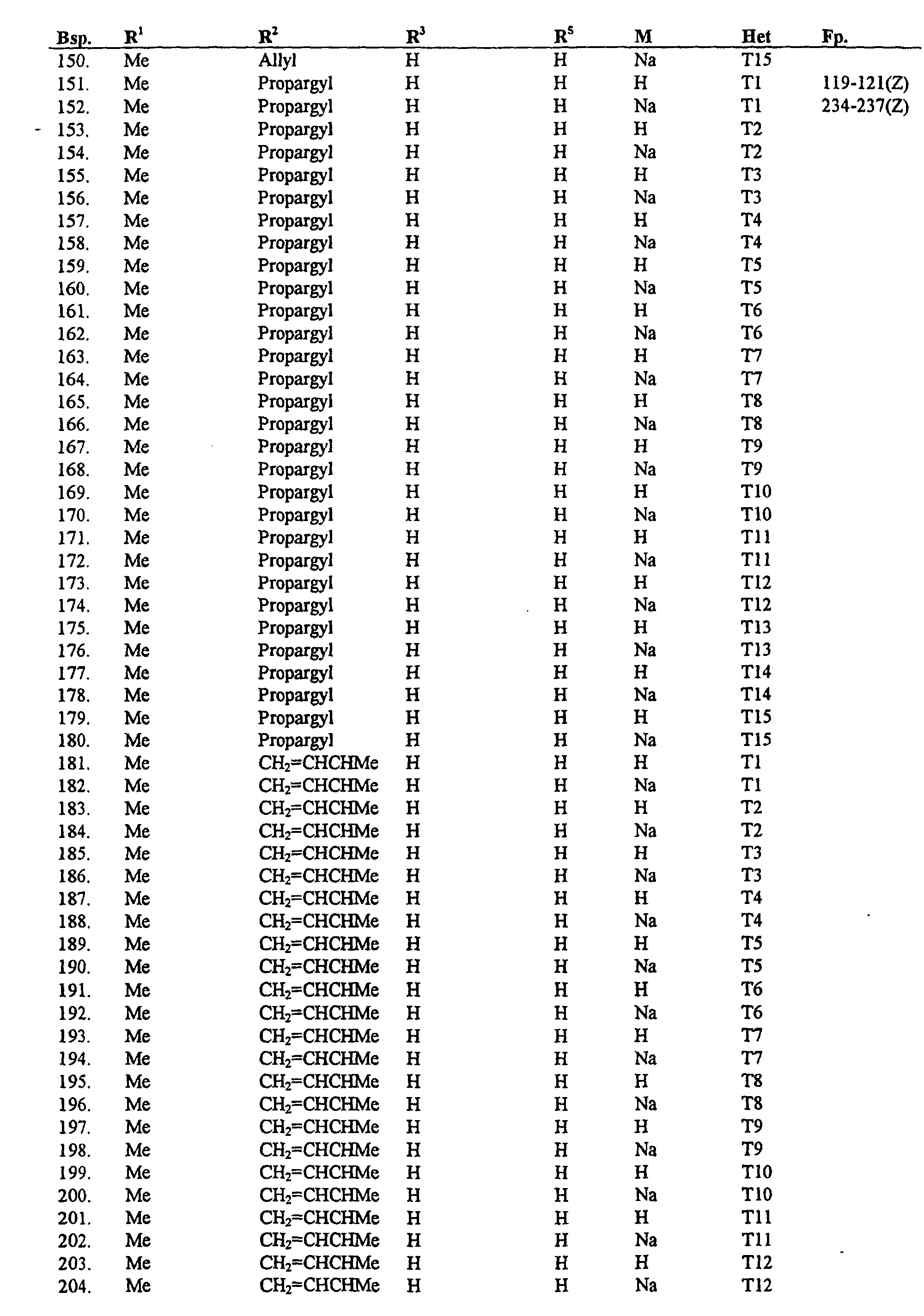

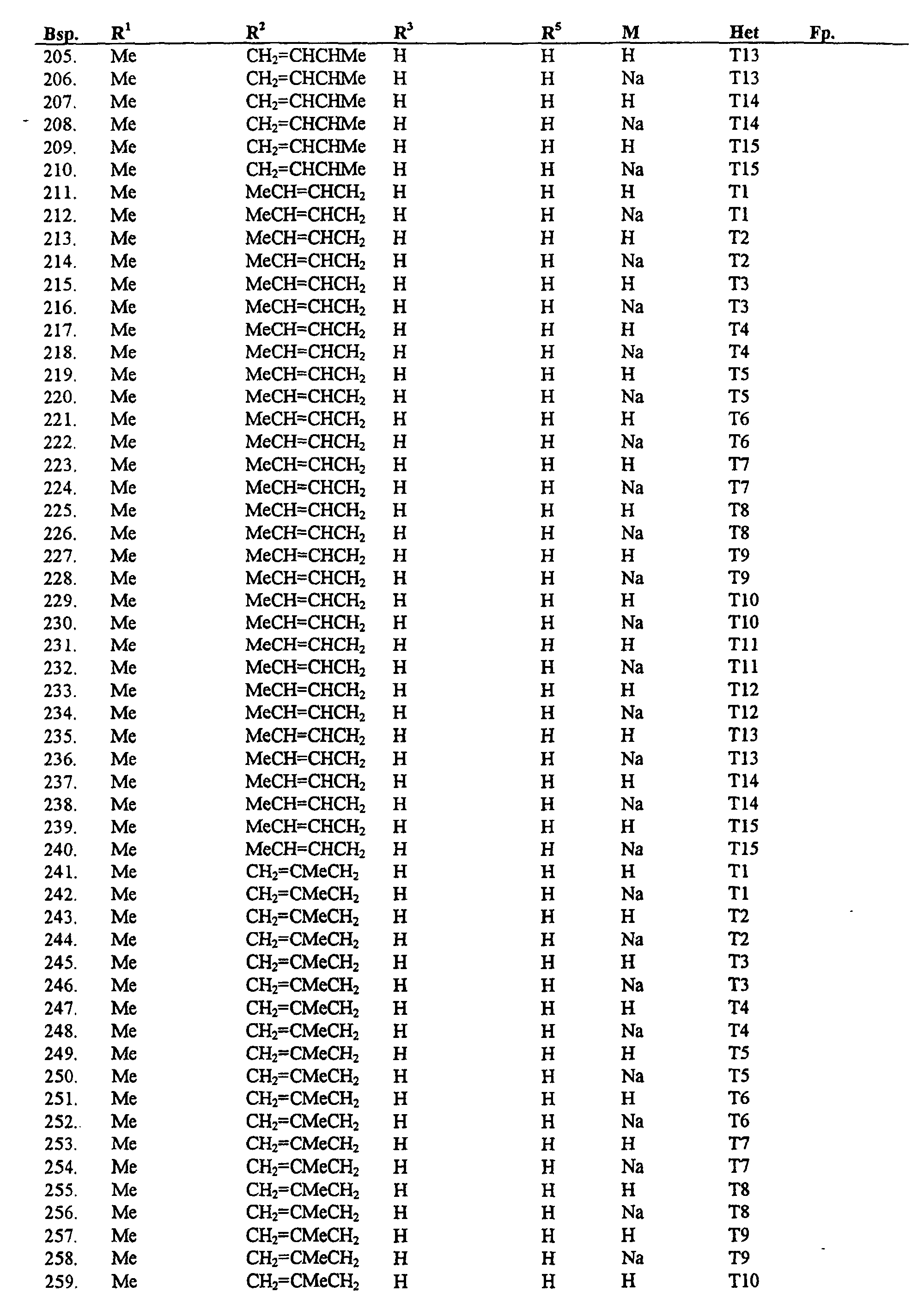

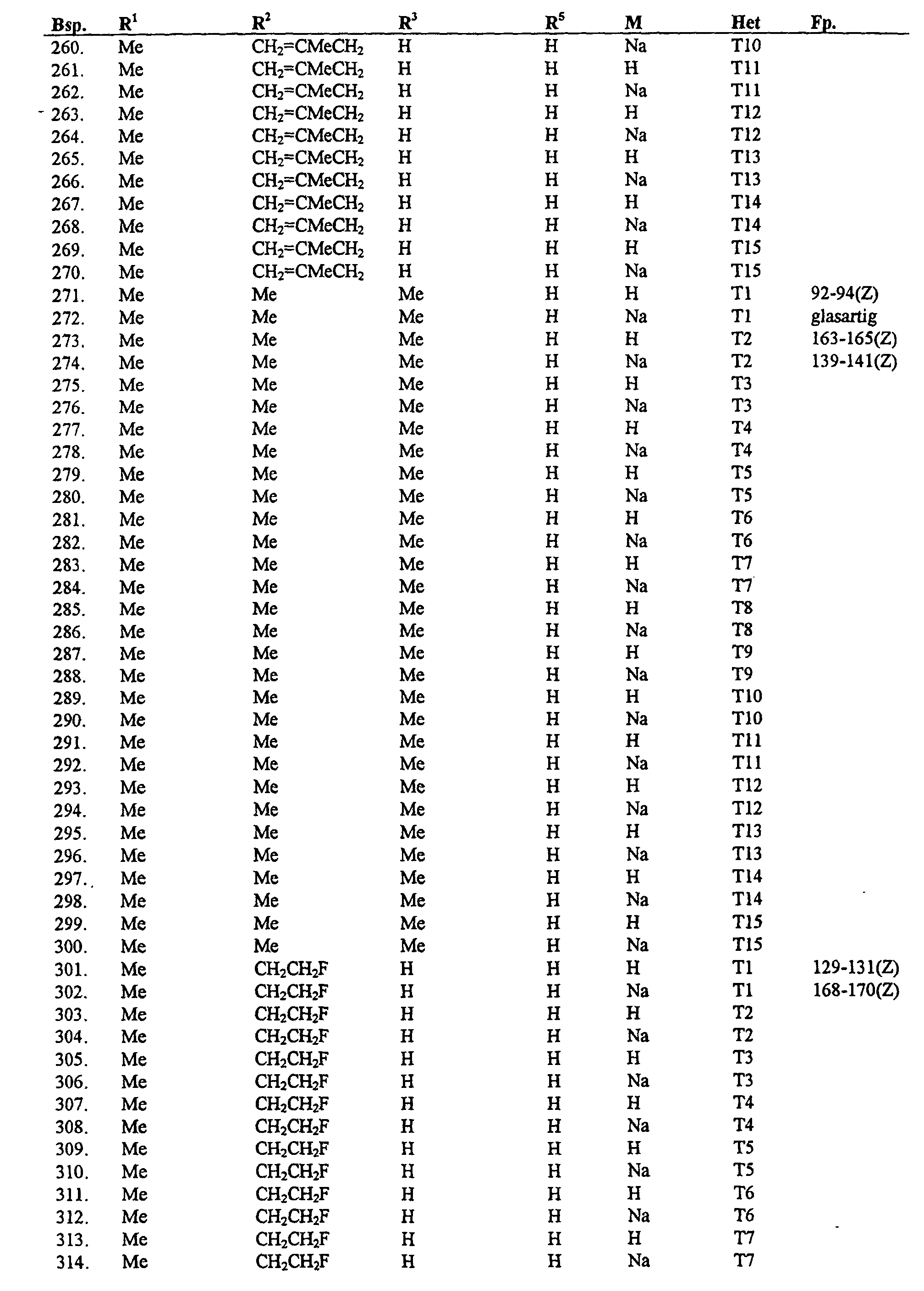

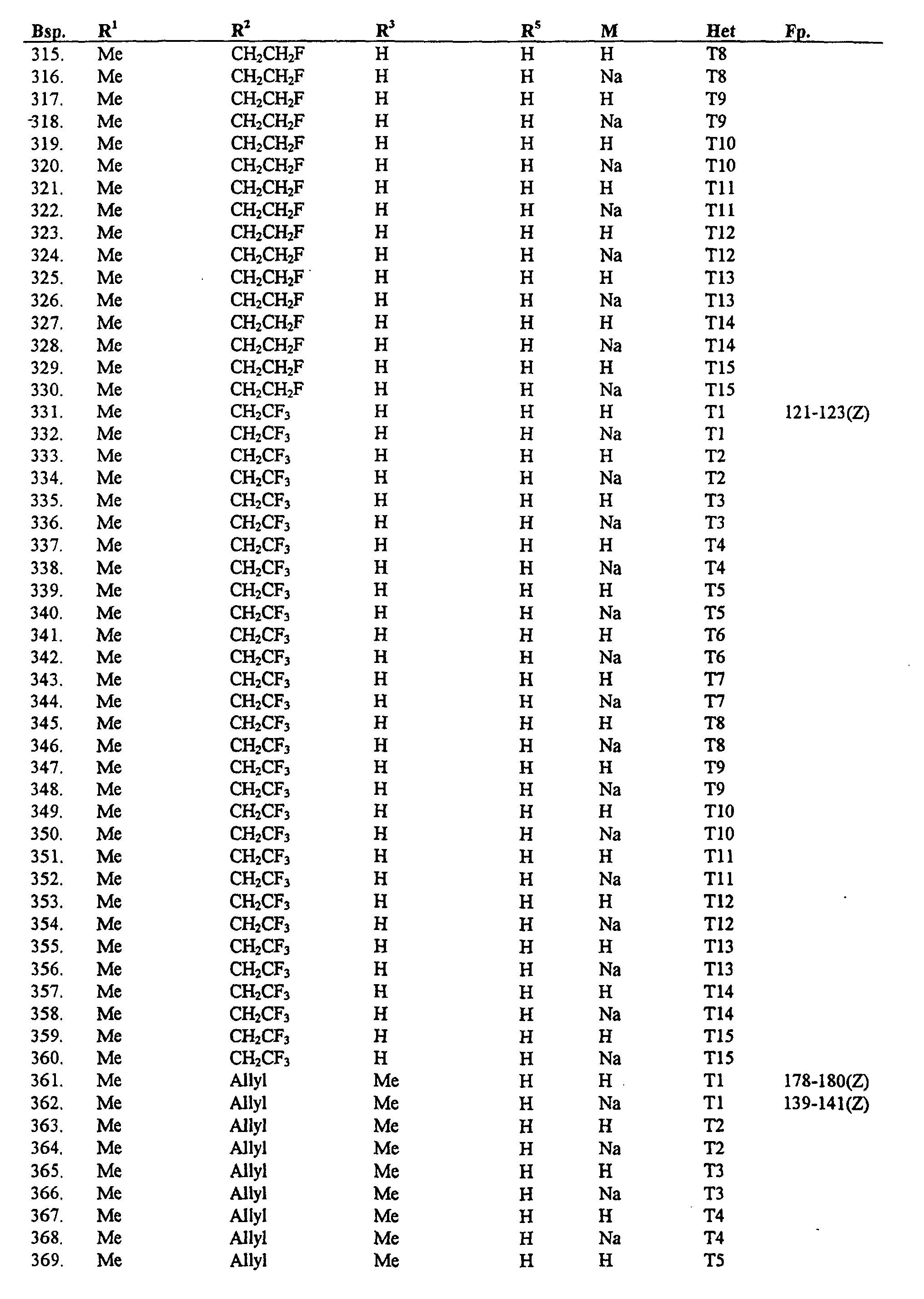

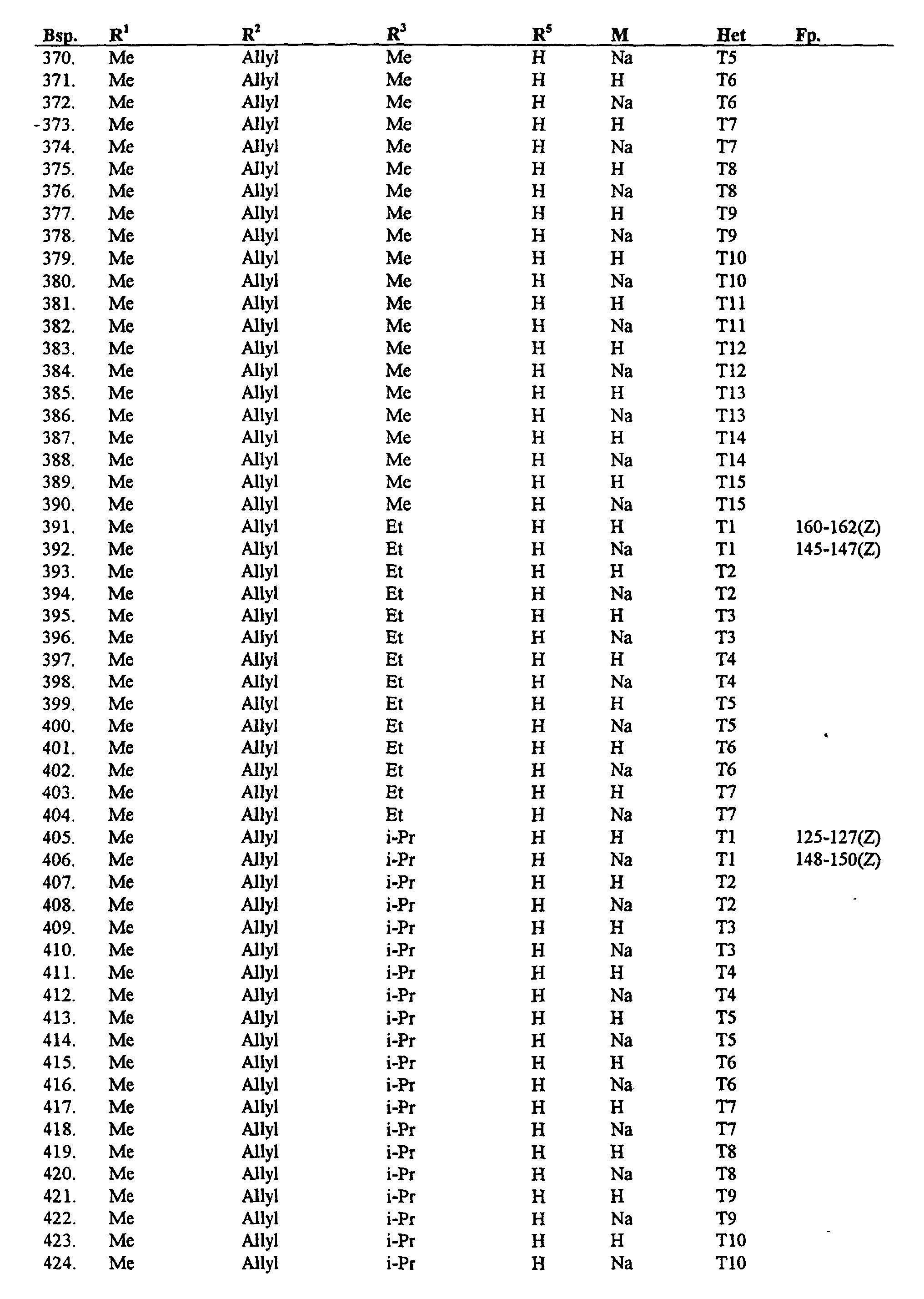

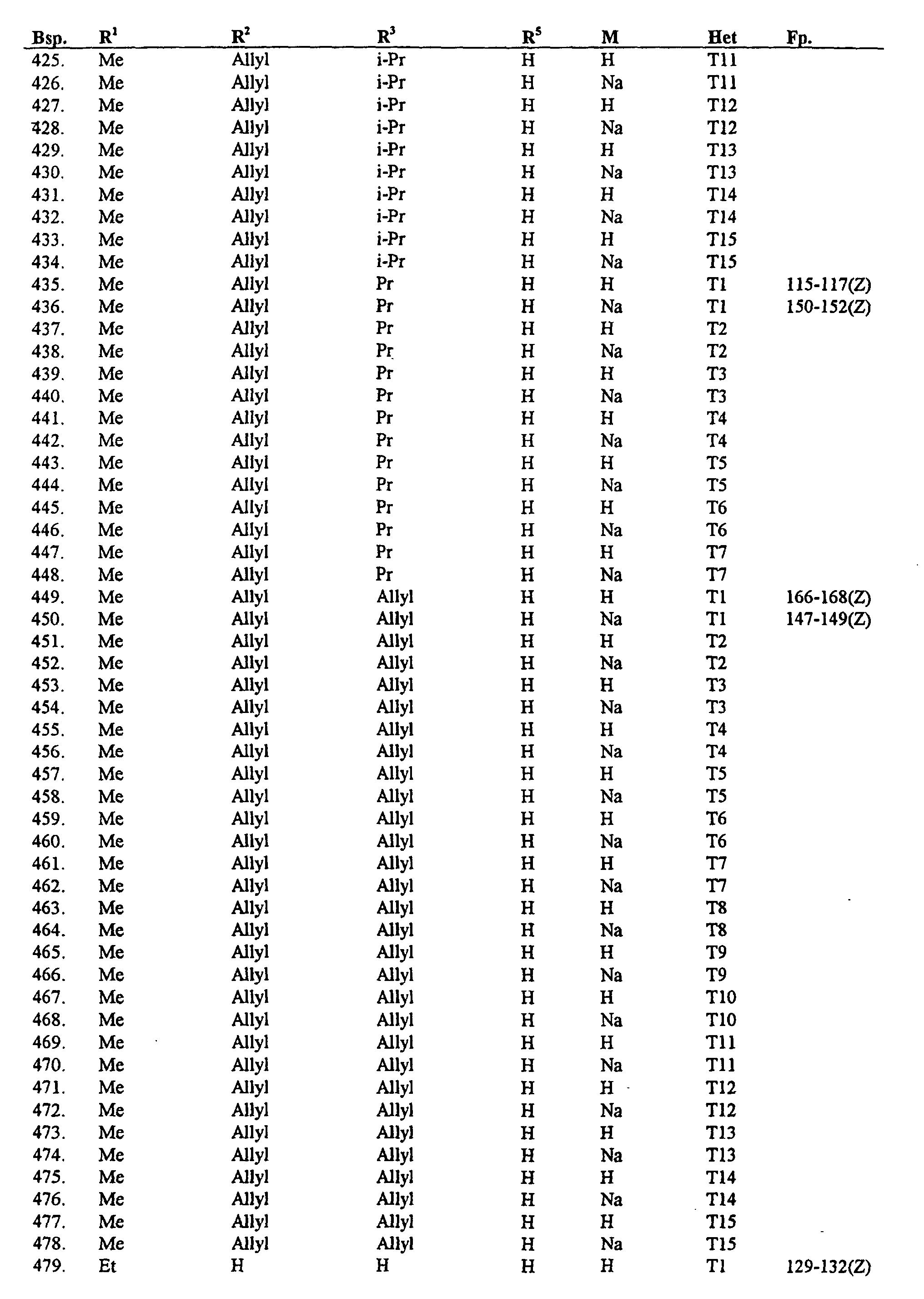

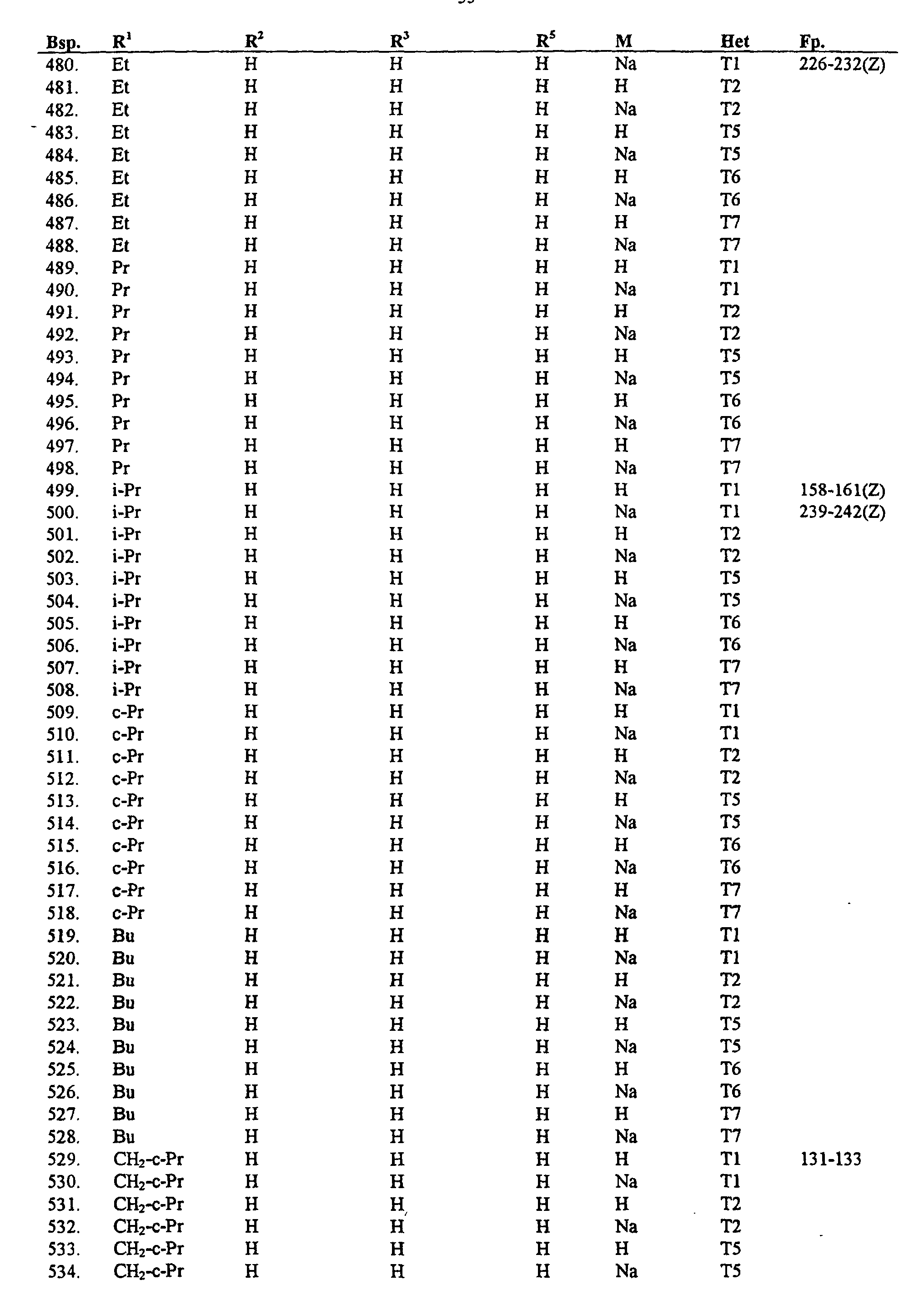

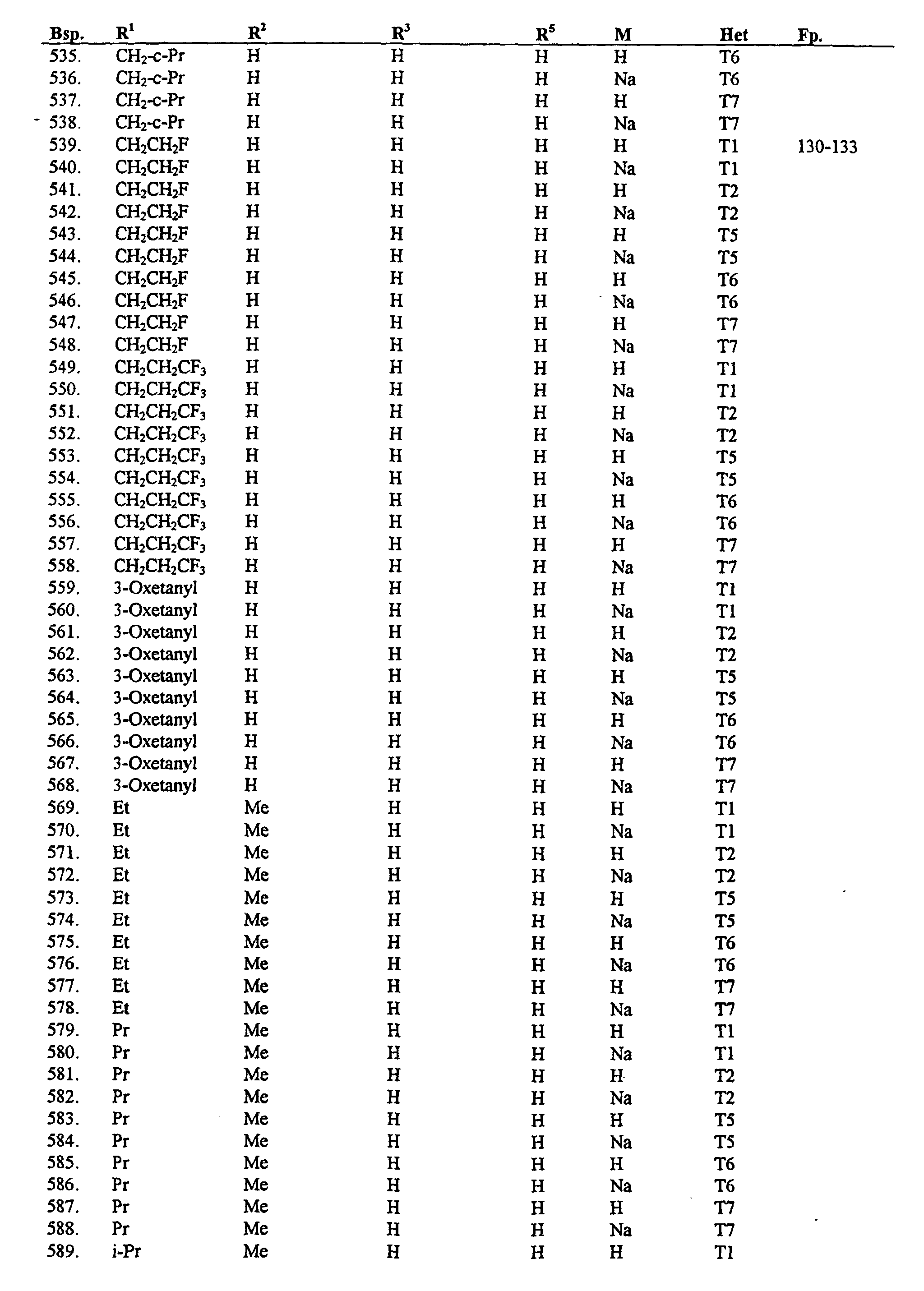

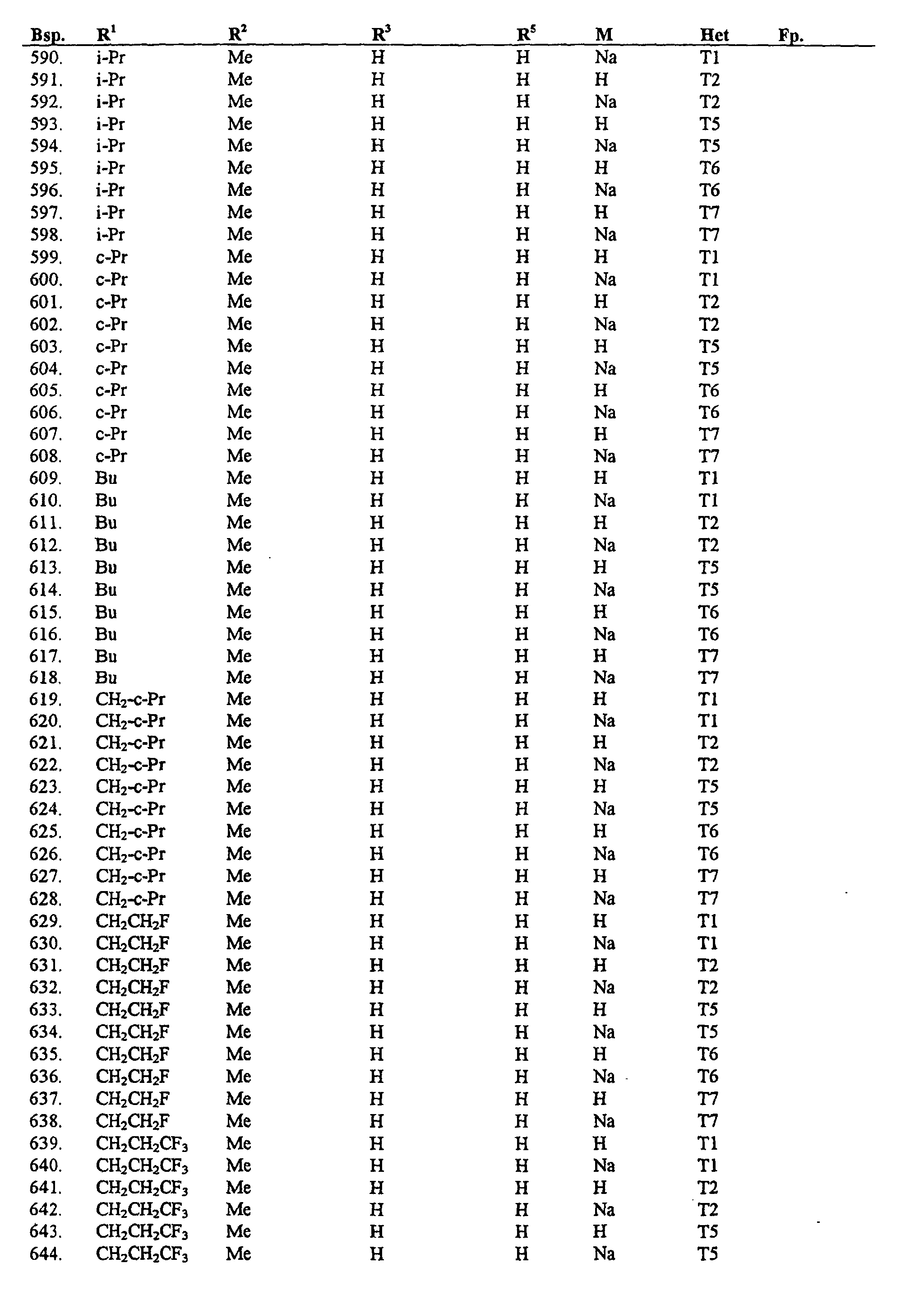

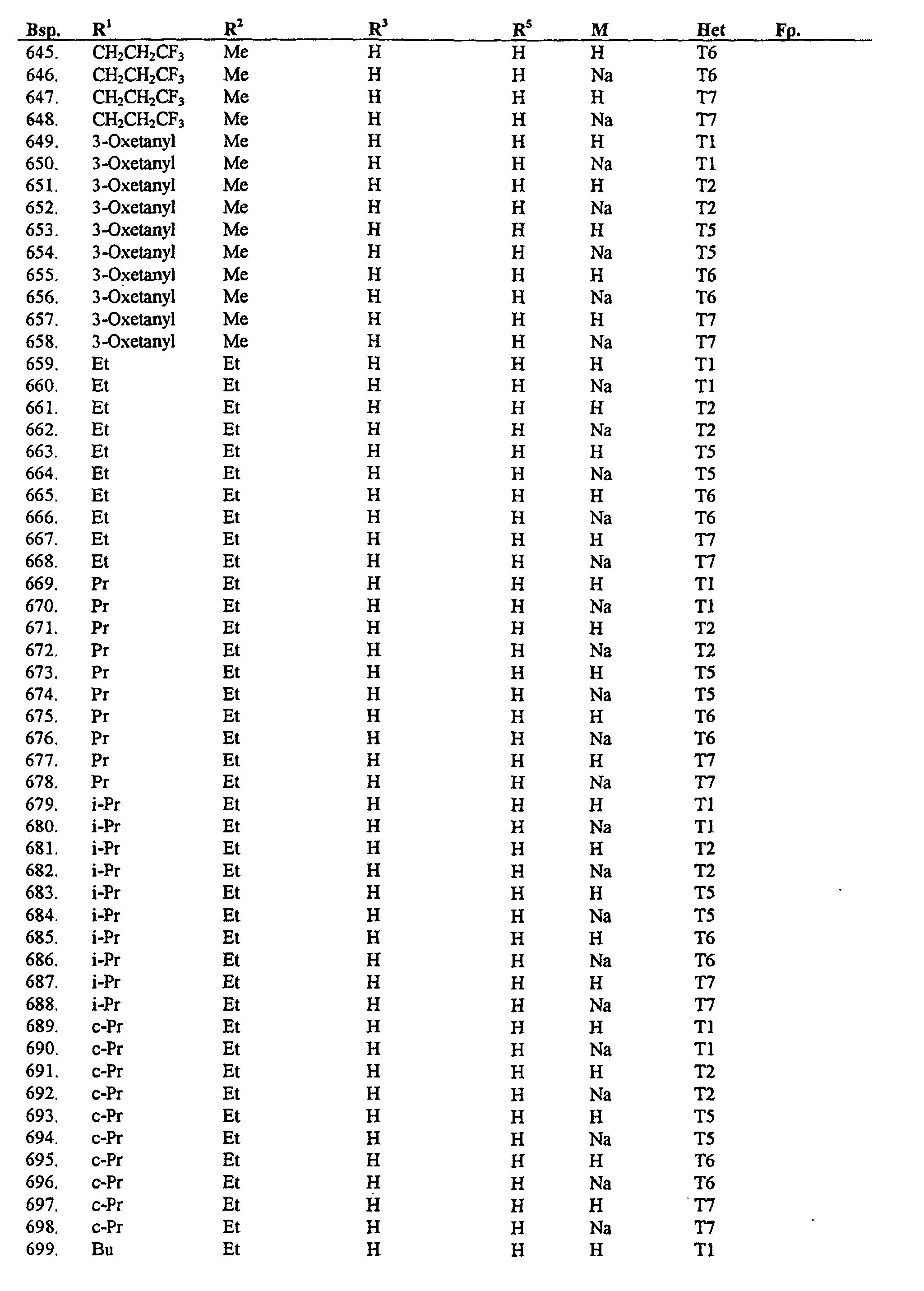

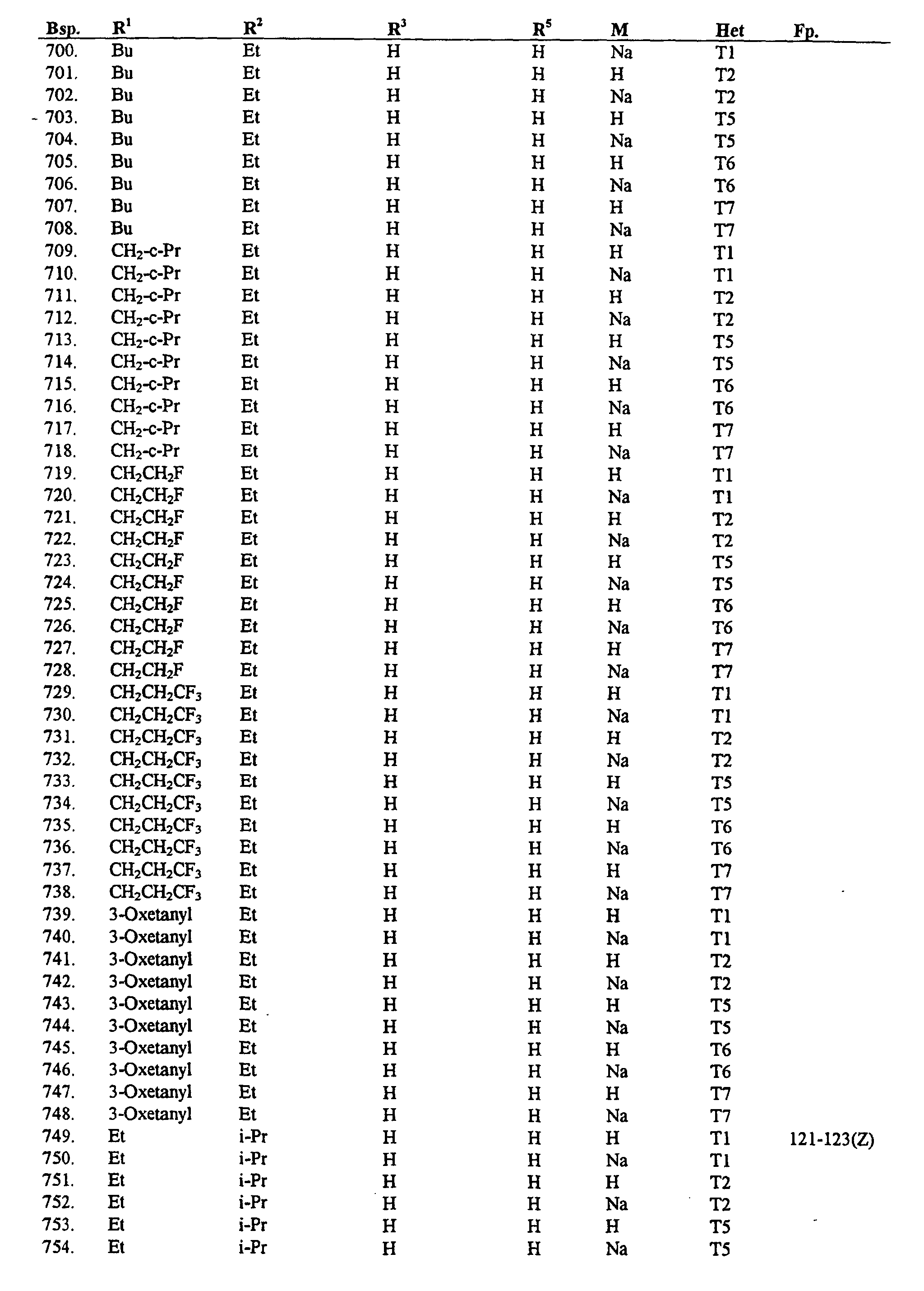

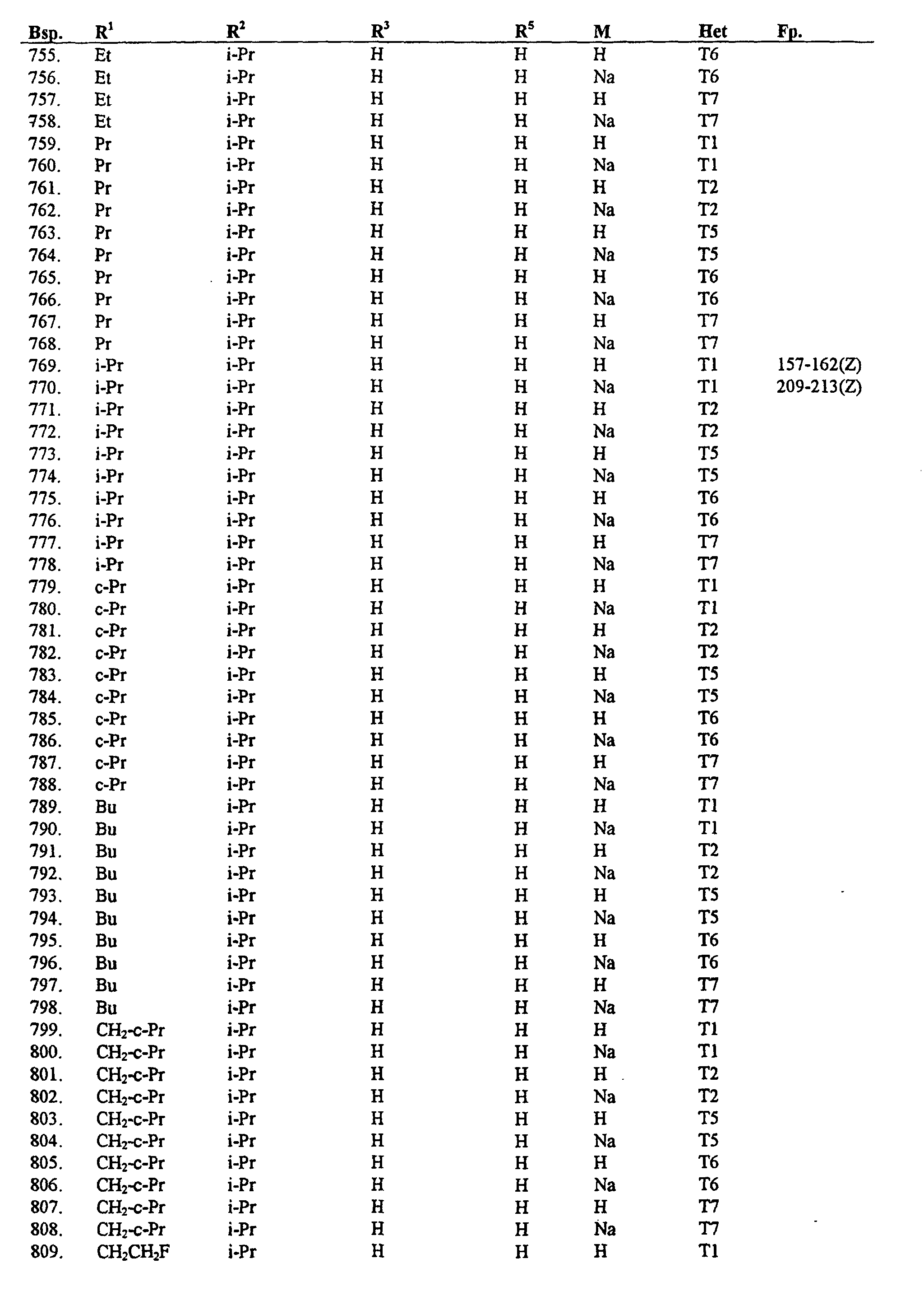

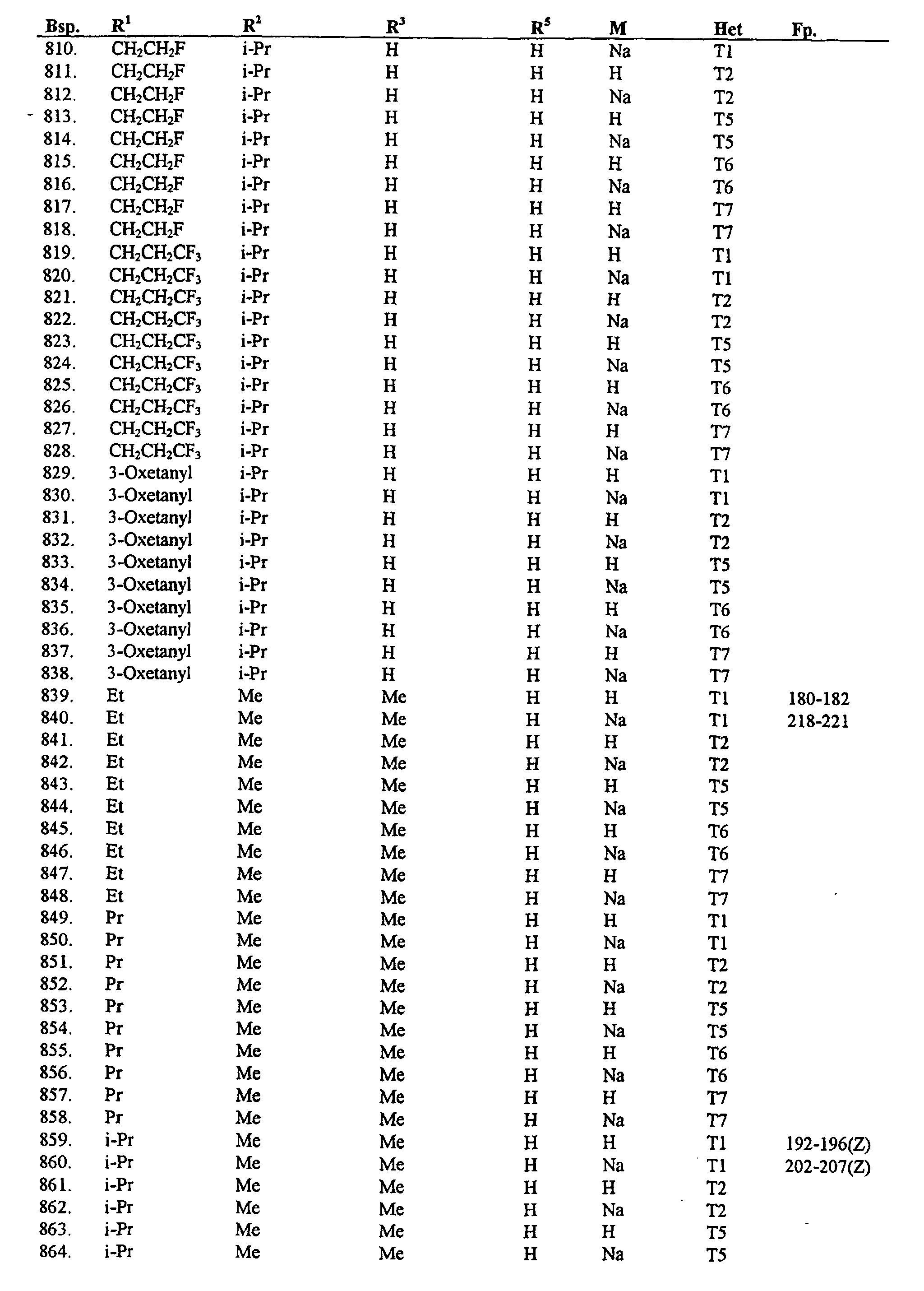

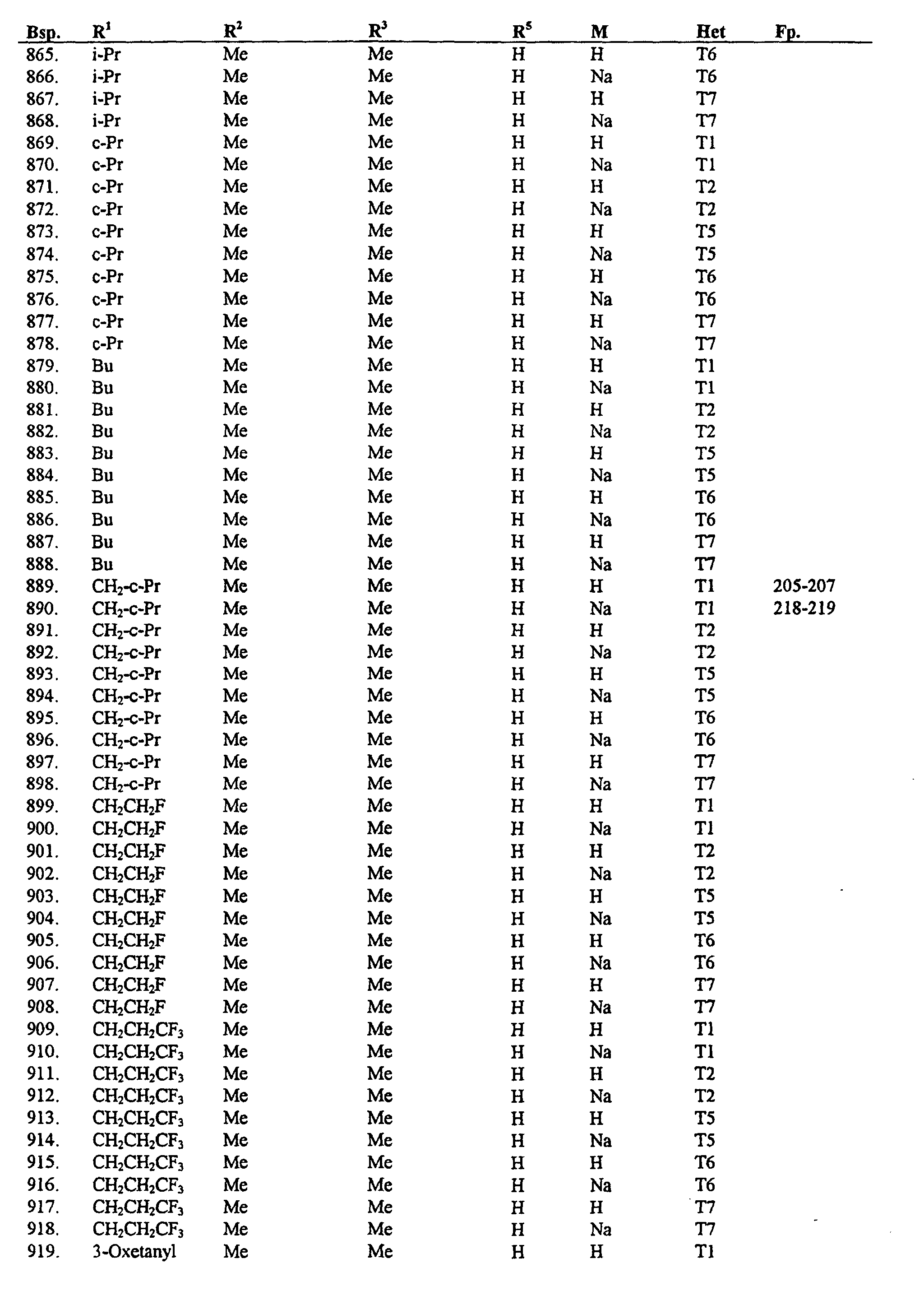

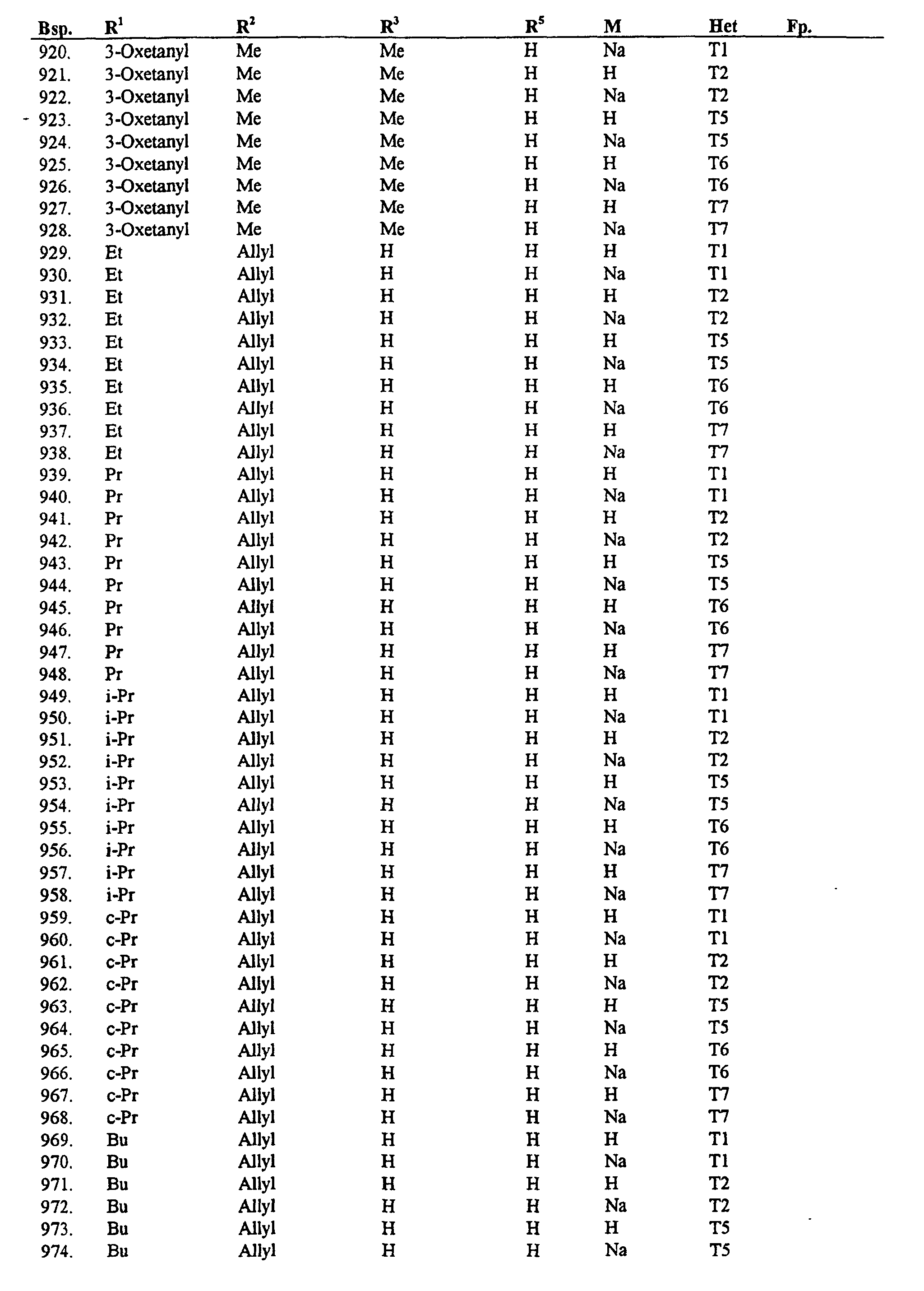

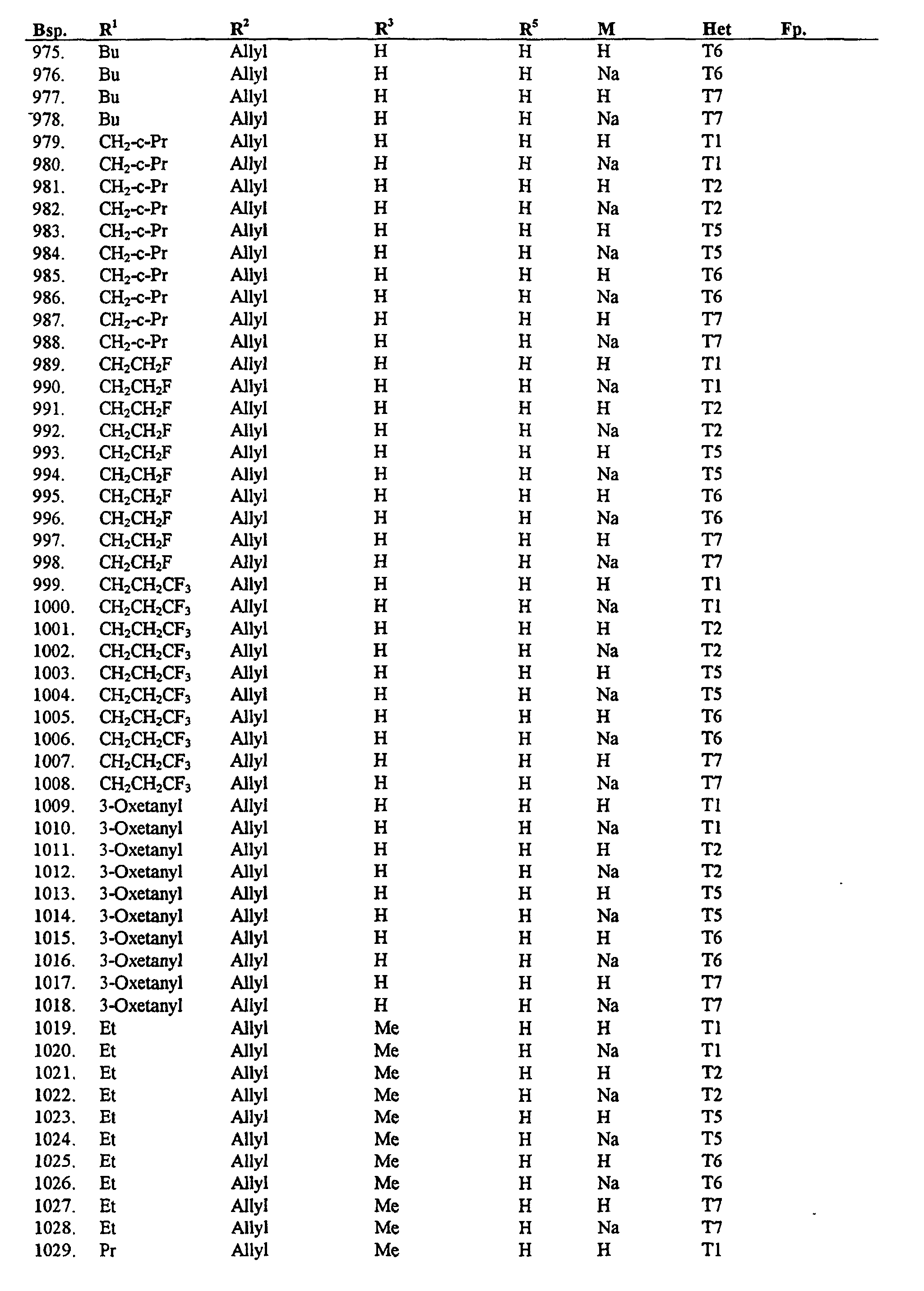

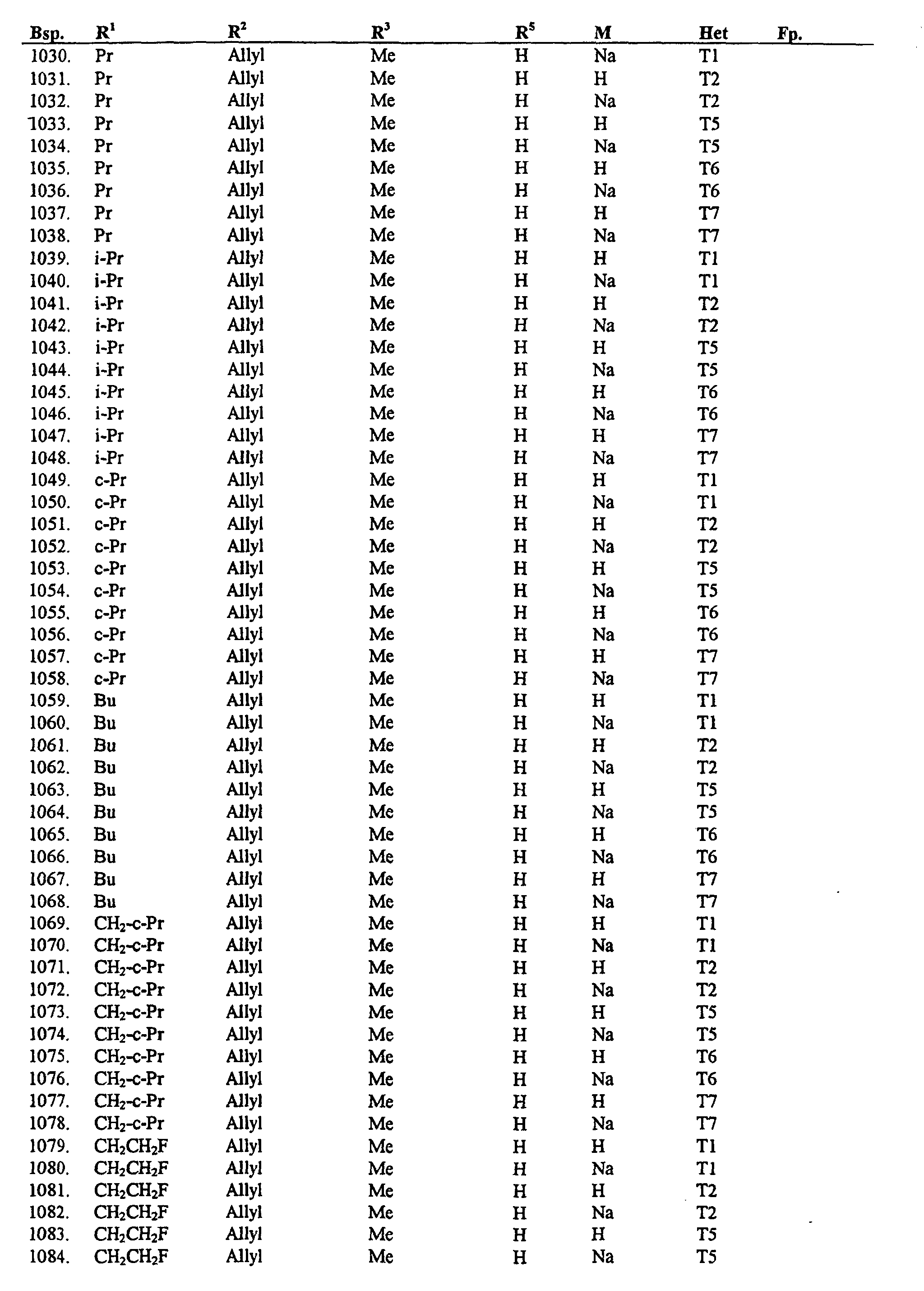

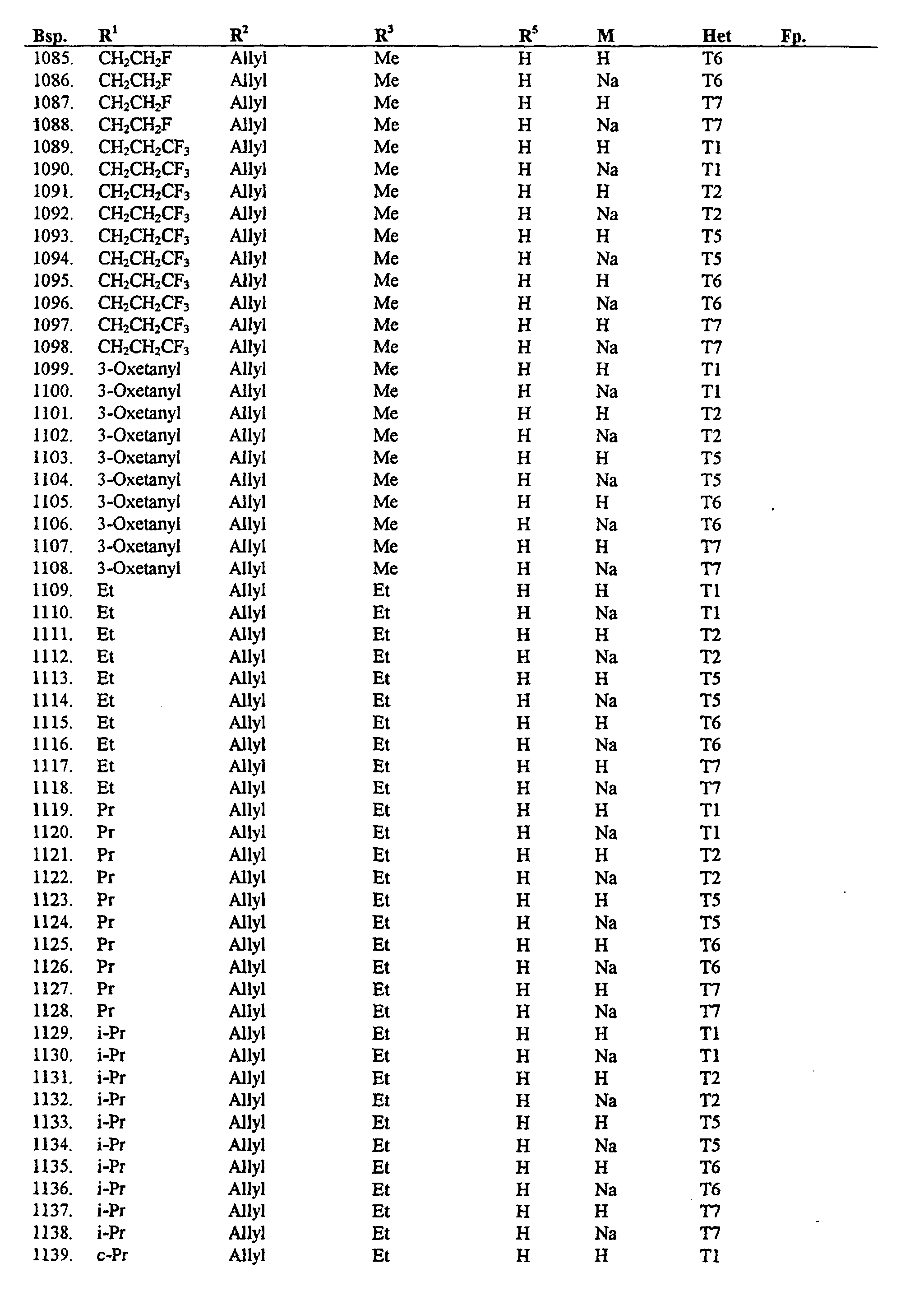

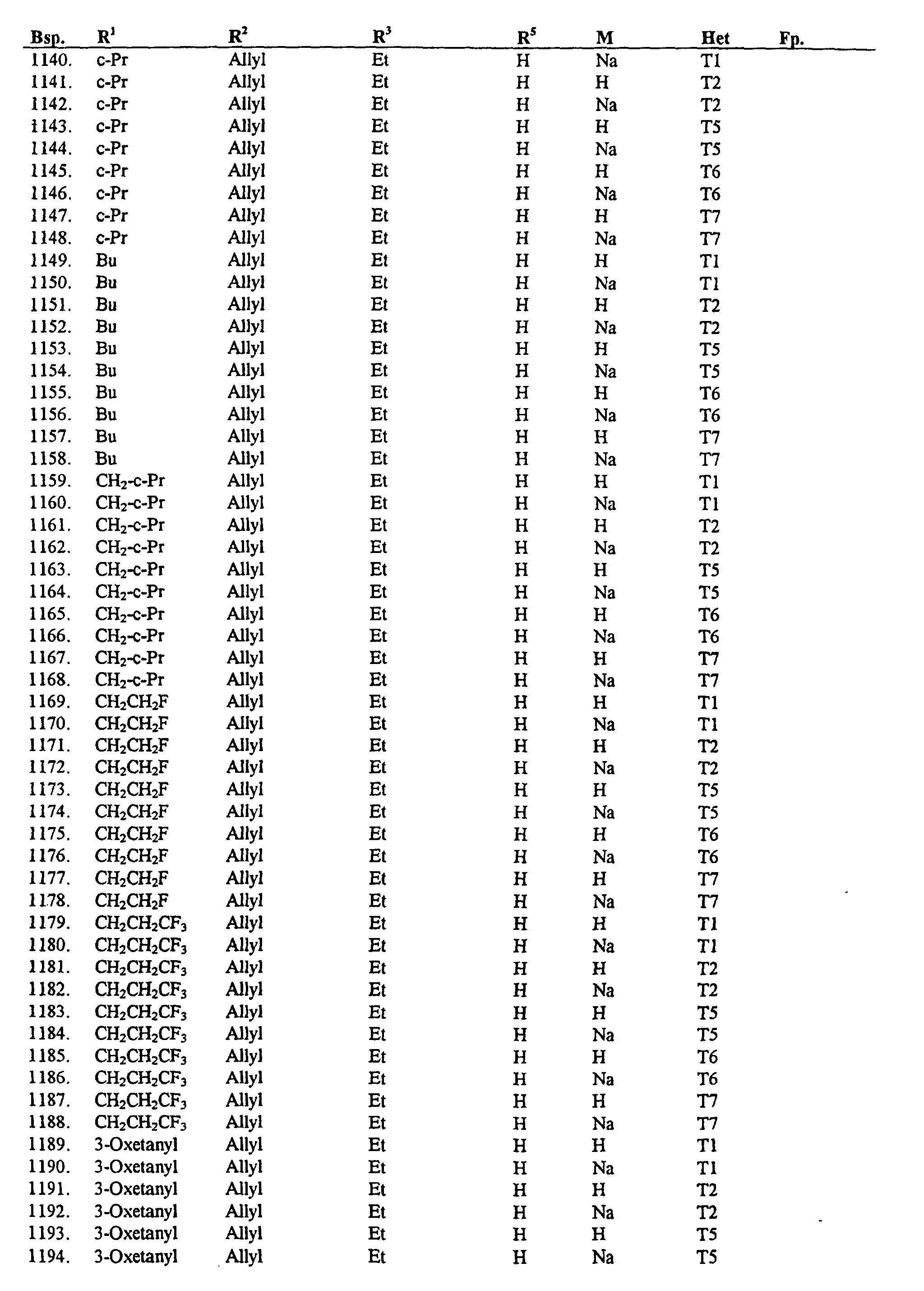

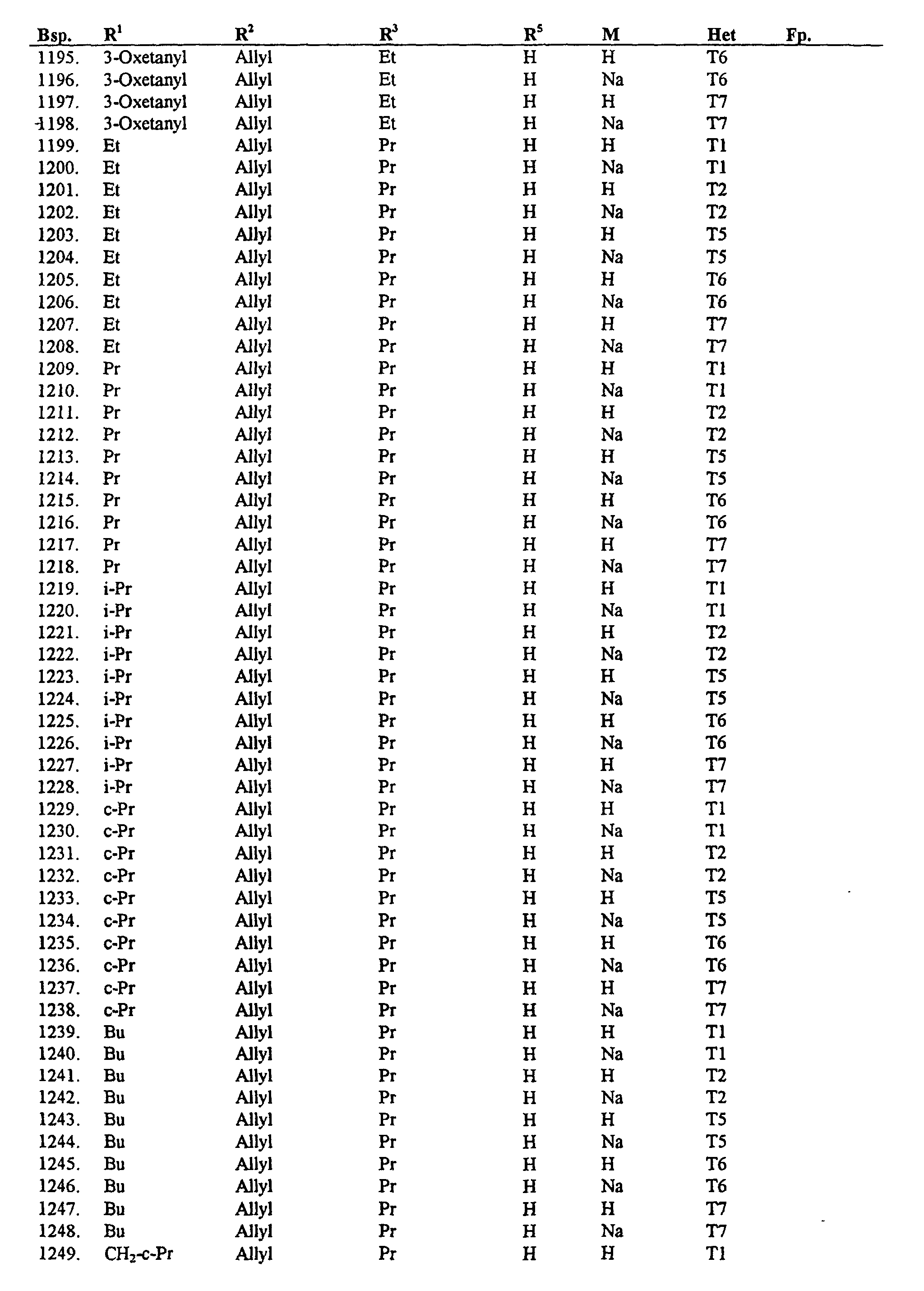

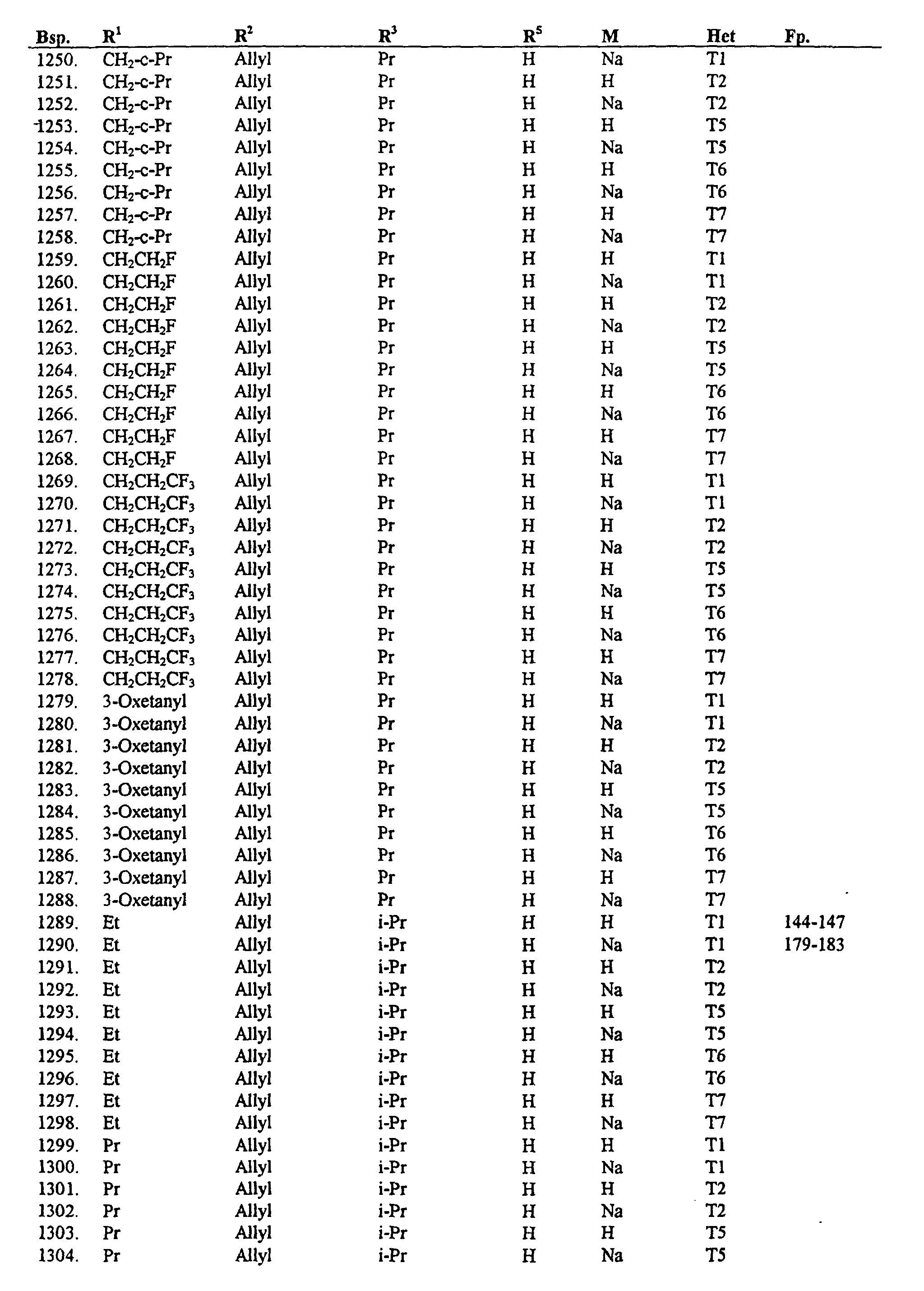

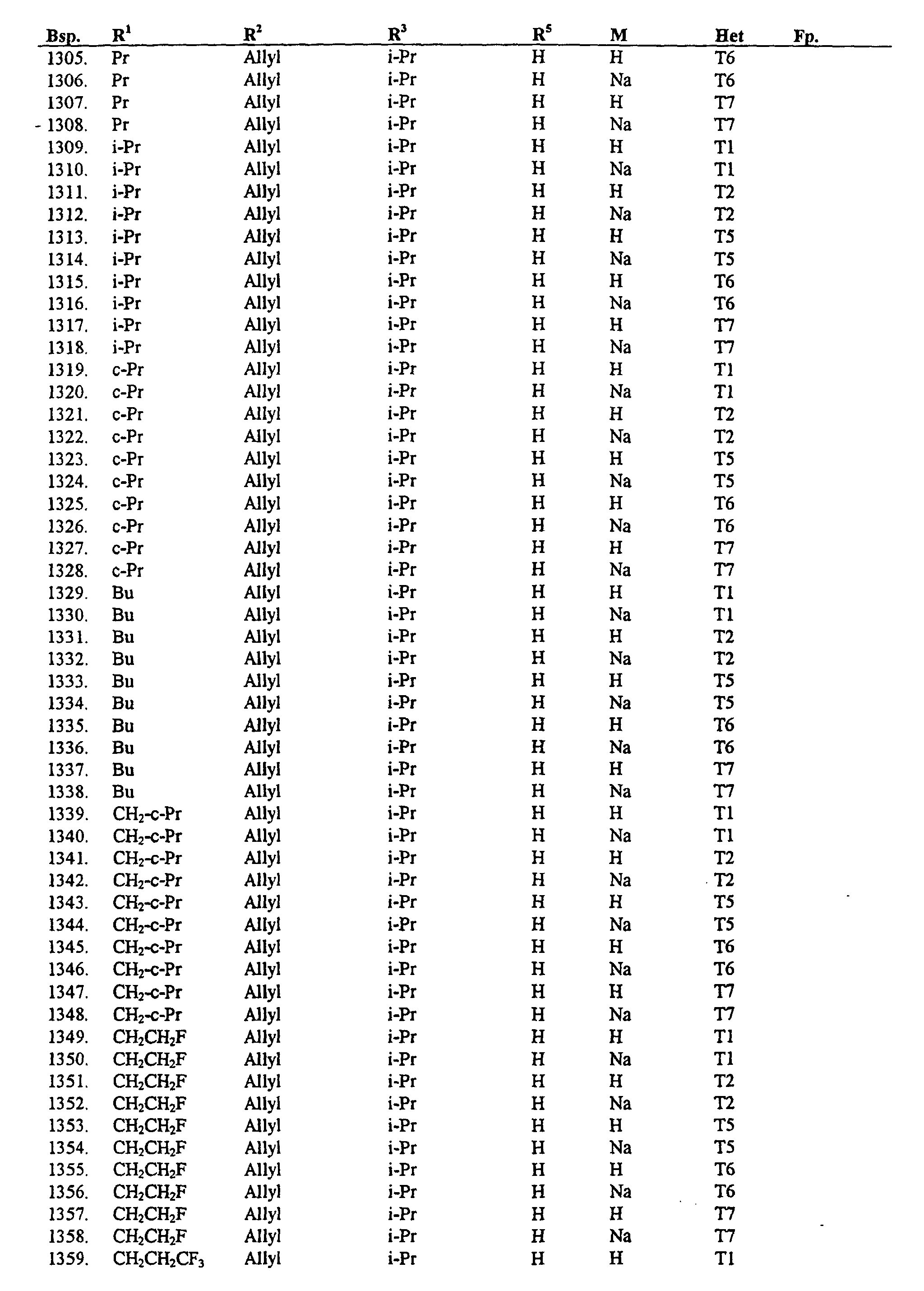

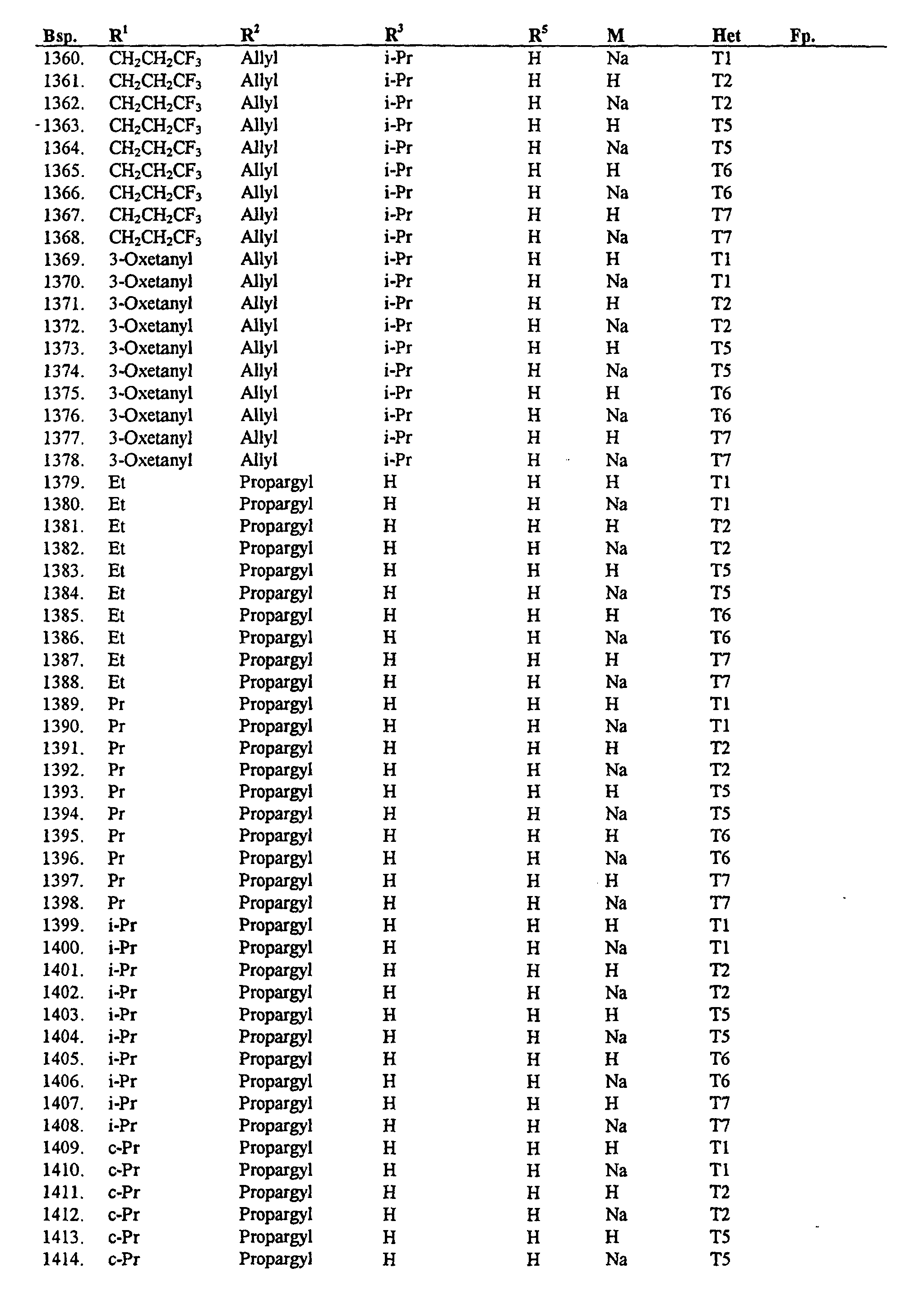

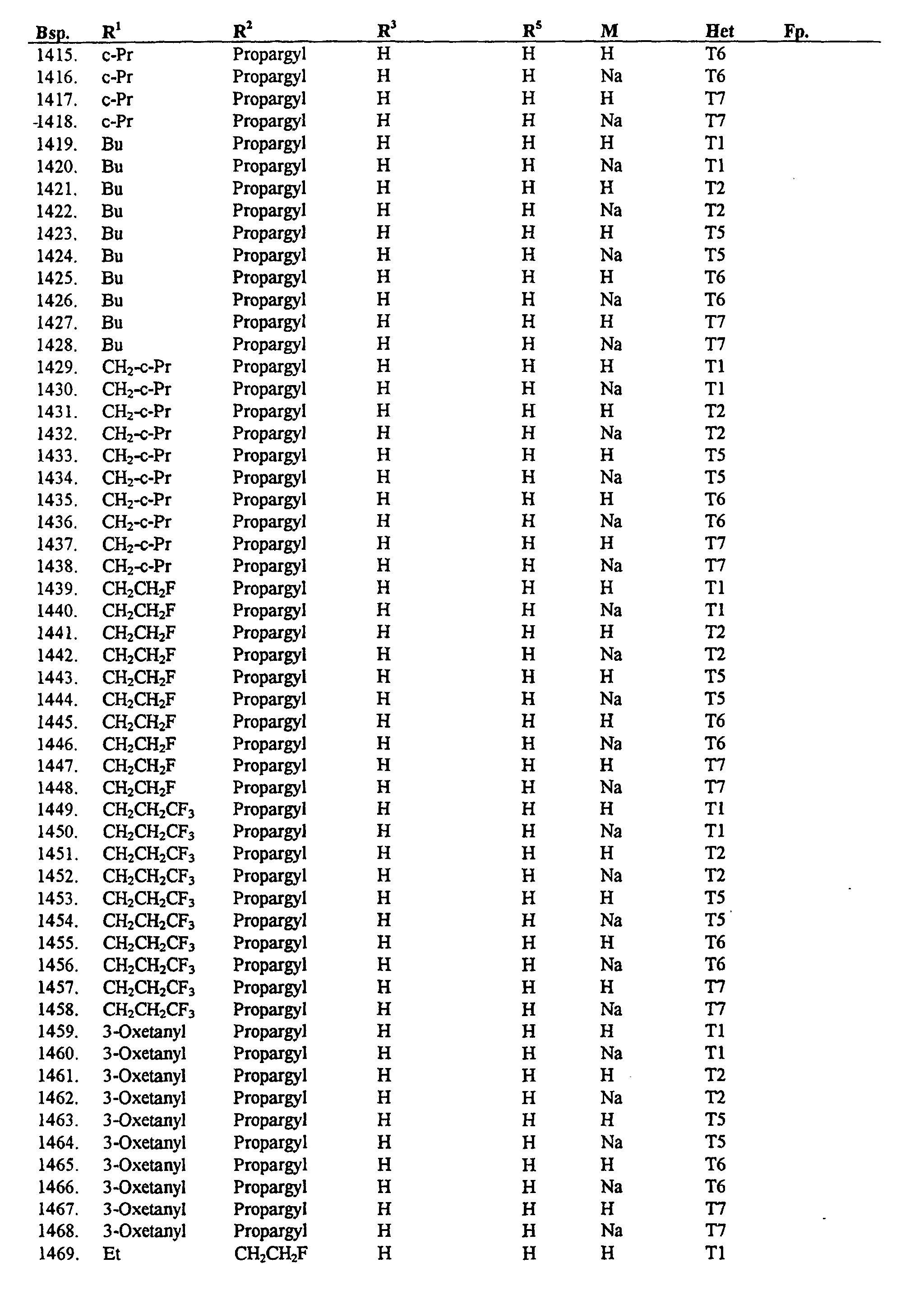

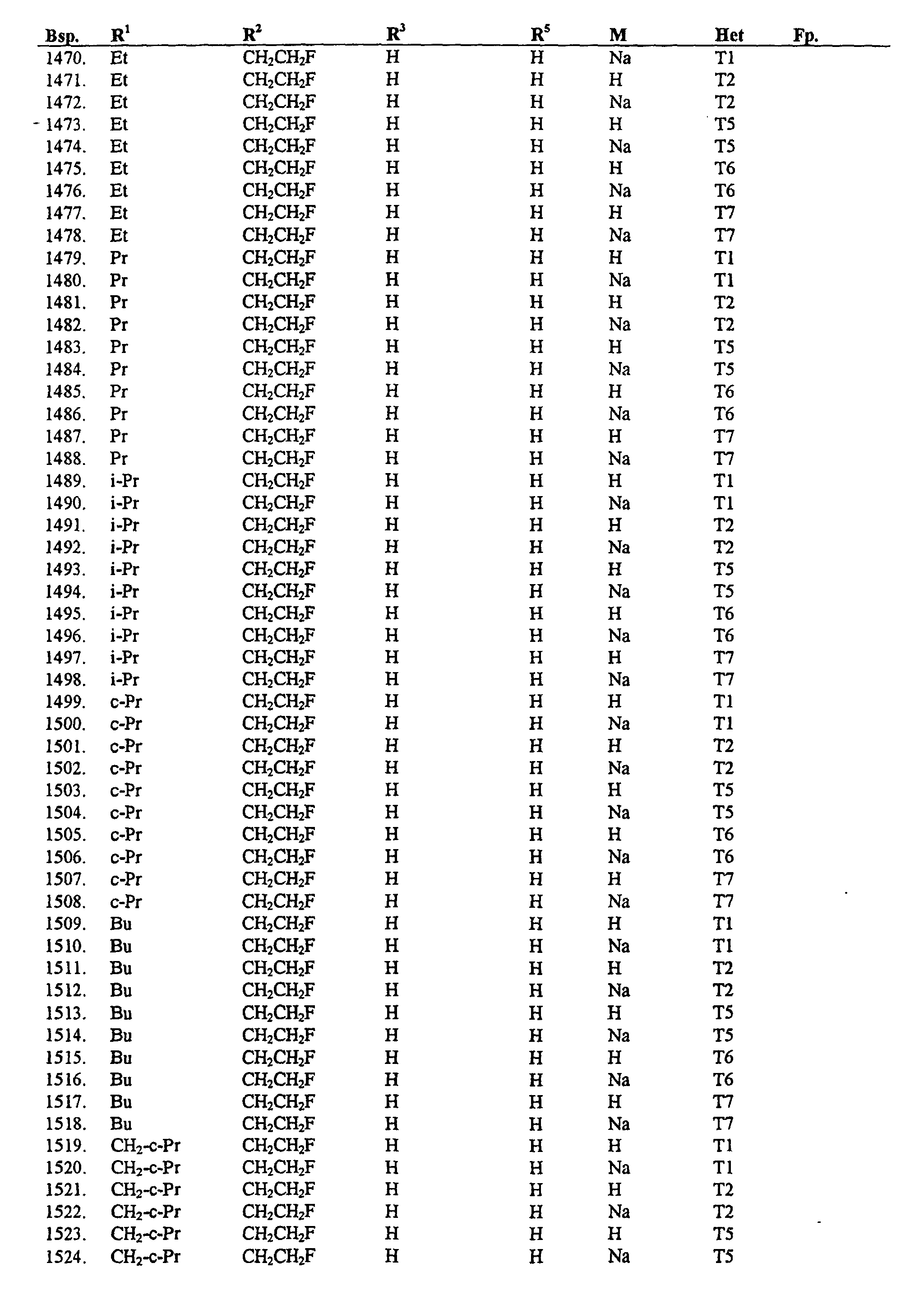

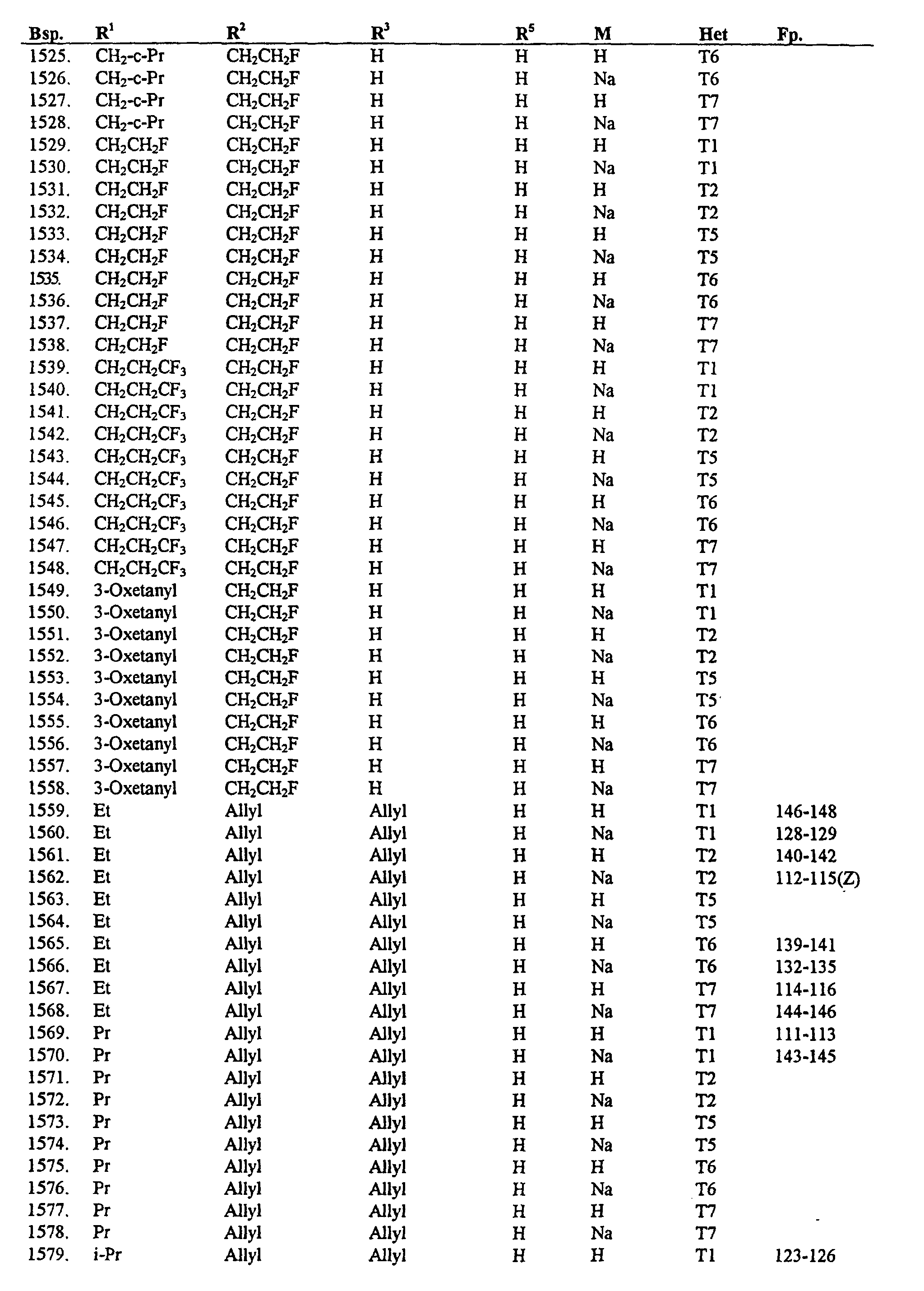

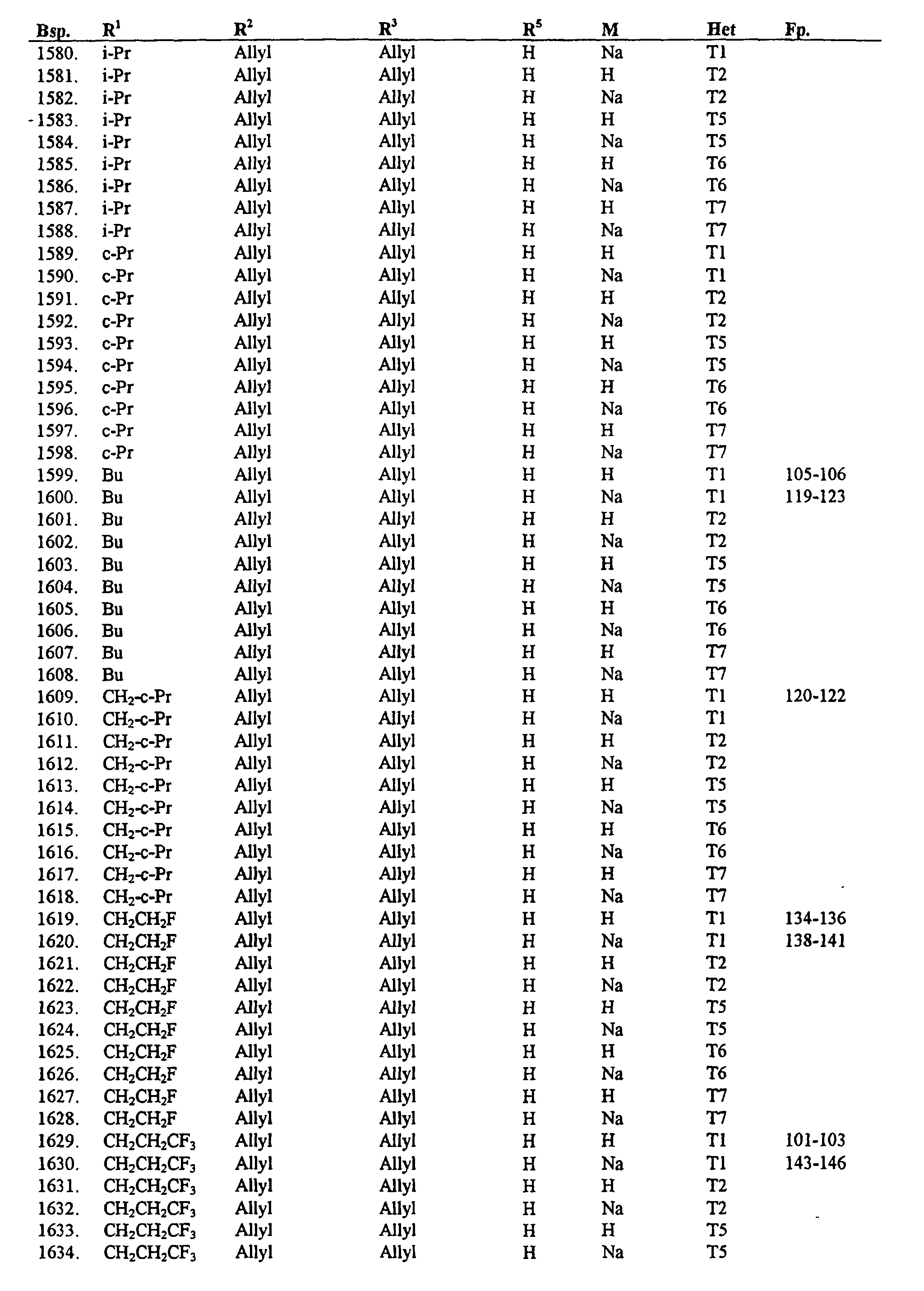

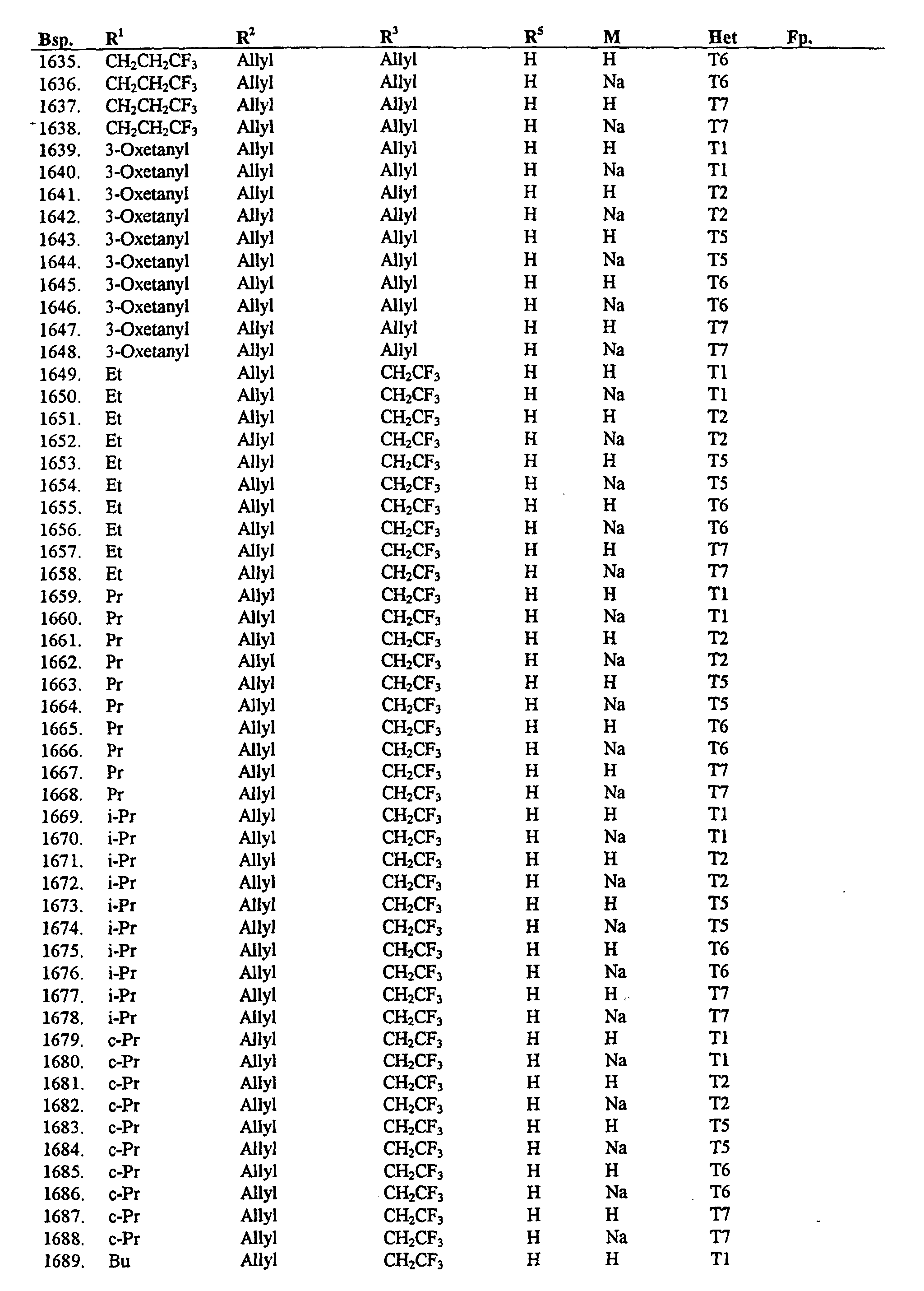

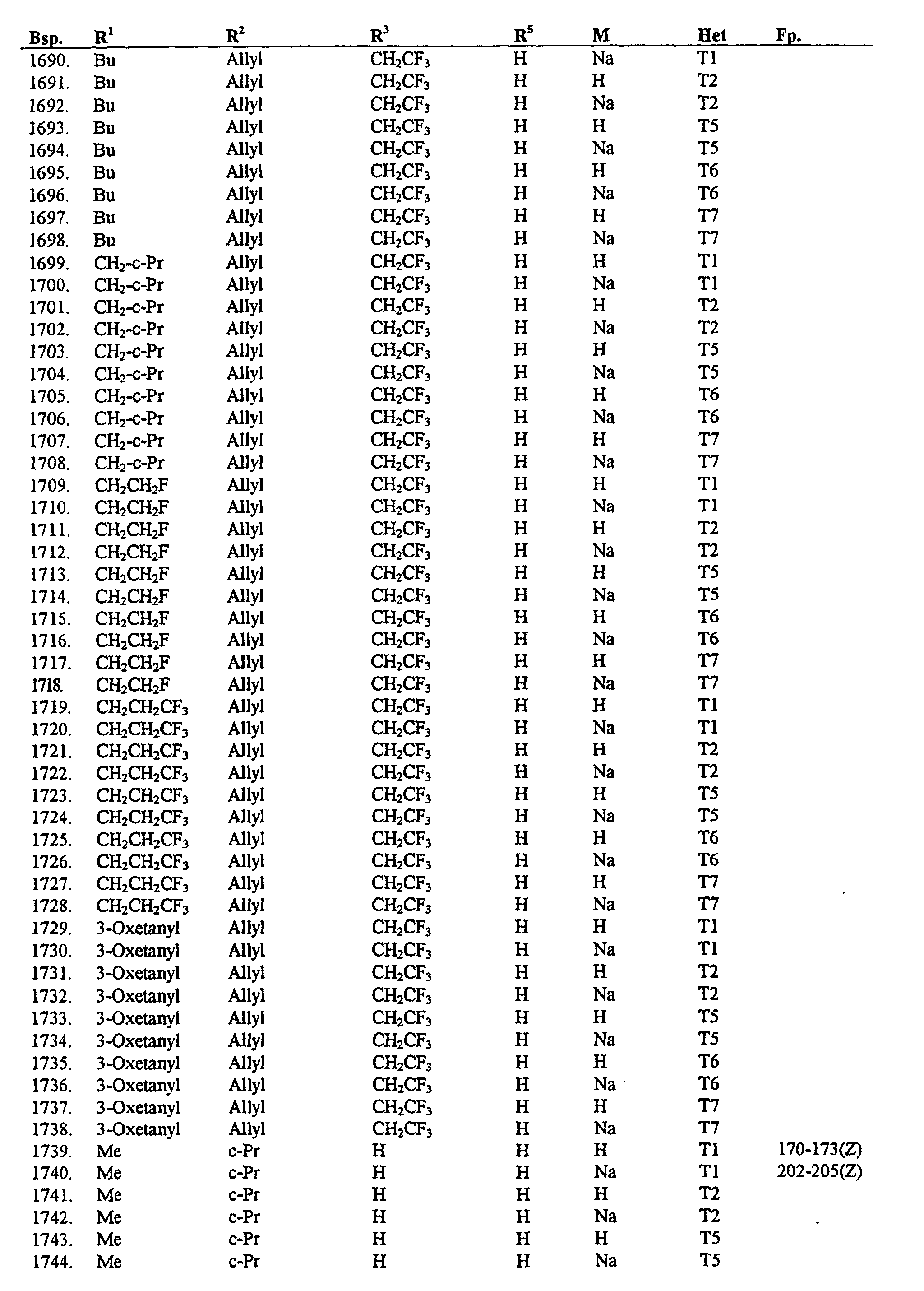

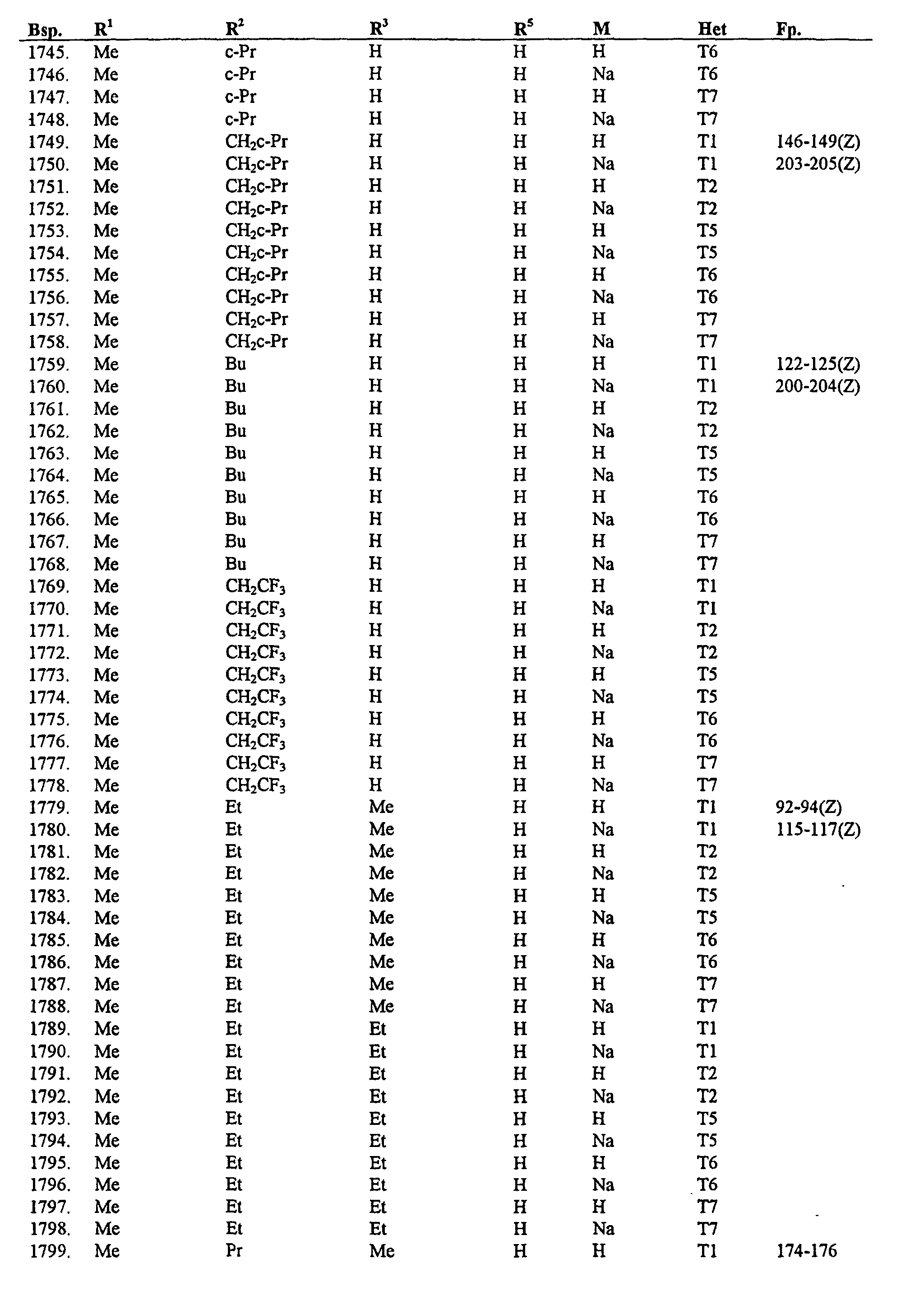

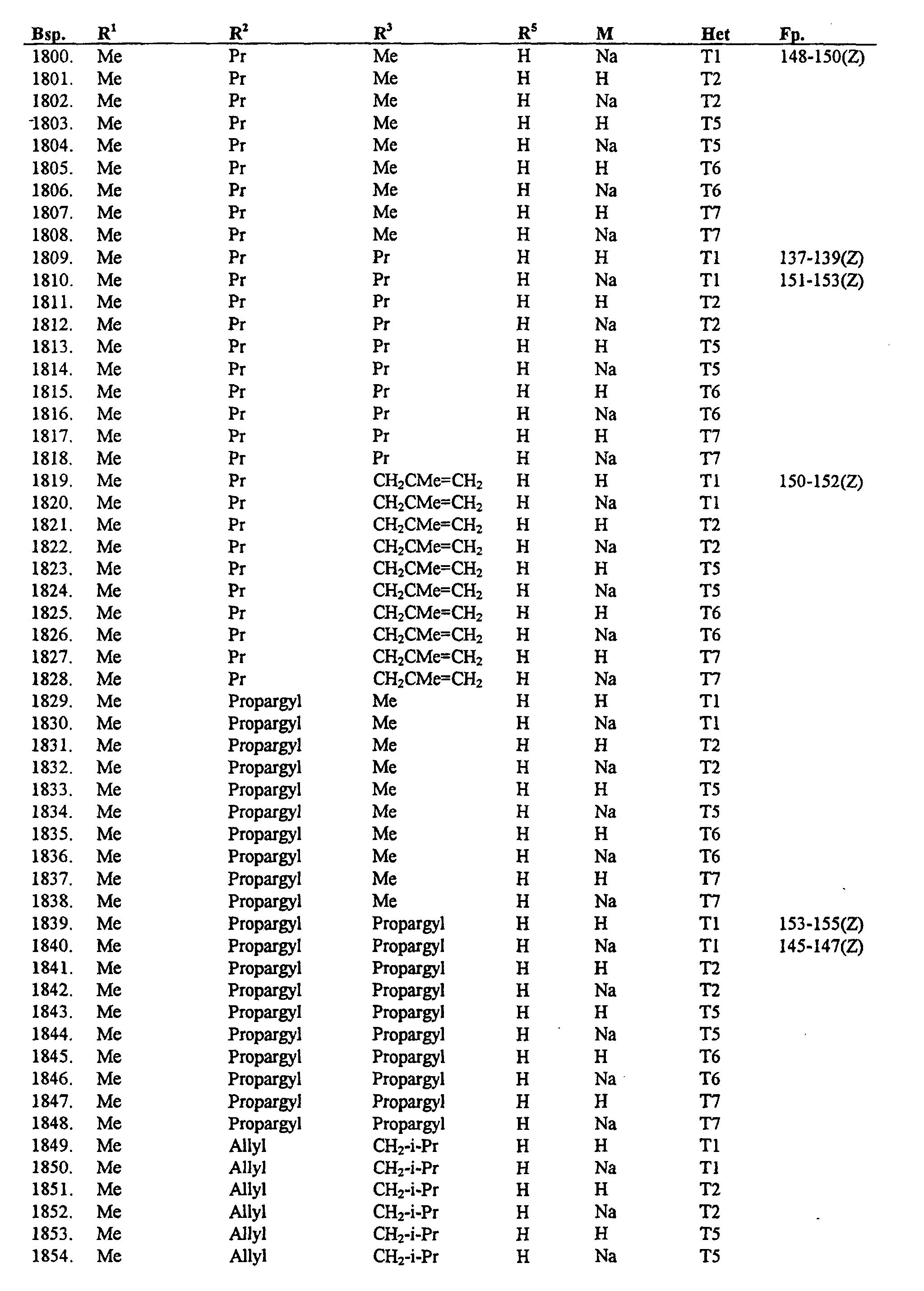

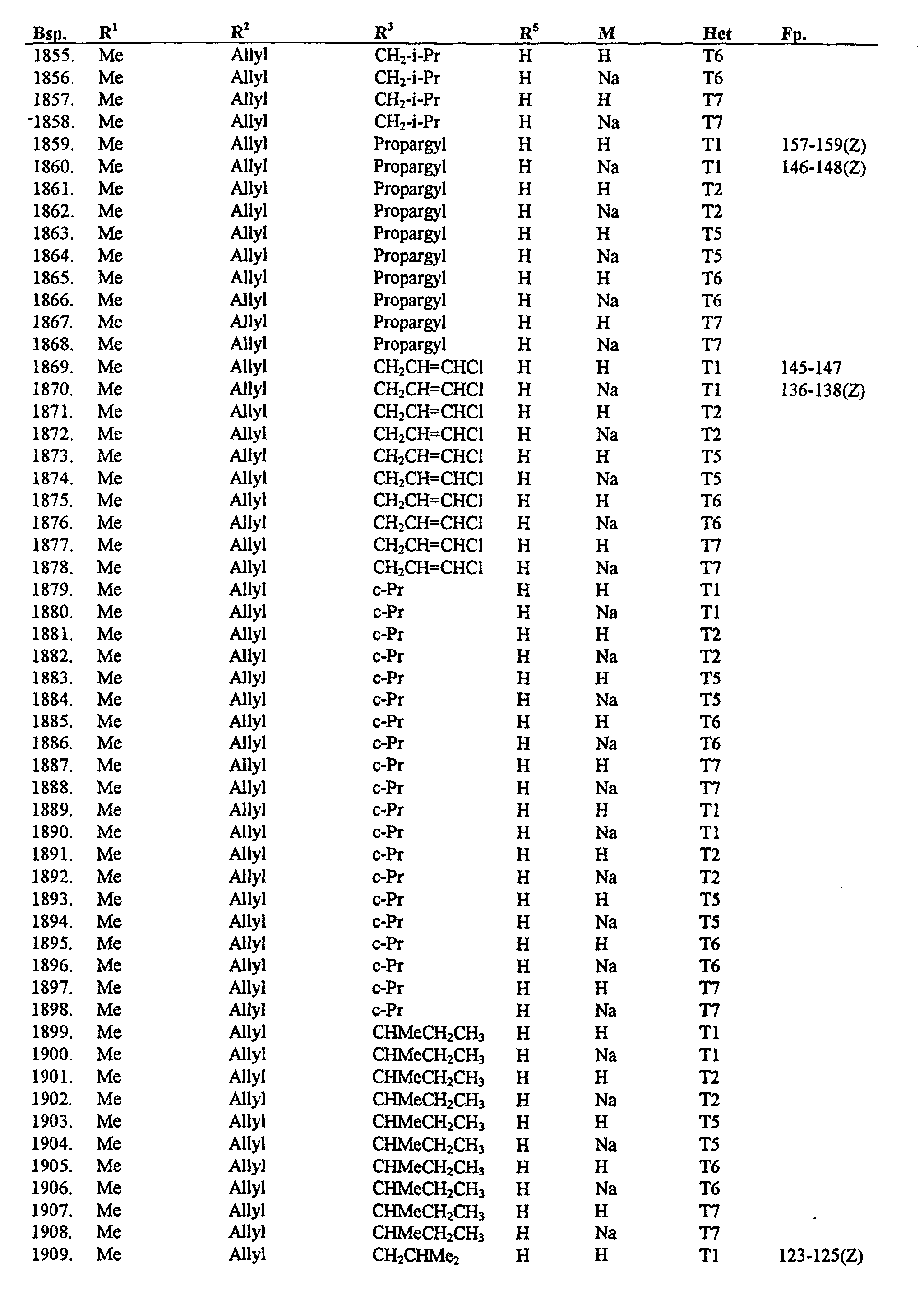

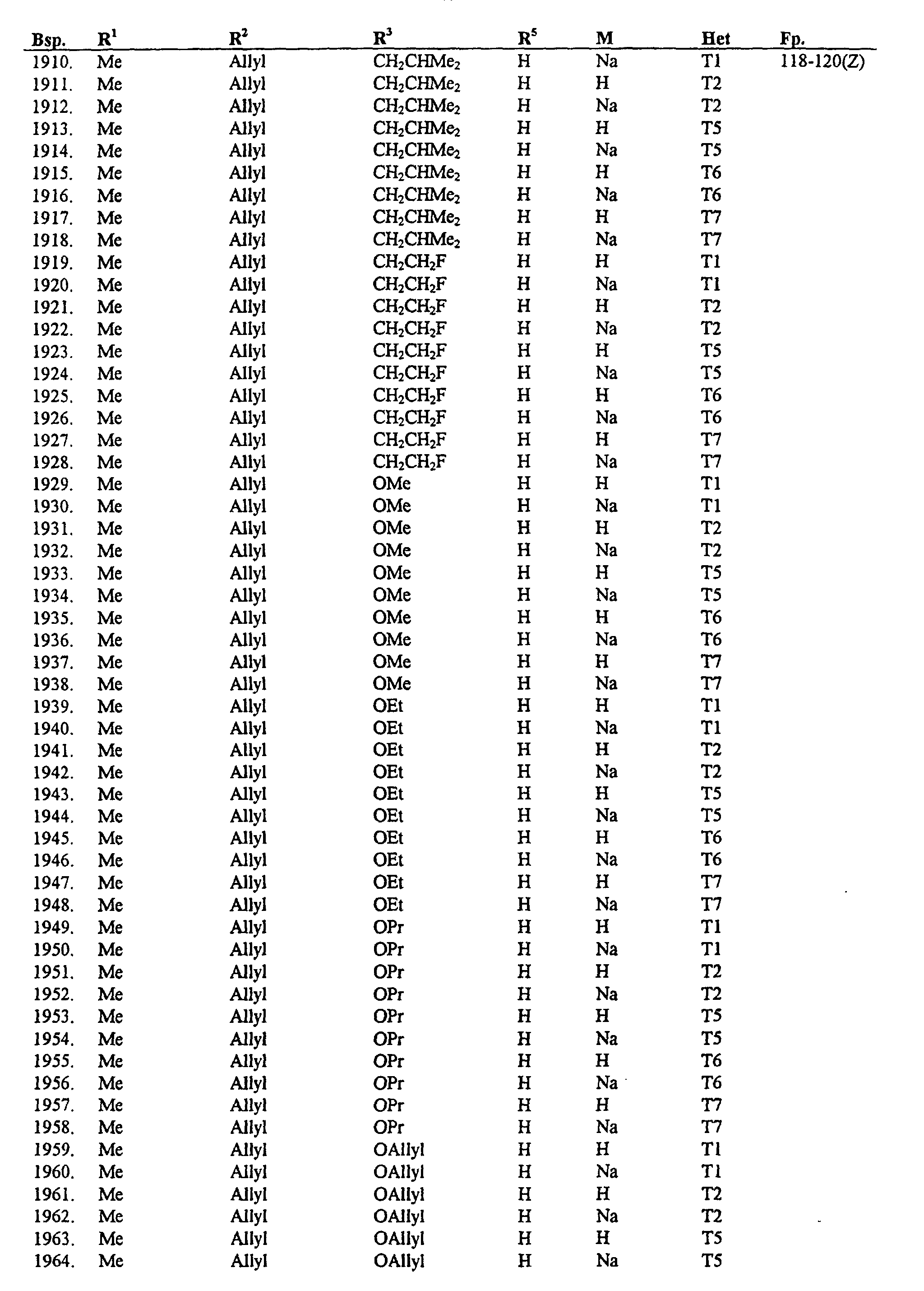

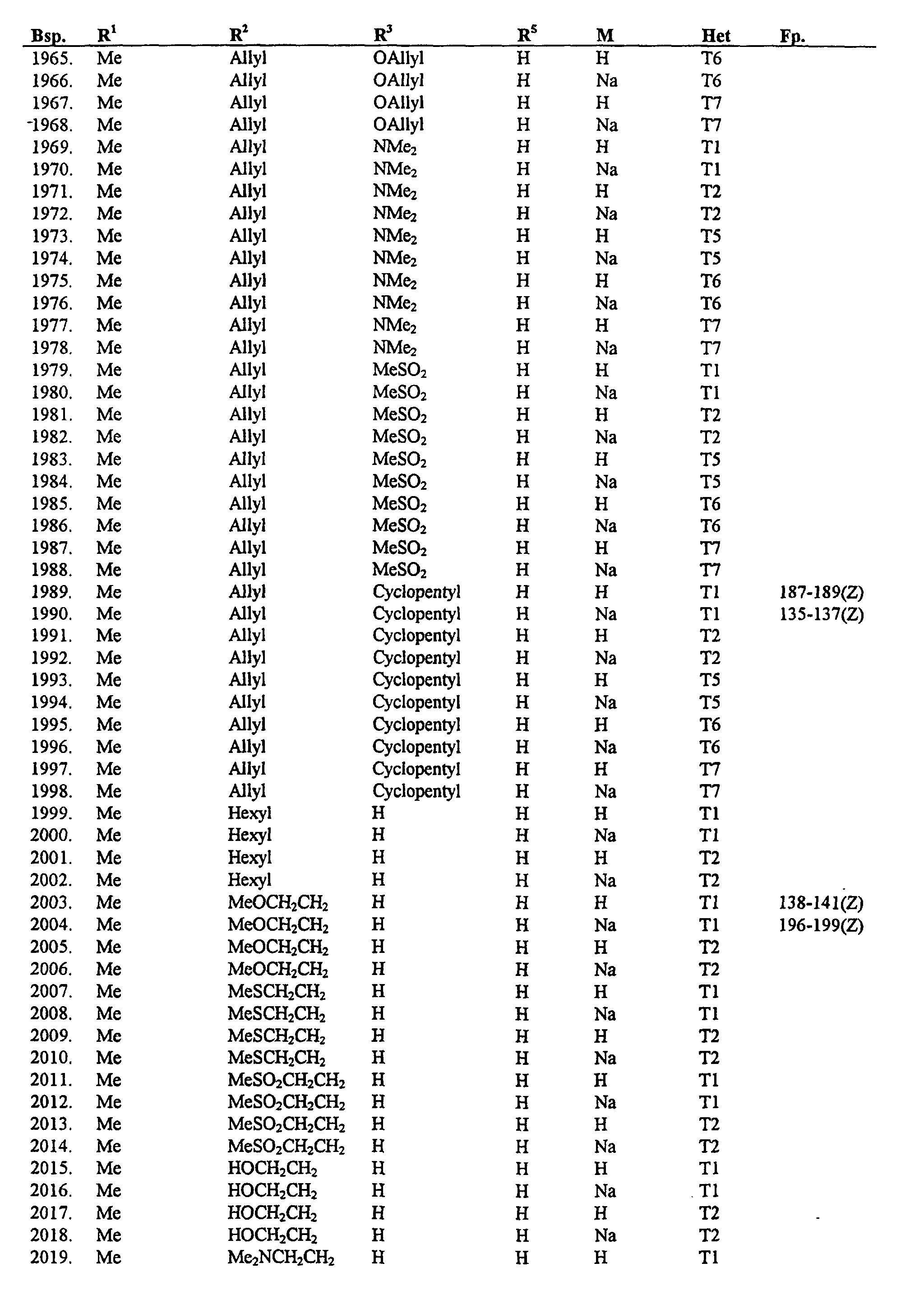

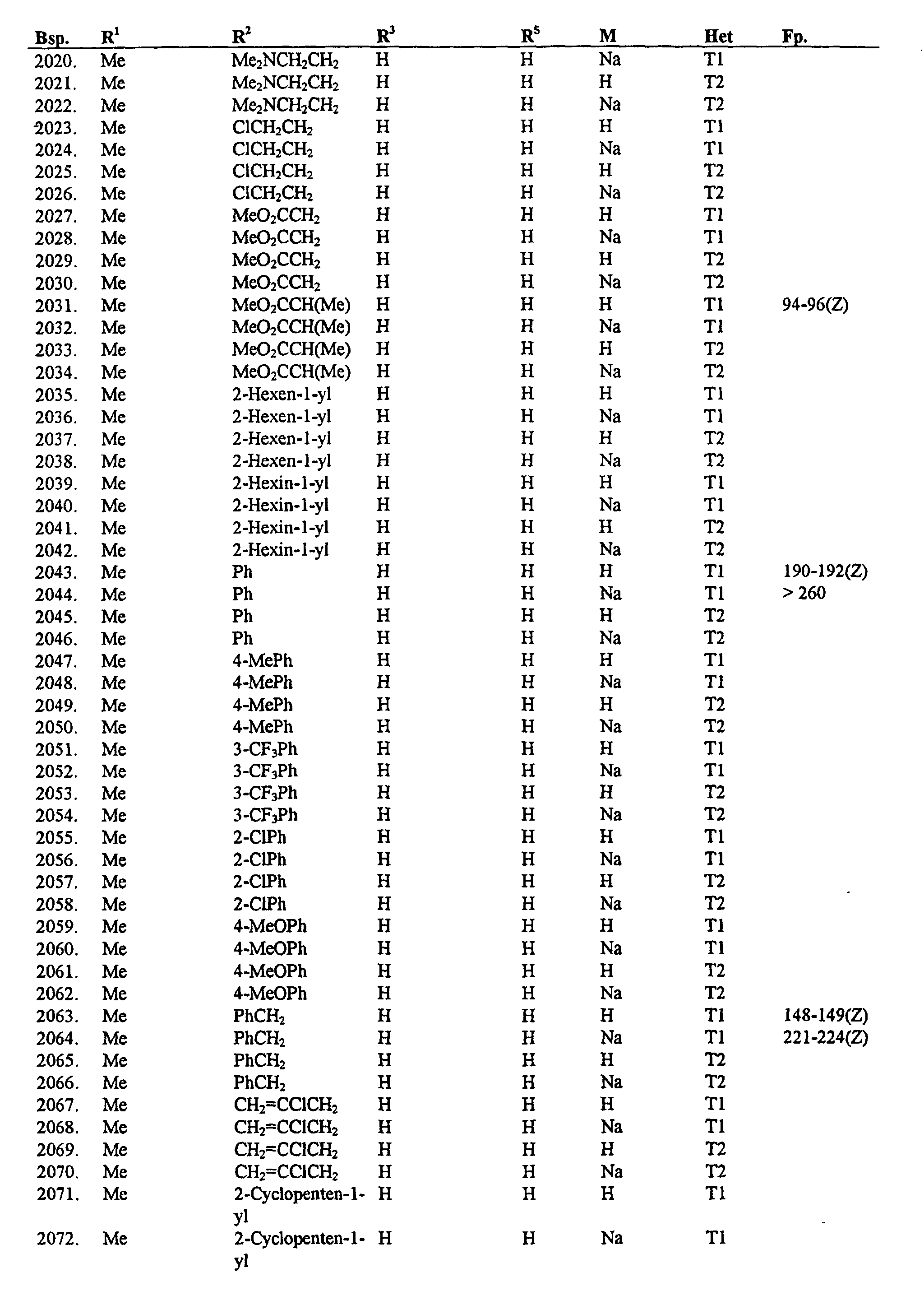

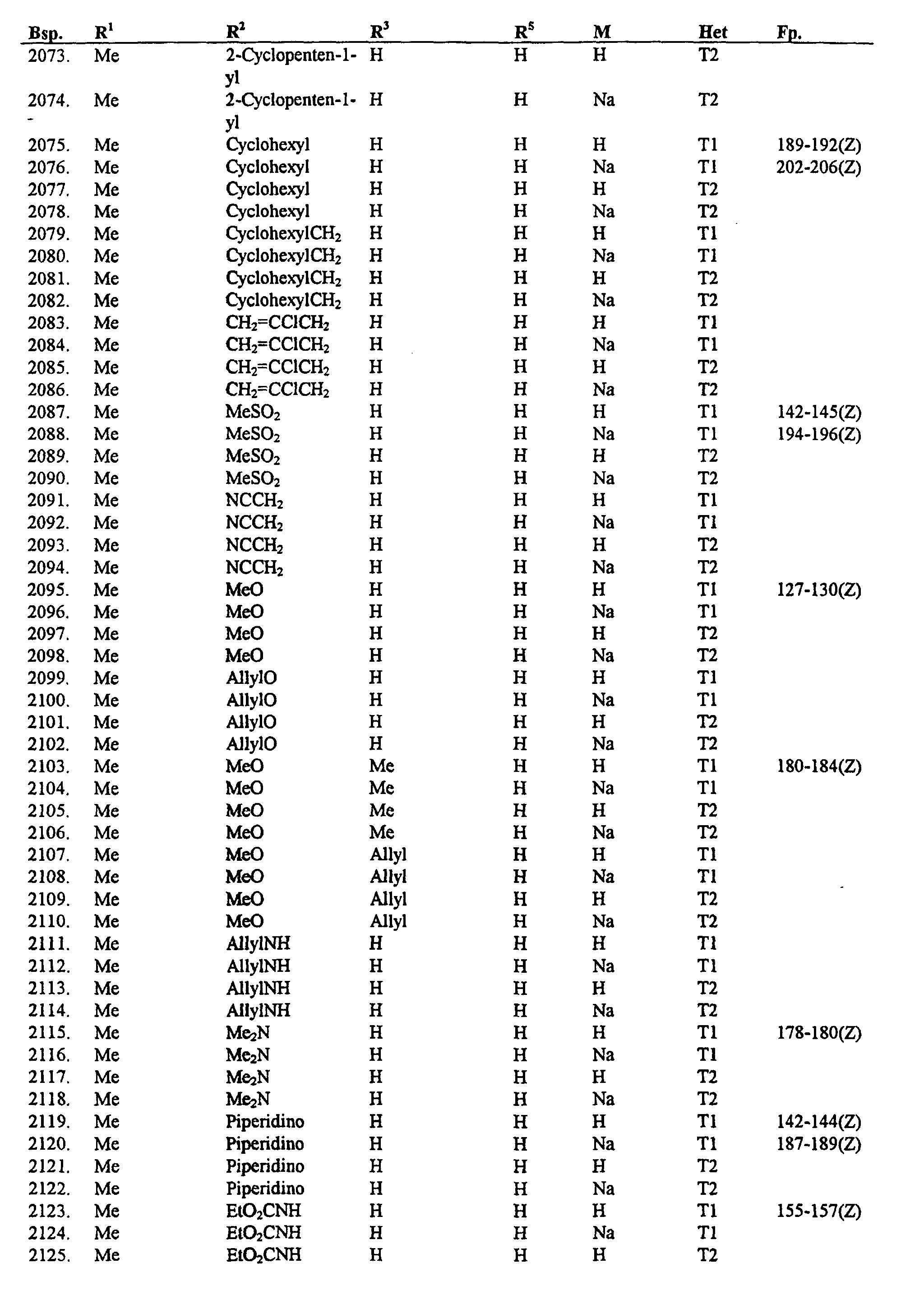

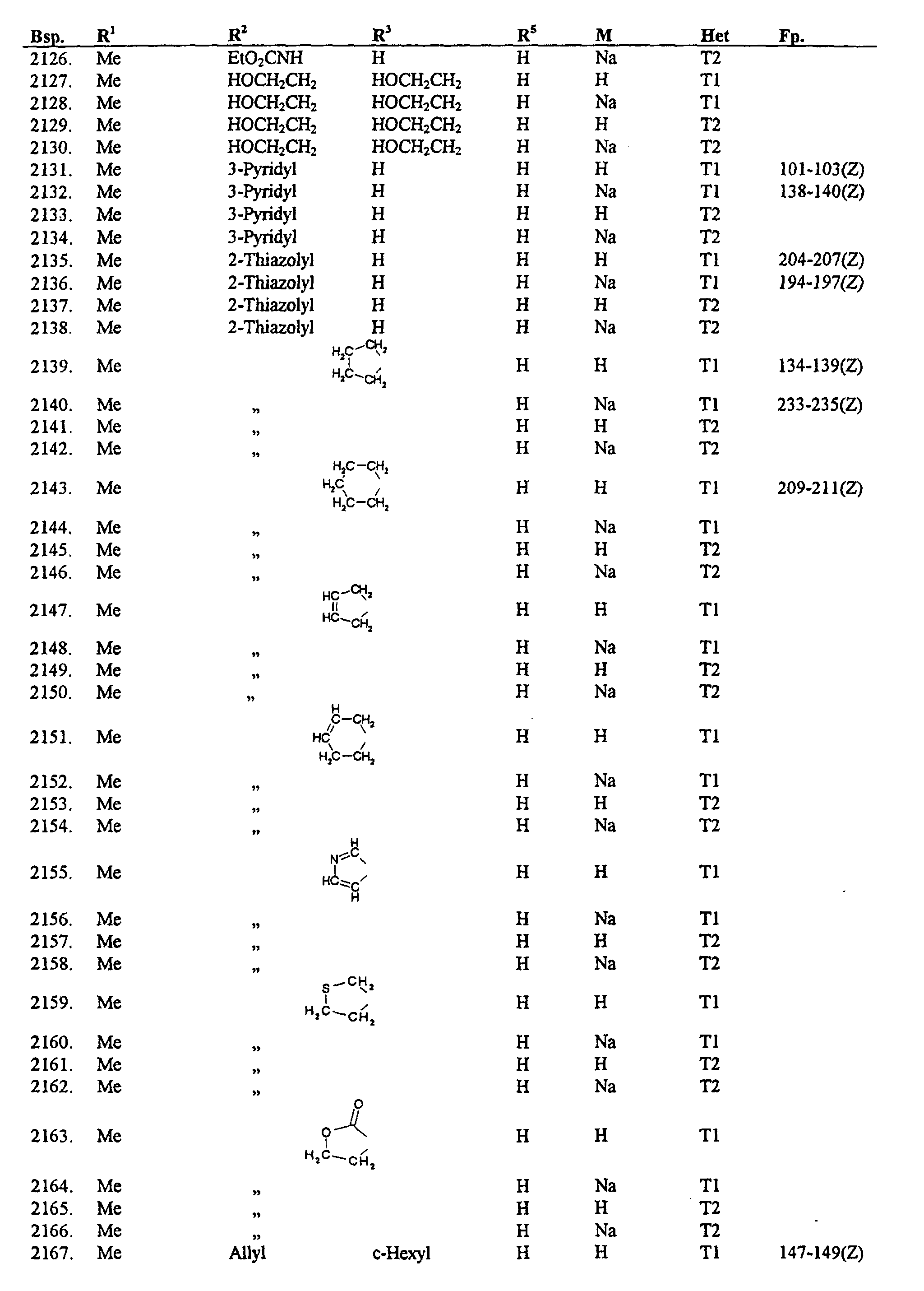

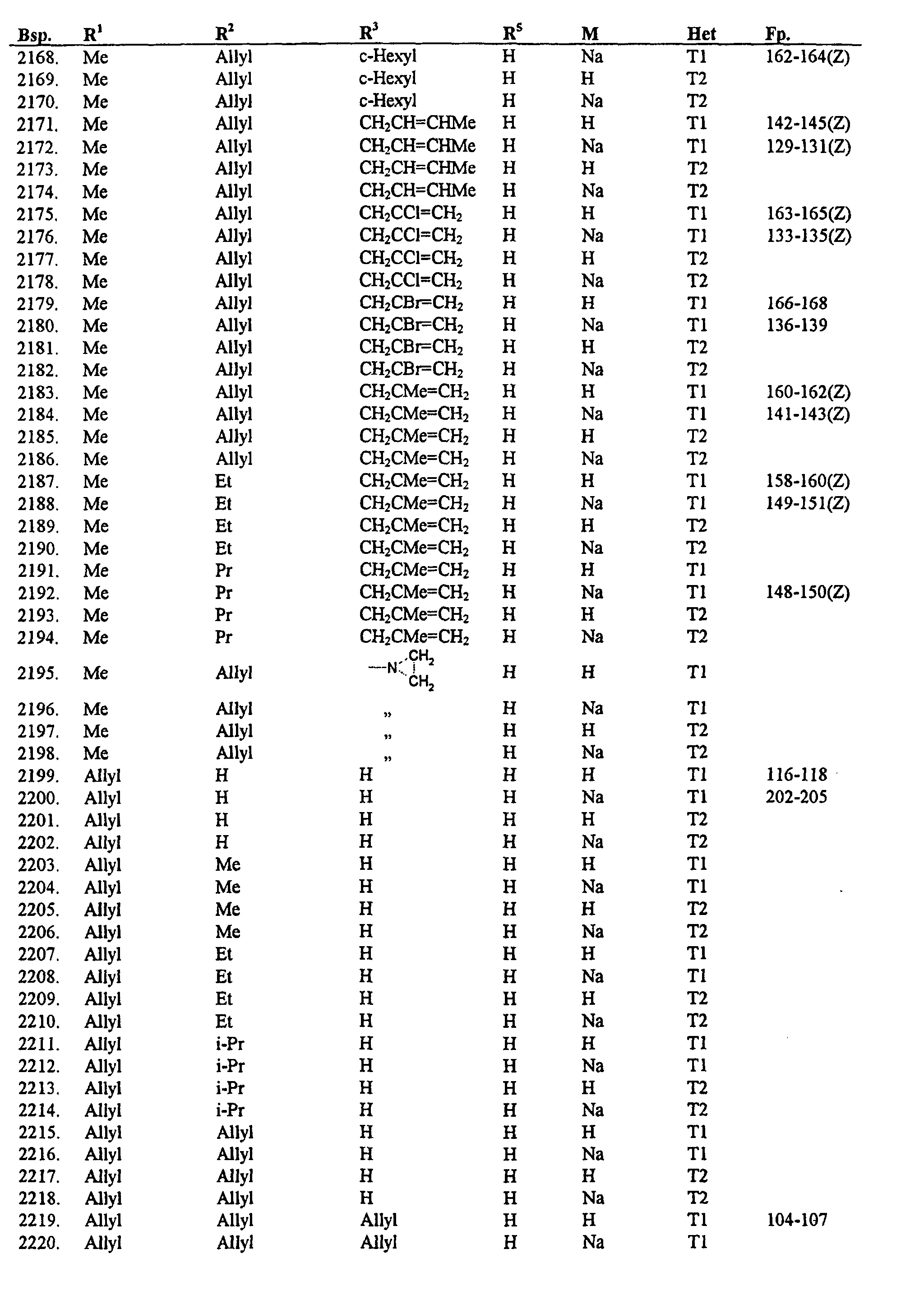

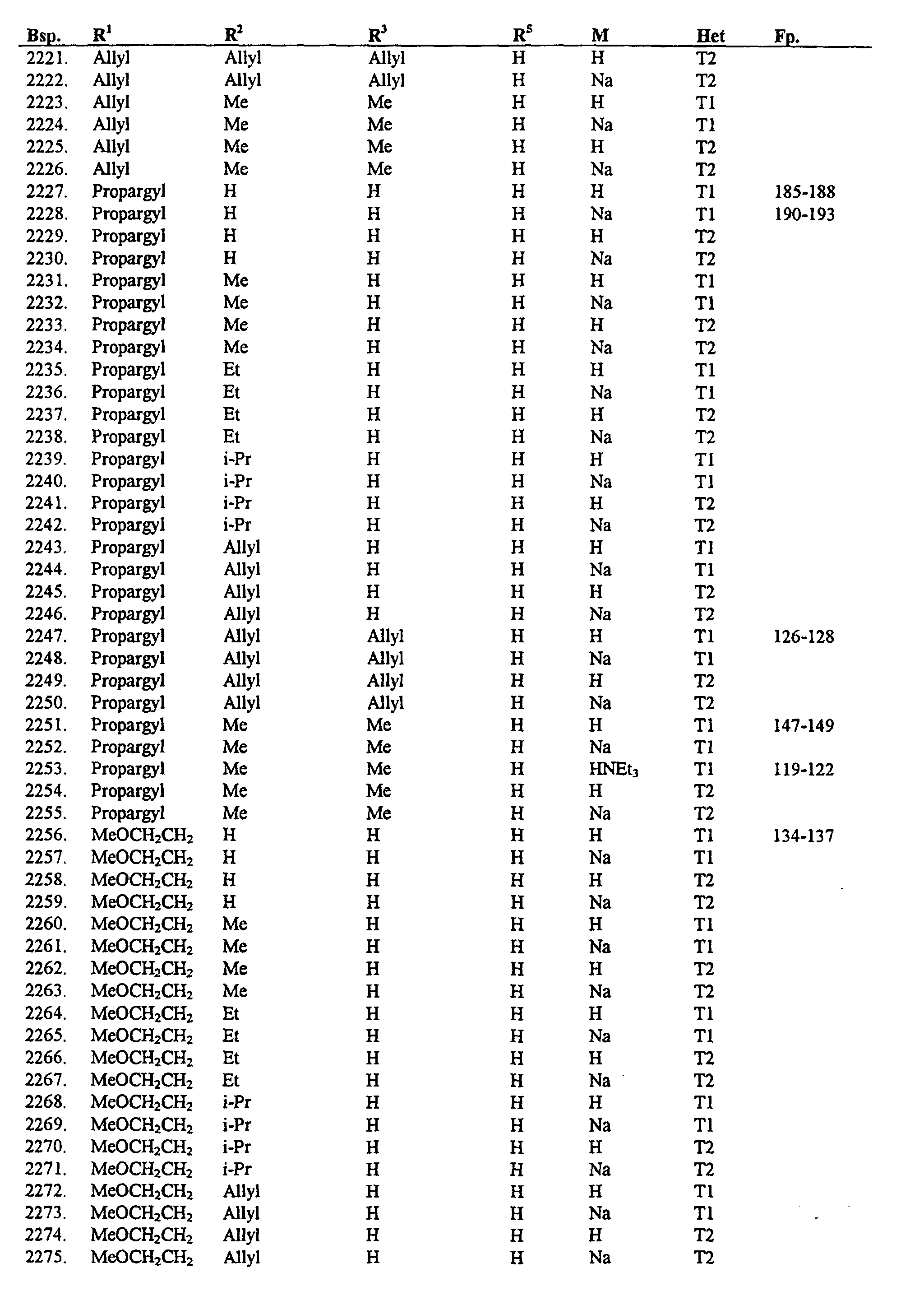

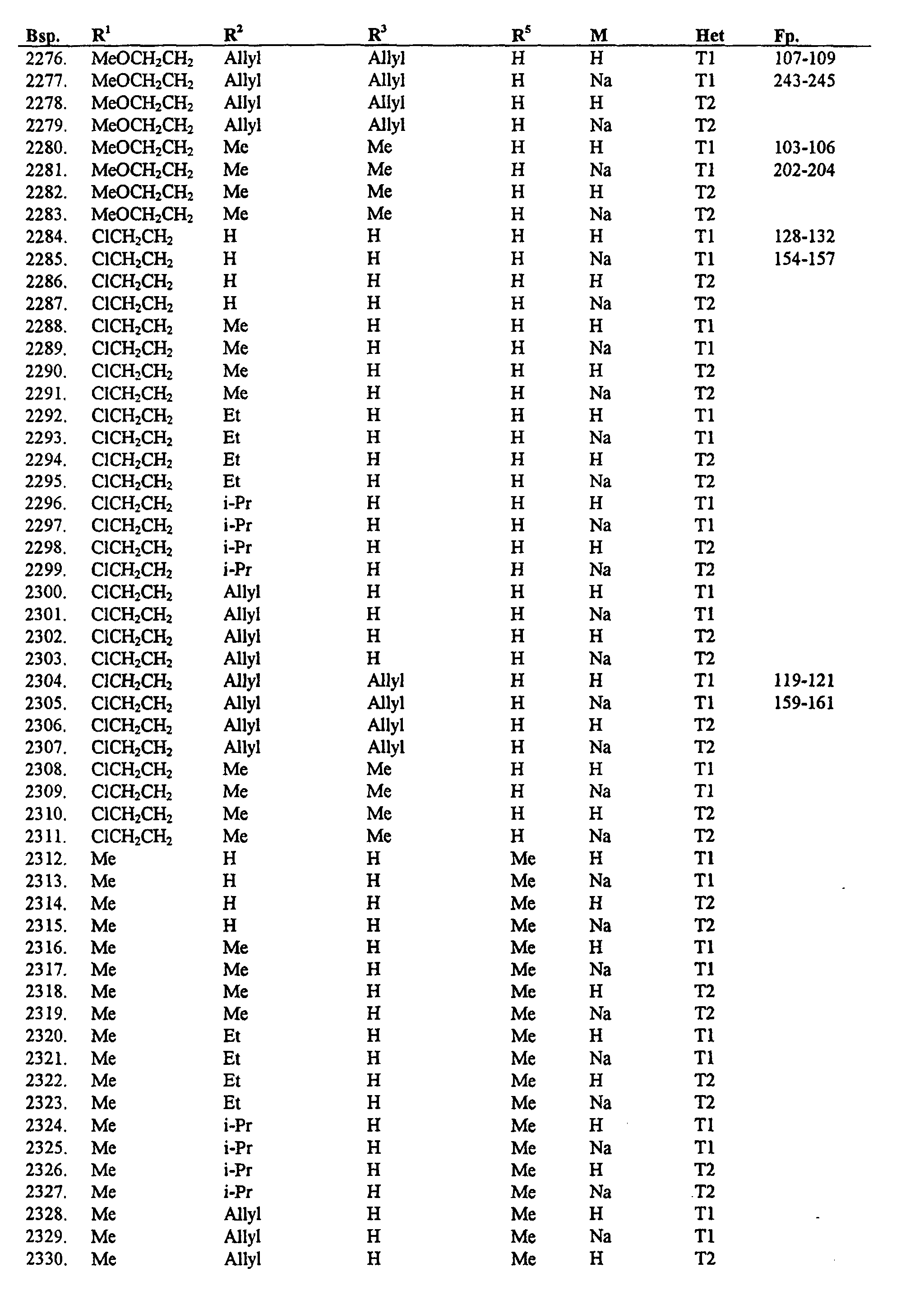

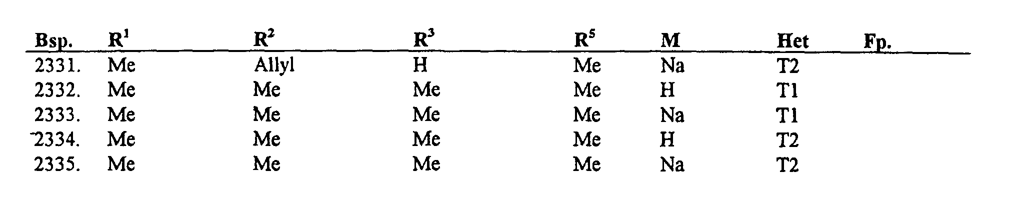

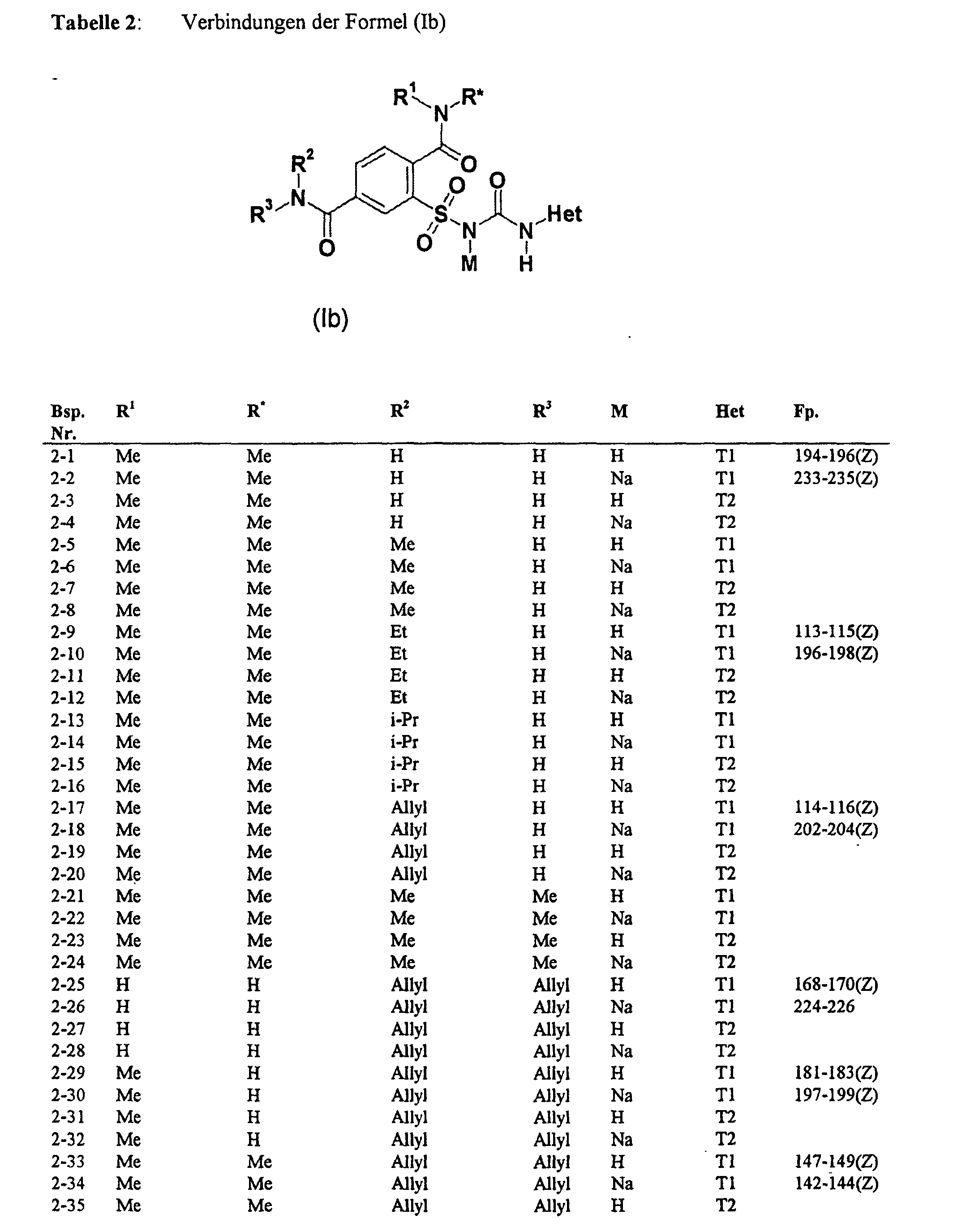

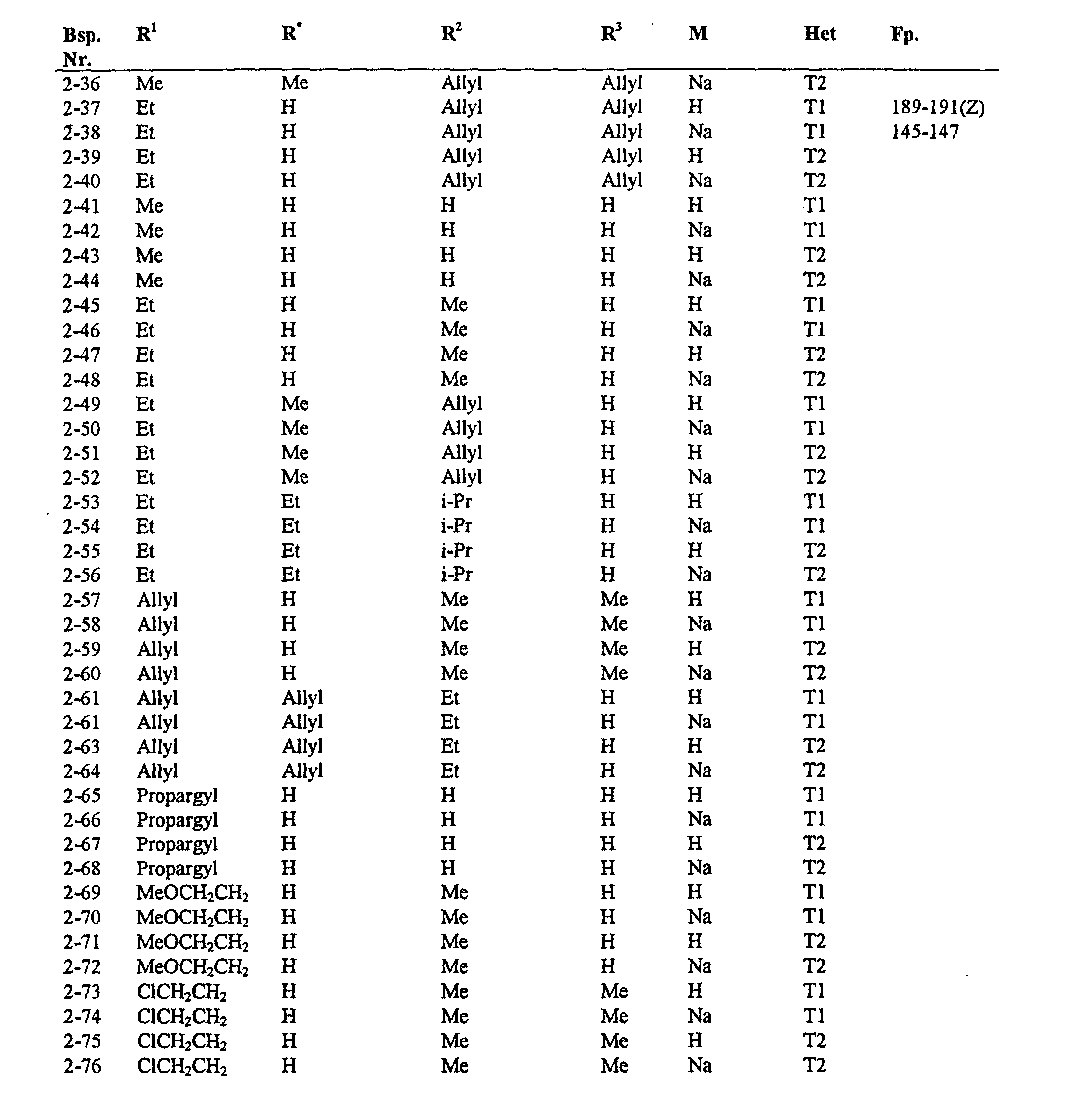

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gewichtsteile einer Verbindung der Formel (I),
   10 Gewichtsteile ligninsulfonsaures Calcium,
   5 Gewichtsteile Natriumlaurylsulfat,
   3 Gewichtsteile Polyvinylalkohol und
   7 Gewichtsteile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   - 25 Gewichtsteile einer Verbindung der Formel (I),
   5 Gewichtsteile 2,2'-dinaphthylmethan-6,6'-disulfonsaures Natrium
   2 Gewichtsteile oleoylmethyltaurinsaures Natrium,
   1 Gewichtsteil Polyvinylalkohol,
   17 Gewichtsteile Calciumcarbonat und
   50 Gewichtsteile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen wurden in Papptöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. Auflaufschäden erfolgte nach dem Auflaufen der Versuchspflanzen nach einer Versuchszeit von 3 bis 4 Wochen im Vergleich zu unbehandelten Kontrollen. Wie die Ergebnisse zeigen, weisen erfindungsgemäße Verbindungen eine gute herbizide Vorauflaufwirksamkeit gegen ein breites Spektrum von Ungräsern und Unkräutern auf. Beispielsweise haben die Beispiele Nr. 1, 2, 3, 4, 11, 12, 13, 14, 31, 32, 33, 34, 41, 42, 43, 44, 61, 62, 63, 64, 71, 72, 91, 92, 93, 94, 101, 102, 103, 104, 121, 122, 123, 124, 131, 132, 133, 134, 151, 152, 271, 272, 273, 274, 301, 302, 331, 361, 362, 391, 392, 405, 406, 435, 436, 449, 450, 479, 480, 499, 500, 529, 539, 749, 769, 770, 839, 840, 859, 860, 889, 890, 1289, 1290, 1559, 1560, 1561, 1562, 1565, 1566, 1567, 1568, 1569, 1570, 1579, 1599, 1600, 1609, 1619,1620, 1629, 1630, 1739, 1740, 1749, 1750, 1759, 1760, 1779, 1780, 1799, 1800, 1809, 1810, 1819, 1839, 1840, 1859, 1860, 1869, 1870, 1909, 1910, 1989, 1990, 2003, 2004, 2031, 2043, 2044, 2063, 2064, 2075, 2076, 2087, 2088, 2095, 2103, 2115, 2119, 2120, 2123, 2131, 2132, 2135, 2136, 2139, 2140, 2143, 2167, 2168, 2171, 2172, 2175, 2176, 2179, 2180, 2183, 2184, 2187, 2188, 2192, 2199, 2219, 2227, 2228, 2247, 2251, 2253, 2256, 2276, 2277, 2280, 2281, 2284, 2285, 2304, 2305, 2-1, 2-2, 2-9, 2-10, 2-17, 2-18, 2-25, 2-26, 2-29, 2-30, 2-33, 2-34, 2-37, 2-38 und andere Verbindungen aus Tabellen 1 und 2 sehr gute herbizide Wirkung gegen Schadpflanzen wie Sinapis alba, Chrysanthemum segetum, Avena sativa, Stellaria media, Echinochloa crus-galli, Lolium multiflorum, Setaria spp., Abutilon theophrasti, Amaranthus retroflexus und Panicum miliaceum im Vorauflaufverfahren bei einer Aufwandmenge von 0,3 kg und weniger Aktivsubstanz pro Hektar.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt. Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht. Nach ca. 3 bis 4 Wochen Standzeit der Versuchspflanzen im Gewächshaus unter optimalen Wachstumsbedingungen wurde die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Beispiele Nr. 1, 2, 3, 4, 11, 12, 13, 14, 31, 32, 33, 34, 41, 42, 43, 44, 61, 62, 63, 64, 71, 72, 91, 92, 93, 94, 101, 102, 103, 104, 121, 122, 123, 124, 131, 132, 133, 134, 151, 152, 271, 272, 273, 274, 301, 302, 331, 361, 362, 391, 392, 405, 406, 435, 436, 449, 450, 479, 480, 499, 500, 529, 539, 749, 769, 770, 839, 840, 859, 860, 889, 890, 1289, 1290, 1559, 1560, 1561, 1562, 1565, 1566, 1567, 1568, 1569, 1570, 1579, 1599, 1600, 1609, 1619, 1620, 1629, 1630, 1739, 1740, 1749, 1750, 1759, 1760, 1779, 1780, 1799, 1800, 1809, 1810, 1819, 1839, 1840, 1859, 1860, 1869, 1870, 1909, 1910, 1989, 1990, 2003, 2004, 2031, 2043, 2044, 2063, 2064, 2075, 2076, 2087, 2088, 2095, 2103, 2115, 2119, 2120, 2123, 2131, 2132, 2135, 2136, 2139, 2140, 2143, 2167, 2168, 2171, 2172, 2175, 2176, 2179, 2180, 2183, 2184, 2187, 2188, 2192, 2199, 2219, 2227, 2228, 2247, 2251, 2253, 2256, 2276, 2277, 2280, 2281, 2284, 2285, 2304, 2305, 2-1, 2-2, 2-9, 2-10, 2-17, 2-18, 2-25, 2-26, 2-29, 2-30, 2-33, 2-34, 2-37, 2-38 und andere Verbindungen aus Tabellen 1 und 2 sehr gute herbizide Wirkung gegen Schadpflanzen wie Sinapis alba, Echinochloa crus-galli, Lolium multiflorum, Chrysanthemum segetum, Setaria spp., Abutilon theophrasti, Amaranthus retroflexus, Panicum miliaceum und Avena sativa im Nachauflaufverfahren bei einer Aufwandmenge von 0,3 kg und weniger Aktivsubstanz pro Hektar.

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt. Ein Teil der Töpfe wurde sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wurde mittels optischer Bonitur festgestellt, daß erfindungsgemäße Verbindungen Gramineen-Kulturen wie z.B. Gerste, Weizen, Roggen, Hirsen, Mais oder Reis im Vor- und Nachauflaufverfahren selbst bei hohen Wirkstoffdosierungen ungeschädigt ließen. Einige Substanzen schonten darüber hinaus auch zweikeimblättrige Kulturen wie z.B. Soja, Baumwolle, Raps, Zuckerrüben oder Kartoffeln. Die Verbindungen der Formel (I) zeigen teilweise eine hohe Selektivität und eignen sich deshalb zur Bekämpfung von unerwünschten Pflanzenwuchs in landwirtschaftlichen Kulturen.

## Patentansprüche

1. Verbindungen der Formel (I) oder deren Salze, 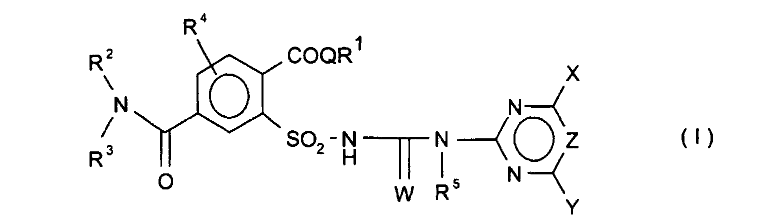 worin
R¹ ein Wasserstoffatom, einen Kohlenwasserstoffrest oder einen Heterocyclylrest, wobei jeder der letztgenannten beiden Reste unsubstituiert oder substituiert ist und inklusive Substituenten 1 bis 30 C-Atome aufweist,
R² eine Gruppe der Formel R⁰-Q⁰-, worin
R⁰ ein Wasserstoffatom, einen Kohlenwasserstoffrest oder einen Heterocyclylrest, wobei jeder der letztgenannten beiden Reste unsubstituiert oder substituiert ist und inklusive Substituenten 1 bis 30 C-Atome aufweist, bedeutet und
Q⁰ eine direkte Bindung oder eine divalente Gruppe der Formel -O-, -SO₂-, -NH-, -N[(C₁-C₆)Alkyl]-, -CO-, -CO-NH- oder -O-CO-NH- bedeutet,
R³ ein Wasserstoffatom, einen Kohlenwasserstoffrest oder einen Heterocyclylrest, wobei jeder der letztgenannten beiden Reste unsubstituiert oder substituiert ist und inklusive Substituenten 1 bis 30 C-Atome aufweist, oder
R² und R³ gemeinsam mit dem N-Atom einen Heterocyclus von 3-6 Ringatomen, der gesättigt oder ungesättigt ist, zusätzlich zum N-Atom ein oder zwei Atome aus der Gruppe N, O und S enthalten kann und unsubstituiert oder durch einen oder mehrere Reste aus der Gruppe (C₁-C₆)Alkyl, (C₁-C₆)Alkoxy, Halogen, [(C₁-C₆)Alkoxy]carbonyl, (C₁-C₆)Haloalkyl und Oxo substituiert ist,
R⁴ H, Halogen, NO₂, CN, (C₁-C₄)Alkyl, (C₁-C₄)Alkoxy, [(C₁-C₄)Alkyl]-carbonyl oder [(C₁-C₄)Alkoxy]carbonyl, wobei jeder der letztgenannten vier Reste unsubstituiert oder im Alkylteil durch ein oder mehrere Halogenatome substituiert ist,
R⁵ H oder (C₁-C₄)Alkyl,
Q O oder NR*,
R* H, (C₁-C₄)Alkyl, (C₃-C₄)Alkenyl oder (C₃-C₄)Alkinyl, wobei jeder der drei letztgenannten Reste unsubstituiert oder durch einen oder mehrere Reste aus der Gruppe Halogen, (C₁-C₄)Alkoxy und (C₁-C₄)Alkylthio substituiert ist,
W ein Sauerstoff- oder Schwefelatom,
X,Y unabhängig voneinander H, Halogen, (C₁-C₄)Alkyl, (C₁-C₄)Alkoxy, (C₁-C₄)-Alkylthio, wobei jeder der letztgenannten 3 Reste unsubstituiert oder durch einen oder mehrere Reste aus der Gruppe Halogen, (C₁-C₄)Alkoxy und (C₁-C₄)Alkylthio substituiert ist, oder Mono- oder Di[((C₁-C₄)alkyl]-amino, (C₃-C₄)-Cycloalkyl, (C₂-C₅)Alkenyl, (C₂-C₅)Alkinyl, (C₂-C₅)Alkenyloxy oder (C₂-C₅)-Alkinyloxy und
Z CH oder N bedeuten.

2. Verbindungen oder deren Salze nach Anspruch 1, **dadurch gekennzeichnet, daß**
R¹ H, (C₁-C₆)Alkyl, (C₃-C₆)Alkenyl, (C₃-C₆)Alkinyl, wobei jeder der drei letztgenannten Reste unsubstituiert oder durch einen oder mehrere Reste aus der Gruppe Halogen, unsubstituiertes und substituiertes Phenyl, unsubstituiertes und substituiertes Heterocyclyl mit 3 bis 6 Ringatomen, unsubstituiertes und substituiertes (C₃-C₆)Cycloalkyl, (C₁-C₄)Alkoxy, (C₁-C₄)Alkylthio, [(C₁-C₄)Alkoxy]-carbonyl und [(C₁-C₄)Haloalkoxy]-carbonyl, substituiert ist, oder unsubstituiertes oder substituiertes (C₃-C₆)Cycloalkyl, unsubstituiertes oder substituiertes Phenyl, unsubstituiertes oder substituiertes Heterocyclyl mit 3 bis 6 Ringatomen und
R² eine Gruppe der Formel R⁰-Q⁰-,
worin R⁰ ein Wasserstoffatom, (C₁-C₁₂)Alkyl, (C₃-C₁₂)Alkenyl oder (C₃-C₁₂)Alkinyl, wobei jeder der letztgenannten 3 Reste unsubstituiert oder durch einen oder mehrere Reste aus der Gruppe Halogen, (C₁-C₆)Alkoxy, (C₁-C₆)Haloalkoxy, (C₁-C₆)Alkylthio, (C₁-C₆)-Haloalkylthio, (C₁-C₆)Alkylsulfinyl, (C₁-C₆)Haloalkylsulfinyl, (C₁-C₆)Alkylsulfonyl, (C₁-C₆)Haloalkylsulfonyl, [(C₁-C₆)Alkoxy]carbonyl, [(C₁-C₆)Haloalkoxy]carbonyl, CONR⁶R⁷, SO₂NR⁶R⁷, CN, OH, (C₃-C₆)Cycloalkyl, NR⁸R⁹, unsubstituiertes Phenyl, substituiertes Phenyl, unsubstituiertes Heterocyclyl und substituiertes Heterocyclyl substituiert ist, oder unsubstituiertes oder substituiertes (C₃-C₆)Cycloalkyl, unsubstituiertes oder substituiertes (C₃-C₆)Cycloalkenyl, unsubstituiertes oder substituiertes Heterocyclyl oder unsubstituiertes oder substituiertes Phenyl bedeutet und worin Q⁰ eine direkte Bindung oder eine divalente Gruppe der Formel -O-, -SO₂-, -NH-, -N[(C₁-C₆)Alkyl]-, -CO-, -CO-NH- oder -O-CO-NH- bedeutet,
R³ unabhängig voneinander wie R⁰ im Rest R² definiert ist, oder
R² und R³ gemeinsam mit dem N-Atom einen Heterocyclus von 3-6 Ringatomen, der gesättigt oder ungesättigt ist, zusätzlich zum N-Atom ein oder zwei Atome aus der Gruppe N, O und S enthalten kann und unsubstituiert oder durch einen oder mehrere Reste aus der Gruppe (C₁-C₆)Alkyl, (C₁-C₆)Alkoxy, Halogen, [(C₁-C₆)Alkoxy]carbonyl, (C₁-C₆)Haloalkyl und Oxo substituiert ist, und
R⁶ und R⁷ unabhängig voneinander H, (C₁-C₆)Alkyl, (C₃-C₆)Alkenyl, (C₃-C₆)Alkinyl oder unsubstituiertes oder substituiertes Phenyl oder
R⁶ und R⁷ gemeinsam mit dem N-Atom einen heterocyclischen Ring mit 5 oder 6 Ringgliedern, der gegebenenfalls weitere Heteroatome aus der Gruppe N, O und S enthalten kann und unsubstituiert oder ein- oder mehrfach durch Reste aus der Gruppe (C₁-C₄)Alkyl und Oxo substituiert ist, und
R⁸ und R⁹ unabhängig voneinander und unabhängig von R⁶ und R⁷ wie unter R⁶ und R⁷ definiert oder (C₁-C₄)Alkylcarbonyl, (C₁-C₄)Haloalkylcarbonyl, (C₁-C₄)-Alkoxycarbonyl oder (C₁-C₄)Alkylsulfonyl,
Q O oder NR*, wobei R* wie oben definiert ist,
X und Y unabhängig voneinander H, Halogen, (C₁-C₄)Alkyl, (C₁-C₄)Alkoxy, (C₁-C₄)Alkylthio, wobei jeder der drei letztgenannten Reste unsubstituiert oder durch einen oder mehrere Reste aus der Gruppe Halogen, (C₁-C₃)Alkoxy und (C₁-C₄)Alkylthio substituiert ist, Mono- oder Di[(C₁-C₄)alkyl]amino, (C₃-C₆)-Cycloalkyl, (C₃-C₅)Alkenyl, (C₃-C₅)Alkenyloxy oder (C₃-C₅)Alkinyloxy und
Z CH oder N bedeuten, wobei
substituiertes Phenyl, substituiertes Heterocyclyl, substituiertes Cycloalkyl oder substituiertes Cycloalkenyl als Substituenten einen oder mehrere Reste aus der Gruppe Halogen, (C₁-C₄)Alkyl, (C₁-C₄)Haloalkyl, (C₁-C₄)Alkoxy-(C₁-C₄)Alkyl, Di-[(C₁-C₄)Alkoxy]-(C₁-C₄)Alkyl, (C₁-C₄)Haloalkoxy, (C₁-C₄)Alkylthio, (C₁-C₄)Alkylsulfinyl, (C₁-C₄)Haloalkylsulfinyl, (C₁-C₄)Alkylsulfonyl, (C₁-C₄)Haloalkylsulfonyl, NR⁸R⁹, [(C₁-C₄)Alkoxy]carbonyl, [(C₁-C₄)Haloalkoxy]carbonyl, [(C₁-C₄)Alkyl]carbonyl, OH, Phenyl, CN und NO₂ trägt und
wobei jeder der Reste R¹, R² und R³ inklusive Substituenten 1 bis 20 C-Atome, aufweist.

3. Verbindungen oder deren Salze nach Anspruch 1 oder 2, **dadurch gekennzeichnet, daß**
R¹ H, (C₁-C₆)Alkyl, (C₃-C₆)Alkenyl oder (C₃-C₆)Alkinyl, wobei jeder der drei letztgenannten Reste unsubstituiert oder durch einen oder mehrere Reste aus der Gruppe Halogen, Phenyl, (C₁-C₄)Alkoxy, (C₁-C₄)Alkylthio und [(C₁-C₄)Alkoxy]-carbonyl substituiert ist, oder (C₃-C₆)Cycloalkyl, (C₃-C₆)Cycloalkyl(C₁-C₃)alkyl, Heterocyclyl mit 3 bis 6 Ringatomen oder Heterocyclyl-(C₁-C₃)alkyl mit 3 bis 6 Ringatomen, wobei jeder der letztgenannten 4 Reste unsubstituiert oder durch einen oder mehrere Reste aus der Gruppe Halogen, (C₁-C₄)Alkyl und (C₁-C₄)Alkoxy substituiert ist,
R² eine Gruppe der Formel R⁰-Q⁰-, worin
R⁰ ein Wasserstoffatom, (C₁-C₈)Alkyl, (C₃-C₈)Alkenyl oder (C₃-C₈)Alkinyl,
wobei jeder der letztgenannten 3 Reste unsubstituiert oder durch einen oder mehrere Reste aus der Gruppe Halogen (C₁-C₄)Alkoxy, (C₁-C₄)Haloalkoxy, (C₁-C₄)Alkylthio, (C₁-C₄)-Haloalkylthio, (C₁-C₄)Alkylsulfinyl,
(C₁-C₄)Haloalkylsulfinyl, (C₁-C₄)Alkylsulfonyl, (C₁-C₄)Haloalkylsulfonyl,
[(C₁-C₆)Alkoxy]carbonyl, CONR⁶R⁷, SO₂NR⁶R⁷, CN, OH, (C₃-C₆)Cycloalkyl, NR⁸R⁹, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsufonyl, NR⁸R⁹, [(C₁-C₄)Alkoxy]carbonyl, [(C₁-C₄)Alkyl]carbonyl, Phenyl, [(C₁-C₄)Alkyl]-carbonyl, CN und NO₂ substituiert ist, und Heterocyclyl, das unsubstituiert oder durch einen oder mehrere Reste aus der Gruppe Halogen, (C₁-C₄)Alkyl,
(C₁-C₄)Haloalkyl, (C₁-C₄)Alkoxy, (C₁-C₄)Haloalkoxy, (C₁-C₄)Alkylthio,
(C₁-C₄)Alkylsulfinyl, (C₁-C₄)Alkylsufonyl, NR⁸R⁹, [(C₁-C₄)Alkoxy]carbonyl,
[(C₁-C₄)Alkyl]carbonyl, Phenyl, [(C₁-C₄)Alkyl]-carbonyl, CN und NO₂ substituiert ist, substituiert ist, oder
(C₃-C₆)Cycloalkyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)-Haloalkoxy, [(C₁-C₄)Alkoxy]carbonyl, CN, OH und Phenyl substituiert ist, oder (C₃-C₆)Cycloalkenyl, das unsubstituiert oder durch einen oder mehrere Reste aus der Gruppe Halogen, (C₁-C₄)Alkyl, (C₁-C₄)Haloalkyl, (C₁-C₄)Alkoxy und
[(C₁-C₄)Alkoxy]carbonyl substituiert ist, oder
Heterocyclyl oder Phenyl, wobei jeder der letztgenannten 2 Reste unsubstituiert oder durch einen oder mehrere Reste aus der Gruppe Halogen,
(C₁-C₄)Alkyl, (C₁-C₄)Haloalkyl, (C₁-C₄)Alkoxy, (C₁-C₄)Haloalkoxy,
(C₁-C₄)Alkylthio, (C₁-C₄)Alkylsulfinyl, (C₁-C₄)Alkylsufonyl, NR⁸R⁹,
[(C₁-C₄)Alkoxy]carbonyl, [(C₁-C₄)Alkyl]carbonyl, Phenyl, [(C₁-C₄)Alkyl]-carbonyl, CN und NO₂ substituiert ist, und
Q⁰ eine direkte Bindung oder eine divalente Gruppe der Formel -O-, -SO₂-,
-NH-, -CO-NH- oder -O-CO-NH- bedeutet,
R³ unabhängig voneinander wie R⁰ im Rest R² definiert ist,
R² und R³ gemeinsam mit dem N-Atom einen Heterocyclus von 3-6 Ringatomen, der gesättigt oder ungesättigt ist und zusätzlich zum N-Atom ein oder zwei Heteroatome aus der Gruppe N, O und S enthalten kann und unsubstituiert oder durch einen oder mehrere Reste aus der Gruppe (C₁-C₃)Alkyl, (C₁-C₃)Alkoxy, Halogen, [(C₁-C₃)Alkoxy]carbonyl, (C₁-C₃)Haloalkyl und Oxo substituiert ist,
R⁶ und R⁷ unabhängig voneinander H, (C₁-C₄)Alkyl, (C₃-C₄)Alkenyl, (C₃-C₄)Alkinyl oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy,
(C₁-C₄)Alkylthio, (C₁-C₄)Alkylsulfonyl, [(C₁-C₄)Alkoxy]carbonyl, CN und NO₂ substituiert ist, oder
R⁶ und R⁷ gemeinsam mit dem N-Atom einen heterocyclischen Ring mit 5 oder 6 Ringgliedern, der gegebenenfalls weitere Heteroatome aus der Gruppe N, O und S enthalten kann und unsubstituiert oder ein- oder mehrfach durch Reste aus der Gruppe (C₁-C₄)Alkyl und Oxo substituiert ist,
R⁸ und R⁹ unabhängig voneinander unabhängig von R⁶ und R⁷ wie unter R⁶ und R⁷ definiert ist oder (C₁-C₄)Alkylcarbonyl, (C₁-C₄)Haloalkylcarbonyl, (C₁-C₄)-Alkoxycarbonyl oder (C₁-C₄)Alkylsulfonyl,
Q O oder NR*, wobei R* wie weiter oben definiert ist,
X und Y unabhängig voneinander H, Halogen, (C₁-C₄)Alkyl, (C₁-C₄)Alkoxy, (C₁-C₄)Alkylthio, wobei jeder der drei letztgenannten Reste unsubstituiert oder durch einen oder mehrere Reste aus der Gruppe Halogen, (C₁-C₃)Alkoxy und (C₁-C₄)Alkylthio substituiert ist, Mono- oder Di[(C₁-C₄)alkyl]amino, (C₃-C₆)Cycloalkyl, (C₃-C₅)Alkenyl, (C₃-C₅)Alkenyloxy oder (C₃-C₅)Alkinyloxy und
Z CH oder N bedeuten.

4. Verbindungen oder deren Salze nach einem der Ansprüche 1 bis 3, **dadurch gekennzeichnet, daß**
R¹ (C₁-C₆)Alkyl, das unsubstituiert oder durch einen oder mehrere Reste aus der Gruppe Halogen und (C₁-C₄)Alkoxy substituiert ist, oder 3-Oxetanyl, (C₃-C₄)Alkenyl oder (C₃-C₄)Alkinyl,
R² H, (C₁-C₆)Alkyl, (C₃-C₆)Alkenyl, (C₃-C₆)Alkinyl, wobei jeder der letztgenannten drei Reste unsubstituiert oder durch einen oder mehrere Reste aus der Gruppe Halogen, (C₁-C₄)Alkoxy, (C₁-C₄)Alkylthio, (C₁-C₄)Alkylsulfonyl,
[(C₁-C₄)Alkoxy]carbonyl, (C₃-C₆)Cycloalkyl, CN und OH substituiert ist, oder (C₃-C₆)Cycloalkyl, das unsubstituiert oder durch einen oder mehrere Reste aus der Gruppe Halogen, (C₁-C₄)Alkyl, (C₁-C₄)Alkoxy, [(C₁-C₄)Alkoxy]carbonyl, CN und OH substituiert ist, oder (C₃-C₆)Cycloalkenyl, (C₁-C₄)Alkoxy, (C₁-C₄)Alkenyloxy, (C₁-C₄)Alkylsulfonyl, (C₁-C₄)Alkylamino oder Di[(C₁-C₄)Alkyl]amino und
R³ H, (C₁-C₆)Alkyl, (C₃-C₆)Alkenyl, (C₃-C₆)Alkinyl, wobei jeder der letztgenannten drei Reste unsubstituiert oder durch einen oder mehrere Reste aus der Gruppe Halogen, (C₁-C₄)Alkoxy, (C₁-C₄)Alkylthio, (C₁-C₄)Alkylsulfonyl, [(C₁-C₄)Alkoxy]carbonyl, (C₃-C₆)Cycloalkyl, CN und OH substituiert ist, oder (C₃-C₆)Cycloalkyl, das unsubstituiert oder durch einen oder mehrere Reste aus der Gruppe Halogen, (C₁-C₄)Alkyl, (C₁-C₄)Alkoxy, [(C₁-C₄)Alkoxy]carbonyl, CN und OH substituiert ist, oder (C₃-C₆)Cycloalkenyl oder
R² und R³ gemeinsam mit dem N-Atom einen Heterocyclus von 3-6 Ringatomen, der gesättigt oder ungesättigt ist, zusätzlich zu dem N-Atom ein oder zwei Atome aus der Gruppe N, O und S enthalten kann und unsubstituiert oder durch einen oder mehrere Reste aus der Gruppe Halogen, (C₁-C₃)Alkyl, (C₁-C₃)Alkoxy, Oxo und [(C₁-C₃)Alkoxy]carbonyl substituiert ist, und
R⁴ H, (C₁-C₄)Alkyl, (C₁-C₄)Haloalkyl, (C₁-C₄)Alkoxy oder Halogen,
R⁵ H oder Methyl,
R* H oder (C₁-C₄)Alkyl,
X und Y unabhängig voneinander (C₁-C₄)Alkyl, (C₁-C₄)Alkoxy, wobei jeder der letztgenannten zwei Reste unsubstituiert oder durch einen oder mehrere Halogenatome substituiert ist, oder (C₁-C₄)Alkylthio, Halogen oder Mono- oder Di[(C₁-C₂)alkyl]amino und
W ein Sauerstoffatom bedeuten.

5. Verbindungen oder deren Salze nach einem der Ansprüche 1 bis 3, **dadurch gekennzeichnet, daß**
R¹ (C₁-C₃)Alkyl, Allyl oder Propargyl,
R² und R³ unabhängig voneinander H, (C₁-C₄)Alkyl, (C₁-C₃)Alkenyl, (C₁-C₃)Alkinyl, (C₁-C₃)Cycloalkyl oder (C₃-C₆)Cycloalkenyl,
R⁴ H, (C₁-C₃)Alkyl oder Halogen,
R* (C₁-C₃)Alkyl,
X (C₁-C₂)Alkyl, (C₁-C₂)Alkoxy, (C₁-C₂)Alkylthio, (C₁-C₂)Haloalkyl oder (C₁-C₂)-Haloalkoxy und
Y (C₁-C₂)Alkyl, (C₁-C₂)Alkoxy, Halogen, NHCH₃ oder N(CH₃)₂ bedeuten.

6. Verfahren zur Herstellung von Verbindungen der Formel (I) oder deren Salzen, wie sie nach einem der Ansprüche 1 bis 5 definiert sind, **dadurch gekennzeichnet, daß** man
a) eine Verbindung der Formel (II) 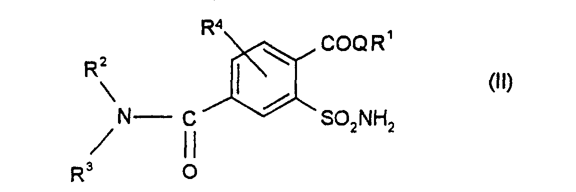 mit einem heterocyclischen Carbamat der Formel (III), 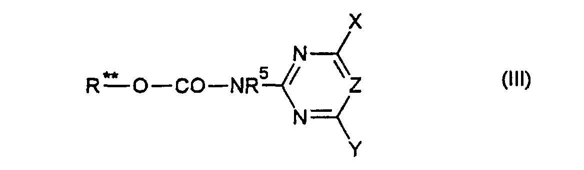 worin R** gegebenenfalls substituiertes Aryl oder einen aliphatischen Rest bedeutet, umsetzt oder
b) ein Sulfonylcarbamat der Formel (IV), 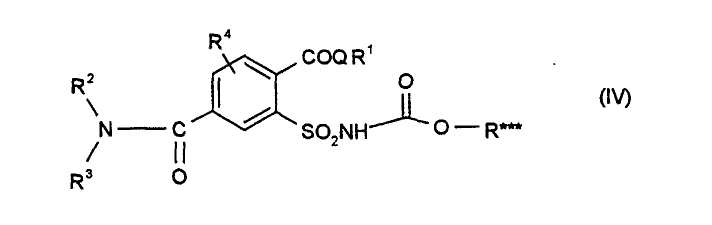 worin R*** gebenenfalls substituiertes Phenyl oder (C₁-C₄)Alkyl bedeutet, mit einem Aminoheterocyclus der Formel (V) 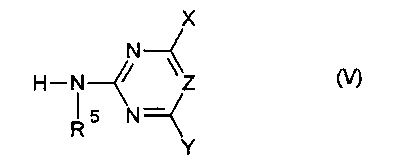 umsetzt oder
c) ein Sulfonylisocyanat der Formel (VI) 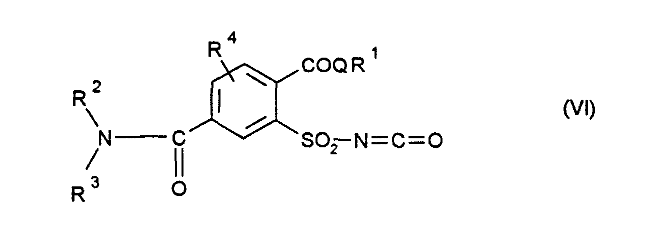 mit einem Aminoheterocyclus der Formel (V) umsetzt oder
d) ein Sulfonamid der Formel (II) mit einem (Thio)-Isocyanat der Formel (VII) 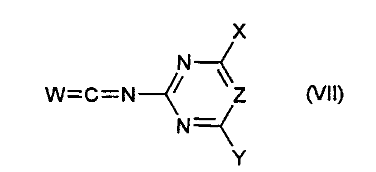 in Gegenwart einer Base umsetzt oder
e) einen Aminoheterocyclus der Formel (V) zunächst basenkatalysiert mit einem Kohlensäureester, z.B. Diphenylcarbonat, umsetzt und das gebildete Intermediat in einer Eintopfreaktion mit einem Sulfonamid der Formel (II) (siehe Variante a) umsetzt,
wobei in den Formeln (II)-(VII) die Reste bzw. Gruppen R¹-R⁵, W, X, Y und Z wie in Formel (I) definiert sind und in Verfahrensvarianten a) bis c) und e) zunächst Verbindungen (I) mit W = O erhalten werden.

7. Herbizides oder pflanzenwachstumsregulierende Mittel, **dadurch gekennzeichnet, daß** es mindestens eine Verbindung der Formel (I) oder deren Salz nach einem der Ansprüche 1 bis 5 und im Pflanzenschutz übliche Formulierungshilfsmittel enthält.

8. Verfahren zur Bekämpfung von Schadpflanzen oder zur Wachstumsregulierung von Pflanzen, **dadurch gekennzeichnet, daß** man eine wirksame Menge von mindestens einer Verbindung der Formel (I) oder deren Salz nach einem der Ansprüche 1 bis 5 auf die Schadpflanzen bzw. Pflanzen, deren Pflanzensamen oder die Fläche, auf der sie wachsen, appliziert.

9. Verwendung der Verbindungen der Formel (I) oder deren Salze nach einem der Ansprüche 1 bis 5 als Herbizide oder Pflanzenwachstumsregulatoren.

10. Verwendung nach Anspruch 9, **dadurch gekennzeichnet, daß** die Verbindungen der Formel (I) oder deren Salze zur Bekämpfung von Schadpflanzen oder zur Wachstumsregulierung in Kulturen von Nutz- oder Zierpflanzen eingesetzt werden.

11. Verwendung nach Anspruch 10, **dadurch gekennzeichnet, daß** die Kulturpflanzen transgene Kulturpflanzen sind.

12. Verbindungen der Formel (II)* 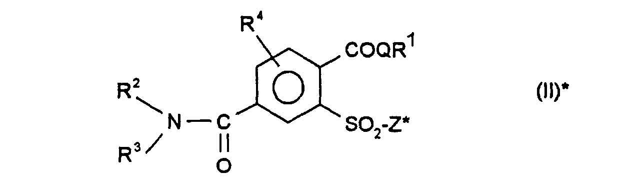 worin Z*= NH₂, NHCOOR***, NCO, NH-tert.-Butyl oder Cl bedeutet und R¹-R⁴, R*** und Q wie in Formel (I) bzw. Formel (IV) nach Anspruch 6 definiert sind.

13. Verbindungen der Formel (IX)' 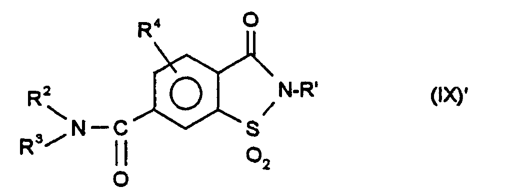 worin R² bis R⁴ wie in Formel (I) nach Anspruch 1 definiert sind und R' eine Alkylgruppe darstellt.

## Claims

1. A compound of the formula (I) or a salt thereof 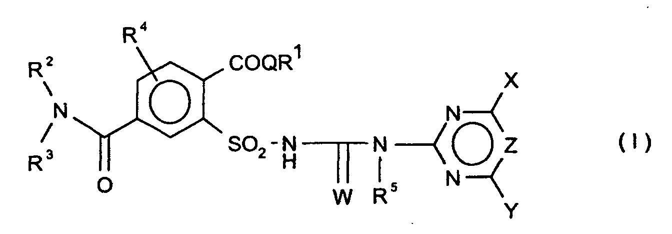 in which
R¹ is a hydrogen atom, a hydrocarbon radical or a heterocyclyl radical, where each of the two last mentioned radicals is unsubstituted or substituted and has, including substituents, 1 to 30 carbon atoms,
R² is a group of the formula R⁰-Q⁰-, in which
R⁰ is a hydrogen atom, a hydrocarbon radical or a heterocyclyl radical, where each of the two last mentioned radicals is unsubstituted or substituted and has, including substituents, 1 to 30 carbon atoms, and
Q⁰ is a direct bond or a divalent group of the formula -O-, -SO₂-, -NH-, -N[(C₁-C₆)alkyl]-, -CO-, -CO-NH- or -O-CO-NH-,
R³ is a hydrogen atom, a hydrocarbon radical or a heterocyclyl radical, where each of the two last mentioned radicals is unsubstituted or substituted and has, including substituents, 1 to 30 carbon atoms, or
R² and R³ together with the nitrogen atom are a heterocycle of 3-6 ring atoms which is saturated or unsaturated, which may, in addition to the nitrogen atom, contain one or two atoms selected from the group consisting of N, O and S and which is unsubstituted or substituted by one or more radicals selected from the group consisting of (C₁-C₆)alkyl, (C₁-C₆)alkoxy, halogen, [(C₁-C₆)alkoxy]carbonyl, (C₁-C₆)haloalkyl and oxo,
R⁴ is H, halogen, NO₂, CN, (C₁-C₄)alkyl, (C₁-C₄)alkoxy, [(C₁-C₄)alkyl]-carbonyl or [(C₁-C₄)alkoxy]carbonyl, where each of the four last mentioned radicals is unsubstituted or substituted in the alkyl moiety by one or more halogen atoms,
R⁵ is H or (C₁-C₄)alkyl,
Q is O or NR*,
R* is H, (C₁-C₄)alkyl, (C₃-C₄)alkenyl or (C₃-C₄)alkynyl, where each of the three last mentioned radicals is unsubstituted or substituted by one or more radicals selected from the group consisting of halogen, (C₁-C₄)alkoxy and (C₁-C₄)alkylthio,
W is an oxygen or sulfur atom,
X,Y independently of one another are H, halogen, (C₁-C₄)alkyl, (C₁-C₄)alkoxy, (C₁-C₄)alkylthio, where each of the three last mentioned radicals is unsubstituted or substituted by one or more radicals selected from the group consisting of halogen, (C₁-C₄)alkoxy and (C₁-C₄)alkylthio, or are mono- or di[(C₁-C₄)alkyl]amino, (C₃-C₄)cycloalkyl, (C₂-C₅)alkenyl, (C₂-C₅)alkynyl, (C₂-C₅)alkenyloxy or (C₂-C₅)alkynyloxy and
Z is CH or N.

2. The compound or a salt thereof as claimed in claim 1, wherein
R¹ is H, (C₁-C₆)alkyl, (C₃-C₆)alkenyl, (C₃-C₆)alkynyl, where each of the three last mentioned radicals is unsubstituted or substituted by one or more radicals selected from the group consisting of halogen, unsubstituted and substituted phenyl, unsubstituted and substituted heterocyclyl having 3 to 6 ring atoms, unsubstituted and substituted (C₃-C₆)cycloalkyl, (C₁-C₄)alkoxy, (C₁-C₄)alkylthio, [(C₁-C₄)alkoxy]-carbonyl and [(C₁-C₄)haloalkoxy]carbonyl, or is unsubstituted or substituted (C₃-C₆)cycloalkyl, unsubstituted or substituted phenyl, unsubstituted or substituted heterocyclyl having 3 to 6 ring atoms and
R² is a group of the formula R⁰-Q⁰-,
in which R⁰ is a hydrogen atom, (C₁-C₁₂)alkyl, (C₃-C₁₂)alkenyl or (C₃-C₁₂)alkynyl, where each of the three last mentioned radicals is unsubstituted or substituted by one or more radicals selected from the group consisting of halogen, (C₁-C₆)alkoxy, (C₁-C₆)haloalkoxy, (C₁-C₆)alkylthio, (C₁-C₆)haloalkylthio, (C₁-C₆)alkylsulfinyl, (C₁-C₆)haloalkylsulfinyl, (C₁-C₆)alkylsulfonyl, (C₁-C₆)haloalkylsulfonyl, [(C₁-C₆)alkoxy]carbonyl, [(C₁-C₆)haloalkoxy]carbonyl, CONR⁶R⁷, SO₂NR⁶R⁷, CN, OH, (C₃-C₆)cycloalkyl, NR⁸R⁹, unsubstituted phenyl, substituted phenyl, unsubstituted heterocyclyl and substituted heterocyclyl, or
is unsubstituted or substituted (C₃-C₆)cycloalkyl, unsubstituted or substituted (C₃-C₆)cycloalkenyl, unsubstituted or substituted heterocyclyl or unsubstituted or substituted phenyl and in which Q⁰ is a direct bond or a divalent group of the formula -O-, -SO₂-, -NH-, -N[(C₁₋C₆)alkyl]-, -CO-, -CO-NH- or -O-CO-NH-,
R³ independently of one another are defined as R⁰ in the radical R², or
R² and R³ together with the nitrogen atom are a heterocycle of 3-6 ring atoms which is saturated or unsaturated, which may, in addition to the nitrogen atom, contain one or two atoms selected from the group consisting of N, O and S and which is unsubstituted or substituted by one or more radicals selected from the group consisting of (C₁-C₆)alkyl, (C₁-C₆)alkoxy, halogen, [(C₁-C₆)alkoxy]carbonyl, (C₁-C₆)haloalkyl and oxo, and
R⁶ and R⁷ independently of one another are H, (C₁-C₆)alkyl, (C₃-C₆)alkenyl, (C₃-C₆)alkynyl or unsubstituted or substituted phenyl or
R⁶ and R⁷ together with the nitrogen atom are a heterocyclic ring having 5 or 6 ring members which may optionally contain further heteroatoms selected from the group consisting of N, O and S and which is unsubstituted or mono- or polysubstituted by radicals selected from the group consisting of (C₁-C₄)alkyl and oxo, and
R⁸ and R⁹ independently of one another and independently of R⁶ and R⁷ are as defined under R⁶ and R⁷ or are (C₁-C₄)alkylcarbonyl, (C₁-C₄)haloalkylcarbonyl, (C₁-C₄)-alkoxycarbonyl or (C₁-C₄)alkylsulfonyl,
Q is O or NR*, where R* is as defined above,
X and Y independently of one another are H, halogen, (C₁-C₄)alkyl, (C₁-C₄)alkoxy, (C₁-C₄)alkylthio, where each of the three last mentioned radicals is unsubstituted or substituted by one or more radicals selected from the group consisting of halogen, (C₁-C₃)alkoxy and (C₁-C₄)alkylthio, are mono- or di[(C₁-C₄)alkyl]amino, (C₃-C₆)cycloalkyl, (C₃-C₅)alkenyl, (C₃-C₅)alkenyloxy or (C₃-C₅)alkynyloxy and
Z is CH or N, where
substituted phenyl, substituted heterocyclyl, substituted cycloalkyl or substituted_ cycloalkenyl carries one or more radicals selected from the group consisting of halogen, (C₁-C₄)alkyl, (C₁-C₄)haloalkyl, (C₁-C₄)alkoxy-(C₁-C₄)alkyl, di-[(C₁-C₄)alkoxy]-(C₁-C₄)alkyl, (C₁-C₄)haloalkoxy, (C₁-C₄)alkylthio, (C₁-C₄)alkylsulfinyl, (C₁-C₄)haloalkylsulfinyl, (C₁-C₄)alkylsulfonyl, (C₁-C₄)haloalkylsulfonyl, NR⁸R⁹, [(C₁-C₄)alkoxy]carbonyl, [(C₁-C₄)haloalkoxy]carbonyl, [(C₁-C₄)alkyl]-carbonyl, OH, phenyl, CN and NO₂ as substituents and
where each of the radicals R¹, R² and R³ has, including substituents, 1 to 20 carbon atoms.

3. The compound or a salt thereof as claimed in claim 1 or 2, wherein
R¹ is H, (C₁-C₆)alkyl, (C₃-C₆)alkenyl or (C₃-C₆)alkynyl, where each of the three last mentioned radicals is unsubstituted or substituted by one or more radicals selected from the group consisting of halogen, phenyl, (C₁-C₄)alkoxy, (C₁-C₄)alkylthio and [(C₁-C₄)alkoxy]carbonyl or is (C₃-C₆)cycloalkyl, (C₃-C₆)cycloalkyl(C₁-C₃)alkyl, heterocyclyl having 3 to 6 ring atoms or heterocyclyl-(C₁-C₃)alkyl having 3 to 6 ring atoms, where each of the four last mentioned radicals is unsubstituted or substituted by one or more radicals selected from the group consisting of halogen, (C₁-C₄)alkyl and (C₁-C₄)alkoxy,
R² is a group of the formula R⁰-Q⁰-, in which R⁰ is a hydrogen atom, (C₁-C₈)alkyl, (C₃-C₈)alkenyl or (C₃-C₈)alkynyl, where each of the three last mentioned radicals is unsubstituted or substituted by one or more radicals selected from the group consisting of halogen, (C₁-C₄)alkoxy, (C₁-C₄)haloalkoxy, (C₁-C₄)alkylthio, (C₁-C₄)haloalkylthio, (C₁-C₄)alkylsulfinyl, (C₁-C₄)haloalkylsulfinyl, (C₁-C₄)alkylsulfonyl, (C₁-C₄)haloalkylsulfonyl, [(C₁-C₆)alkoxy]carbonyl, CONR⁶R⁷, SO₂NR⁶R⁷, CN, OH, (C₃-C₆)cycloalkyl, NR⁸R⁹, phenyl, which is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy, (C₁-C₄)alkylthio, (C₁-C₄)alkylsulfinyl, (C₁-C₄)alkylsufonyl, NR⁸R⁹, [(C₁-C₄)alkoxy]carbonyl, [(C₁-C₄)alkyl]carbonyl, phenyl, [(C₁-C₄)alkyl]carbonyl, CN and NO₂ and heterocyclyl which is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy, (C₁-C₄)alkylthio, (C₁-C₄)alkylsulfinyl, (C₁-C₄)alkylsulfonyl, NR⁸R⁹, [(C₁-C₄)alkoxy]carbonyl, [(C₁-C₄)alkyl]carbonyl, phenyl, [(C₁-C₄)alkyl]carbonyl, CN and NO₂, or
is (C₃-C₆)cycloalkyl, which is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy, [(C₁-C₄)alkoxy]carbonyl, CN, OH and phenyl, or is
(C₃-C₆)cycloalkenyl, which is unsubstituted or substituted by one or more radicals selected from the group consisting of halogen, (C₁-C₄)alkyl, (C₁-C₄)haloalkyl, (C₁-C₄)alkoxy and [(C₁-C₄)alkoxy]carbonyl, or is heterocyclyl or phenyl, where each of the two last mentioned radicals is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy, (C₁-C₄)alkylthio, (C₁-C₄)alkylsulfinyl, (C₁-C₄)alkylsufonyl, NR⁸R⁹, [(C₁-C₄)alkoxy]carbonyl, [(C₁-C₄)alkyl]carbonyl, phenyl, [(C₁-C₄)alkyl]carbonyl, CN and NO₂, and
Q⁰ is a direct bond or a divalent group of the formula -O-, -SO₂-, -NH-, -CO-NH- or -O-CO-NH-,
R³ independently of one another is defined as R⁰ in the radical R²,
R² and R³ together with the nitrogen atom are a heterocycle of 3-6 ring atoms which is saturated or unsaturated and which may, in addition to the nitrogen atom, contain one or two heteroatoms selected from the group consisting of N, O and S and which is unsubstituted or substituted by one or more radicals selected from the group consisting of (C₁-C₃)alkyl, (C₁-C₃)alkoxy, halogen, [(C₁-C₃)- alkoxy]carbonyl, (C₁-C₃)haloalkyl and oxo,
R⁶ and R⁷ independently of one another represent H, (C₁-C₄)alkyl, (C₃-C₄)alkenyl, (C₃-C₄)alkynyl or phenyl, which is unsubstituted or substituted by one or more radicals selected from the group consisting of halogen, (C₁-C₄)alkyl, (C₁-C₄)haloalkyl, (C₁-C₄)alkoxy, (C₁-C₄)alkylthio, (C₁-C₄)alkylsulfonyl, [(C₁-C₄)alkoxy]carbonyl, CN and NO₂, or
R⁶ and R⁷ together with the nitrogen atom are a heterocyclic ring having 5 or 6 ring members which may optionally contain other heteroatoms selected from the group consisting of N, O and S and
which is unsubstituted or mono- or polysubstituted by radicals selected from the group consisting of (C₁-C₄)alkyl and oxo,
R⁸ and R⁹ independently of one another and independently of R⁶ and R⁷ are as defined under R⁶ and R⁷ or are (C₁-C₄)alkylcarbonyl, (C₁-C₄)haloalkylcarbonyl, (C₁-C₄)-alkoxycarbonyl or (C₁-C₄)alkylsulfonyl,
Q is O or NR*, where R* is as defined further above,
X and Y independently of one another are H, halogen, (C₁-C₄)alkyl, (C₁-C₄)alkoxy, (C₁-C₄)alkylthio, where each of the three last mentioned radicals is unsubstituted or substituted by one or more radicals selected from the group consisting of halogen, (C₁-C₃)alkoxy and (C₁-C₄)alkylthio, are mono- or di[(C₁-C₄)alkyl]amino, (C₃-C₆)-cycloalkyl, (C₃-C₅)alkenyl, (C₃-C₅)alkenyloxy or (C₃-C₅)alkynyloxy and
Z is CH or N.

4. The compound or a salt thereof as claimed in any of claims 1 to 3, wherein
R¹ is (C₁-C₆)alkyl, which is unsubstituted or substituted by one or more radicals selected from the group consisting of halogen and (C₁-C₄)alkoxy, or is 3-oxetanyl, (C₃-C₄)alkenyl or (C₃-C₄)alkynyl,
R² is H, (C₁-C₆)alkyl, (C₃-C₆)alkenyl, (C₃-C₆)alkynyl, where each of the three last mentioned radicals is unsubstituted or substituted by one or more radicals selected from the group consisting of halogen, (C₁-C₄)alkoxy, (C₁-C₄)alkylthio, (C₁-C₄)alkylsulfonyl, [(C₁-C₄)alkoxy]carbonyl, (C₃-C₆)cycloalkyl, CN and OH, or is (C₃-C₆)cycloalkyl, which is unsubstituted or substituted by one or more radicals selected from the group consisting of halogen, (C₁-C₄)alkyl, (C₁-C₄)alkoxy, [(C₁-C₄)alkoxy]carbonyl, CN and OH, or is (C₃-C₆)cycloalkenyl, (C₁-C₄)alkoxy, (C₁-C₄)alkenyloxy, (C₁-C₄)alkylsulfonyl, (C₁-C₄)alkylamino or di[(C₁-C₄)alkyl]amino and
R³ is H, (C₁-C₆)alkyl, (C₃-C₆)alkenyl, (C₃-C₆)alkynyl, where each of the three last mentioned radicals is unsubstituted or substituted by one or more radicals selected from the group consisting of halogen, (C₁-C₄)alkoxy, (C₁-C₄)alkylthio, (C₁-C₄)alkylsulfonyl, [(C₁-C₄)alkoxy]carbonyl, (C₃-C₆)cycloalkyl, CN and OH, or is (C₃-C₆)cycloalkyl, which is unsubstituted or substituted by one or more radicals selected from the group consisting of halogen, (C₁-C₄)alkyl, (C₁-C₄)alkoxy, [(C₁-C₄)alkoxy]carbonyl, CN and OH, or is (C₃-C₆)cycloalkenyl or
R² and R³ together with the nitrogen atom are a heterocycle of 3-6 ring atoms which is saturated or unsaturated, which may, in addition to the nitrogen atom, contain one or two atoms selected from the group consisting of N, O and S and which is unsubstituted or substituted by one or more radicals selected from the group consisting of halogen, (C₁-C₃)alkyl, (C₁-C₃)alkoxy, oxo and [(C₁-C₃)alkoxy]carbonyl, and
R⁴ is H, (C₁-C₄)alkyl, (C₁-C₄)haloalkyl, (C₁-C₄)alkoxy or halogen,
R⁵ is H or methyl,
R* is H or (C₁-C₄)alkyl,
X and Y independently of one another are (C₁-C₄)alkyl, (C₁-C₄)alkoxy, where each of the two last mentioned radicals is unsubstituted or substituted by one or more halogen atoms, or are (C₁-C₄)alkylthio, halogen or mono- or di[(C₁-C₂)alkyl]amino and
W is an oxygen atom.

5. The compound or a salt thereof as claimed in any of claims 1 to 3, wherein
R¹ is (C₁-C₃)alkyl, allyl or propargyl,
R² and R³ independently of one another are H, (C₁-C₄)alkyl, (C₁-C₃)alkenyl, (C₁-C₃)alkynyl, (C₁-C₃)cycloalkyl or (C₃-C₆)cycloalkenyl,
R⁴ is H, (C₁-C₃)alkyl or halogen,
R* is (C₁-C₃)alkyl,
X is (C₁-C₂)alkyl, (C₁-C₂)alkoxy, (C₁-C₂)alkylthio, (C₁-C₂)haloalkyl or (C₁-C₂)haloalkoxy and
Y is (C₁-C₂)alkyl, (C₁-C₂)alkoxy, halogen, NHCH₃ or N(CH₃)₂.

6. A process for preparing compounds of the formula (I) or salts thereof as defined in any of claims 1 to 5, which comprises
a) reacting a compound of the formula (II) 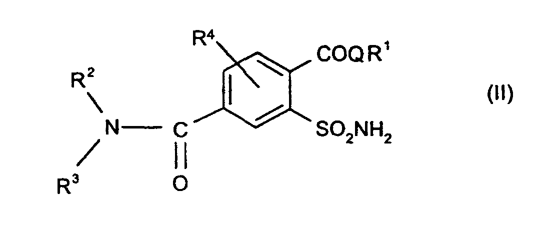
with a heterocyclic carbamate of the formula (III), 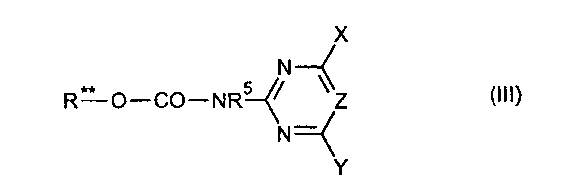 in which R** is optionally substituted aryl or an aliphatic radical, or
b) reacting a sulfonylcarbamate of the formula (IV) 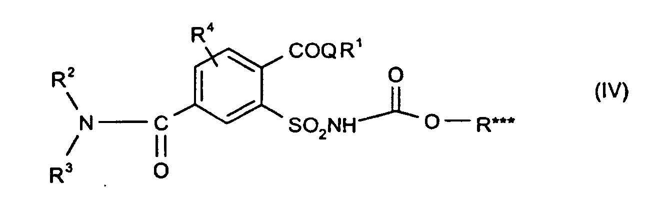 in which R*** is optionally substituted phenyl or (C₁-C₄)alkyl with an amino heterocycle of the formula (V) 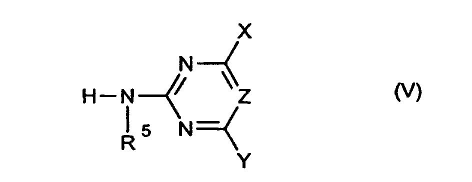 or
c) reacting a sulfonyl isocyanate of the formula (VI) 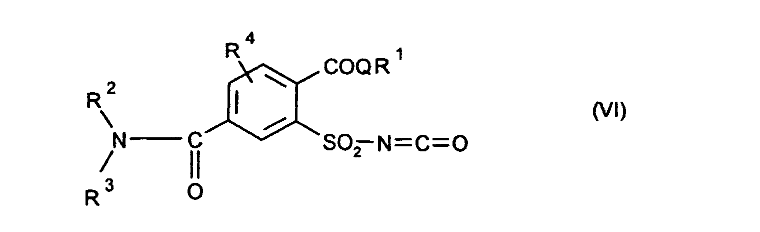 with an amino heterocycle of the formula (V) or
d) reacting a sulfonamide of the formula (II) with a (thio)isocyanate of the formula (VII) 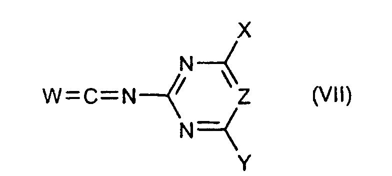 in the presence of a base or
e) reacting an amino heterocycle of the formula (V) initially under base-catalysis with a carbonate, for example diphenyl carbonate, and reacting the intermediate formed in a one-pot reaction with a sulfonamide of the formula (II) (see variant a),
where in the formulae (II)-(VII) the radicals or groups R¹-R⁵, W, X, Y and Z are as defined in formula (I) and in process variants a) to c) and e), initially compounds (I) where W = O are obtained.

7. A herbicidal or plant-growth-regulating composition, which comprises at least one compound of the formula (I) or a salt thereof as claimed in any of claims 1 to 5 and formulation auxiliaries which are customary in crop protection.

8. A method for controlling harmful plants or for regulating the growth of plants, which comprises applying an effective amount of at least one compound of the formula (I) or a salt thereof as claimed in any of claims 1 to 5 onto the harmful plants or plants, their plant seeds or the area on which they grow.

9. The use of a compound of the formula (I) or salts thereof as claimed in any of claims 1 to 5 as herbicides or plant growth regulators.

10. The use as claimed in claim 9, wherein a compound of the formula (I) or salts thereof are employed for controlling harmful plants or for regulating the growth in crops of useful plants or ornamentals.

11. The use as claimed in claim 10, wherein the crop plants are transgenic crop plants.

12. A compound of the formula (II)* 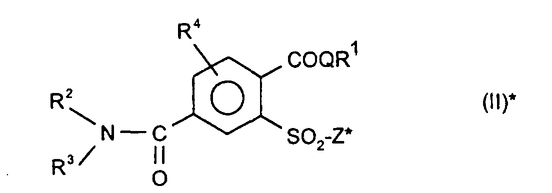 in which Z* = NH₂, NHCOOR***, NCO, NH-tert-butyl or Cl and R¹-R⁴, R*** and Q are as defined in formula (I) or formula (IV) as claimed in claim 6.

13. A compound of the formula (IX)' 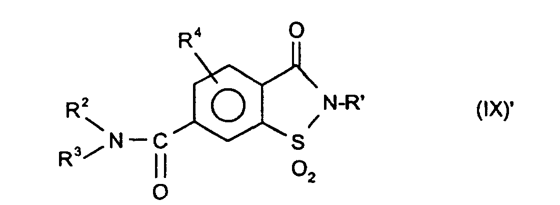 in which R² to R⁴ are as defined in formula (I) as claimed in claim 1 and R' is an alkyl group.

## Revendications

1. Composés de formule (I) ou leurs sels 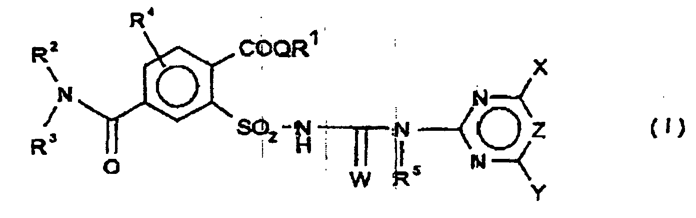 où
R¹ représente un atome d'hydrogène, un reste hydrocarboné ou un reste hétérocyclyle, chacun des deux derniers restes cités étant non substitué ou substitué et présente, y compris les substituants, 1 à 30 atomes de carbone,
R² représente un groupe de formule R⁰-Q⁰-, où
R⁰ représente un atome d'hydrogène, un reste hydrocarboné ou un reste hétérocyclyle, chacun des deux derniers restes cités est non substitué ou substitué et présente, y compris les substituants, 1 à 30 atomes de carbone, et
Q⁰ représente une liaison directe ou un groupe bivalent de formule -O-, -SO₂-, -NH-, -N-(alkyle en C₁-C₆)-, -CO-, -CO-NH-ou -O-CO-NH-,
R³ représente un atome d'hydrogène, un reste hydrocarboné ou un reste hétérocyclyle, chacun des deux derniers restes cités est non substitué ou substitué et présente, y compris les substituants, 1 à 30 atomes de carbone, ou
R² et R³ conjointement avec l'atome d'azote forment un hétérocycle à 3 à 6 chaînons, qui est saturé ou insaturé, peut contenir en plus de l'atome d'azote 1 ou 2 atomes pris dans le groupe comprenant N, O et S et est non substitué ou substitué par un ou plusieurs restes pris dans le groupe comprenant alkyle en C₁-C₆, alkoxy en C₁-C₆, halogène, (alkoxy en C₁-C₆) carbonyle, haloalkyle en C₁-C₆ et oxo,
R⁴ représente un atome d'hydrogène, un atome d'halogène, un groupe NO₂, CN, alkyle en C₁-C₄, alkoxy en C₁-C₄, (alkyl en C₁-C₄)carbonyle ou (alkoxy en C₁-C₄)carbonyle, chacun des quatre derniers restes cités est non substitué ou substitué sur le fragment alkyle par un ou plusieurs atomes d'halogène,
R⁵ représente un atome d'hydrogène ou un groupe alkyle en C₁-C₄,
Q représente un atome O ou un groupe NR*,
R* représente un atome d'hydrogène, un groupe alkyle en C₁-C₄, alcényle en C₃-C₄ ou alcynyle en C₃-C₄, chacun des derniers trois restes cités étant non substitué ou substitué par un ou plusieurs restes pris dans le groupe comprenant halogène, alkoxy en C₁-C₄ et (alkyl en C₁-C₄)thio,
W représente un atome d'oxygène ou de soufre,
X, Y indépendamment l'un de l'autre représentent un atome d'hydrogène, un atome d'halogène, un groupe alkyle en C₁-C₄, alkoxy en C₁-C₄, (alkyl en C₁-C₄)thio, chacun des trois derniers restes cités est non substitué ou substitué par un ou plusieurs restes pris dans le groupe comprenant halogène, alkoxy en C₁-C₄ et (alkyl en C₁-C₄)-thio, ou représente mono- ou di(alkyl en C₁-C₄)amino, cycloalkyle en C₃-C₄, alcényle en C₂-C₅, alcynyle en C₂-C₅, (alcényl en C₂-C₅)oxy ou (alcynyl en C₂-C₅)oxy et
Z représente un groupe CH ou un atome de N.

2. Composés ou leurs sels selon la revendication 1, **caractérisés en ce que**
R¹ représente un atome d'hydrogène, un reste alkyle en C₁-C₆, alcényle en C₃-C₆, alcynyle en C₃-C₆, chacun des trois derniers restes cités étant non substitué ou substitué par un ou plusieurs restes pris dans le groupe comprenant halogène, phényle non substitué et substitué, hétérocyclyle non substitué et substitué à 3 à 6 chaînons, cycloalkyle en C₃-C₆ non substitué et substitué, alkoxy en C₁-C₄, (alkyl en C₁-C₄)thio, (alkoxy en C₁-C₄)carbonyle et halo(alkoxy en C₁-C₄)carbonyle, ou représente cycloalkyle en C₃-C₆ non substitué ou substitué, phényle non substitué ou substitué, hétérocyclyle non substitué ou substitué à 3 à 6 chaînons, et
R² représente un groupe de formule R⁰-Q⁰-, où
R⁰ représente un atome d'hydrogène, un groupe alkyle en C₁-C₁₂, alcényle en C₃-C₁₂ ou alcynyle en C₃-C₁₂, chacun des trois derniers restes cités étant non substitué ou substitué par un ou plusieurs restes pris dans le groupe comprenant halogène, alkoxy en C₁-C₆, haloalkoxy en C₁-C₆, (alkyl en C₁-C₆)thio, halo(alkyl en C₁-C₆)thio, (alkyl en C₁-C₆)-sulfinyle, halo(alkyl en C₁-C₆)-sulfinyle, (alkyl en C₁-C₆)sulfonyle, halo (alkyl en C₁-C₆)-sulfonyle, (alkoxy en C₁-C₆)-carbonyle, halo(alkoxy en C₁-C₆)carbonyle, CONR⁶R⁷, SO₂NR⁶R⁷, CN, OH, cycloalkyle en C₃-C₆, NR⁸R⁹, phényle non substitué, phényle substitué, hétérocyclyle non substitué et hétérocyclyle substitué, ou cycloalkyle en C₃-C₆ non substitué ou substitué, cycloalcényle en C₃-C₆ non substitué ou substitué, hétérocyclyle non substitué ou substitué ou phényle non substitué ou substitué, et où
Q⁰ représente une liaison directe ou un groupe bivalent de formule -O-, -SO₂-, -NH-, -N-(alkyle en C₁-C₆)-, -CO-, -CO-NH-ou -O-CO-NH-,
R³ indépendamment l'un de l'autre est défini comme R⁰ dans le reste R², ou
R² et R³ conjointement avec l'atome d'azote forment un hétérocycle à 3 à 6 chaînons, qui est saturé
ou insaturé, pouvant contenir, en plus de l'atome d'azote, 1 ou 2 atomes pris dans le groupe comprenant N, O et S et est non substitué ou substitué par un ou plusieurs restes pris dans le groupe comprenant alkyle en C₁-C₆, alkoxy en C₁-C₆, halogène, (alkoxy en C₁-C₆)carbonyle, haloalkyle en C₁-C₆ et oxo, et
R⁶ et R⁷ indépendamment l'un de l'autre représentent un atome d'hydrogène, un groupe alkyle en C₁-C₆, alcényle en C₃-C₆, alcynyle en C₃-C₆ ou phényle non substitué ou substitué, ou
R⁶ et R⁷ conjointement avec l'atome d'azote forment un hétérocycle à 5 ou 6 chaînons, qui peut éventuellement renfermer d'autres hétéroatomes pris dans le groupe comprenant N, O et S, et est non substitué ou substitué une ou plusieurs fois par des restes pris dans le groupe comprenant alkyle en C₁-C₄ et oxo, et
R⁸ et R⁹ indépendamment l'un de l'autre et indépendamment de R⁶ et R⁷ sont définis comme sous R⁶ et R⁷ ou représentent un groupe (alkyle en C₁-C₄)carbonyle, halo (alkyl en C₁-C₄)-carbonyle, (alkoxy en C₁-C₄)carbonyle ou (alkyl en C₁-C₄)sulfonyle,
Q représente un atome de O ou un groupe NR*, R* est défini comme ci-dessus,
X et Y indépendamment l'un de l'autre représentent un atome d'hydrogène, un atome d'halogène, un groupe alkyle en C₁-C₄, alkoxy en C₁-C₄, (alkyl en C₁-C₄)thio, chacun des trois derniers restes cités est non substitué ou substitué par un ou plusieurs restes pris dans le groupe comprenant halogène, alkoxy en C₁-C₃ et (alkyl en C₁-C₄)-thio, mono- ou di (alkyl en C₁-C₄)amino, cycloalkyle en C₃-C₆, alcényle en C₃-C₅, (alcényl en C₃-C₅)oxy ou (alcynyl en C₂-C₅)oxy et
Z représente un groupe CH ou un atome de N,
phényle substitué, hétérocyclyle substitué, cycloalkyle substitué ou cycloalcényle substitué portant en tant que substituants un ou plusieurs restes pris dans le groupe comprenant halogène, alkyle en C₁-C₄, haloalkyle en C₁-C₄, (alkoxy en C₁-C₄)-(alkyle en C₁-C₄), di-[(alkoxy en C₁-C₄)]-(alkyle en C₁-C₄), haloalkoxy en C₁-C₄, (alkyl en C₁-C₄)thio, (alkyl en C₁-C₄)-sulfinyle, halo (alkyl en C₁-C₄)-sulfinyle, (alkyl en C₁-C₄)sulfonyle, halo (alkyl en C₁-C₄)-sulfonyle, NR⁸R⁹, (alkoxy en C₁-C₄)-carbonyle, halo(alkoxy en C₁-C₄)carbonyle, (alkyl en C₁-C₄)-carbonyle, OH, phényle, CN et NO₂, et
chacun des restes R¹, R² et R³ présente, y compris les substituants, 1 à 20 atomes de carbone.

3. Composés ou leurs sels selon la revendication 1 ou 2, **caractérisés en ce que**
R¹ représente un atome d'hydrogène, un reste alkyle en C₁-C₆, alcényle en C₃-C₆ ou alcynyle en C₃-C₆, chacun des trois derniers restes cités étant non substitué ou substitué par un ou plusieurs restes pris dans le groupe comprenant halogène, phényle, alkoxy en C₁-C₄, (alkyl en C₁-C₄)thio et (alkoxy en C₁-C₄)carbonyle, ou représente cycloalkyle en C₃-C₆, (cycloalkyle en C₃-C₆)-alkyle en C₁-C₃, hétérocyclyle à 3 à 6 chaînons ou hétérocyclyl-alkyle en C₁-C₃ à 3 à 6 chaînons, chacun des quatre derniers restes cités est non substitué ou substitué par un ou plusieurs restes pris dans le groupe comprenant halogène, alkyle en C₁-C₄ et alkoxy en C₁-C₄,
R² représente un groupe de formule R⁰-Q⁰-, où
R⁰ représente un atome d'hydrogène, un groupe alkyle en C₁-C₈, alcényle en C₃-C₈ ou alcynyle en C₃-C₈, chacun des trois derniers restes cités est non substitué ou substitué par un ou plusieurs restes pris dans le groupe comprenant halogène, alkoxy en C₁-C₄, haloalkoxy en C₁-C₄, (alkyl en C₁-C₄)thio, halo (alkyl en C₁-C₄)thio, (alkyl en C₁-C₄)-sulfinyle, halo (alkyl en C₁-C₄) -sulfinyle, (alkyl en C₁-C₄)-sulfonyle, halo (alkyl en C₁-C₄)sulfonyle, (alkoxy en C₁-C₆)-carbonyle, CONR⁶R⁷, SO₂NR⁶R⁷, CN, OH, cycloalkyle en C₃-C₆, NR⁸R⁹, phényle qui est non substitué ou substitué par un ou plusieurs restes pris dans le groupe comprenant halogène, alkyle en C₁-C₄, haloalkyle en C₁-C₄, alkoxy en C₁-C₄, haloalkoxy en C₁-C₄, (alkyl en C₁-C₄)thio, (alkyl en C₁-C₄)sulfinyle, (alkyl en C₁-C₄)sulfonyle, NR⁸R⁹, (alkoxy en C₁-C₄)carbonyle, (alkyl en C₁-C₄)-carbonyle, phényle, (alkyl en C₁-C₄)-carbonyle, CN et NO₂, et hétérocyclyle qui est non substitué ou substitué par un ou plusieurs restes pris dans le groupe comprenant halogène, alkyle en C₁-C₄, haloalkyle en C₁-C₄, alkoxy en C₁-C₄, haloalkoxy en C₁-C₄, (alkyl en C₁-C₄)thio, (alkyl en C₁-C₄)-sulfinyle, (alkyl en C₁-C₄)sulfonyle, NR⁸R⁹, (alkoxy en C₁-C₄)-carbonyle, (alkyl en C₁-C₄)carbonyle, phényle, (alkyl en C₁-C₄)carbonyle, CN et NO₂, ou cycloalkyle en C₃-C₆ qui est non substitué ou substitué par un ou plusieurs restes pris dans le groupe comprenant halogène, alkyle en C₁-C₄, haloalkyle en C₁-C₄, alkoxy en C₁-C₄, haloalkoxy en C₁-C₄, (alkoxy en C₁-C₄)carbonyle, CN, OH et phényle, ou
cycloalcényle en C₃-C₆ qui est non substitué ou substitué par un ou plusieurs restes pris dans le groupe comprenant halogène, alkyle en C₁-C₄, haloalkyle en C₁-C₄, alkoxy en C₁-C₄ et (alkoxy en C₁-C₄)carbonyle, ou
hétérocyclyle ou phényle, chacun des deux derniers restes cités est non substitué ou substitué par un ou plusieurs restes pris dans le groupe comprenant halogène, alkyle en C₁-C₄, haloalkyle en C₁-C₄, alkoxy en C₁-C₄, haloalkoxy en C₁-C₄, (alkyl en C₁-C₄)thio, (alkyl en C₁-C₄)sulfinyle, (alkyl en C₁-C₄)sulfonyle, NR⁸R⁹, (alkoxy en C₁-C₄)carbonyle, (alkyl en C₁-C₄)-carbonyle, phényle, (alkyl en C₁-C₄)-carbonyle, CN et NO₂, ou
Q⁰ représente une liaison directe ou un groupe bivalent de formule -O-, -SO₂-, -NH-, -CO-NH- ou -O-CO-NH-,
R³ indépendamment l'un de l'autre est défini comme R⁰ dans le reste R²,
R² et R³ conjointement avec l'atome d'azote forment un hétérocycle à 3 à 6 chaînons, qui est saturé ou insaturé, pouvant contenir, en plus de l'atome d'azote, 1 ou 2 hétéroatomes pris dans le groupe comprenant N, O et S et est non substitué ou substitué par un ou plusieurs restes pris dans le groupe comprenant alkyle en C₁-C₃, alkoxy en C₁-C₃, halogène, (alkoxy en C₁-C₃)-carbonyle, haloalkyle en C₁-C₃ et oxo, et
R⁶ et R⁷ indépendamment l'un de l'autre représentent un atome d'hydrogène, un groupe alkyle en C₁-C₄, alcényle en C₃-C₄, alcynyle en C₃-C₄ ou phényle non substitué ou substitué par un ou plusieurs restes pris dans le groupe comprenant halogène, alkyle en C₁-C₄, haloalkyle en C₁-C₄, alkoxy en C₁-C₄, (alkyl en C₁-C₄)thio, (alkyl en C₁-C₄)-sulfonyle, (alkoxy en C₁-C₄)carbonyle, CN et NO₂, ou
R⁶ et R⁷ conjointement avec l'atome d'azote forment un hétérocycle à 5 ou à 6 chaînons, qui peut éventuellement renfermer d'autres hétéroatomes pris dans le groupe comprenant N, O et S et est non substitué ou substitué une ou plusieurs fois par des restes pris dans le groupe comprenant alkyle en C₁-C₄ et oxo, et
R⁸ et R⁹ indépendamment l'un de l'autre et indépendamment de R⁶ et R⁷ sont définis comme sous R⁶ et R⁷ ou représentent un groupe (alkyle en C₁-C₄)carbonyle, halo (alkyl en C₁-C₄)-carbonyle, (alkoxy en C₁-C₄)carbonyle ou (alkyl en C₁-C₄)sulfonyle,
Q représente un atome de O ou un groupe NR*, R* est défini comme ci-dessus,
X et Y indépendamment l'un de l'autre représentent un atome d'hydrogène, un atome d'halogène, un groupe alkyle en C₁-C₄, alkoxy en C₁-C₄, (alkyl en C₁-C₄)thio, chacun des trois derniers restes cités est non substitué ou substitué par un ou plusieurs restes pris dans le groupe comprenant halogène, alkoxy en C₁-C₃ et (alkyl en C₁-C₄)-thio, représente mono- ou di(alkyl en C₁-C₄)-amino, cycloalkyle en C₃-C₆, alcényle en C₃-C₅, (alcényl en C₃-C₅)oxy ou (alcynyl en C₂-C₅)oxy et
Z représente un groupe CH ou un atome de N.

4. Composés ou leurs sels selon l'une des revendications 1 à 3, **caractérisés en ce que**
R¹ représente un reste alkyle en C₁-C₆ qui est non substitué ou substitué par un ou plusieurs restes pris dans le groupe comprenant halogène et alkoxy en C₁-C₄, ou 3-oxétanyle, alcényle C₃-C₄ ou alcynyle en C₃-C₄,
R² représente un atome d'hydrogène, un groupe alkyle en C₁-C₆, alcényle en C₃-C₆, alcynyle en C₃-C₆, chacun des trois derniers restes cités est non substitué ou substitué par un ou plusieurs restes pris dans le groupe comprenant halogène, alkoxy en C₁-C₄, (alkyl en C₁-C₄)thio, (alkyl en C₁-C₄)-sulfonyle, (alkoxy en C₁-C₄)-carbonyle, cycloalkyle en C₃-C₆, CN et OH, ou représente cycloalkyle en C₃-C₆, qui est non substitué ou substitué par un ou plusieurs restes pris dans le groupe comprenant halogène, alkyle en C₁-C₄, alkoxy en C₁-C₄, (alkoxy en C₁-C₄)carbonyle, CN ou OH, ou cycloalcényle en C₃-C₆, alkoxy en C₁-C₄, (alcényl en C₁-C₄)oxy, (alkyl en C₁-C₄)sulfonyle, (alkyl en C₁-C₄)amino ou di(alkyl en C₁-C₄)amino et
R³ représente un atome d'hydrogène, un groupe alkyle en C₁-C₆, alcényle en C₃-C₆, alcynyle en C₃-C₆, chacun des trois derniers restes cités est non substitué ou substitué par un ou plusieurs restes pris dans le groupe comprenant halogène, alkoxy en C₁-C₄, (alkyl en C₁-C₄)thio, (alkyl en C₁-C₄)-sulfonyle, (alkoxy en C₁-C₄)-carbonyle, cycloalkyle en C₃-C₆, CN et OH, ou représente cycloalkyle en C₃-C₆, qui est non substitué ou substitué par un ou plusieurs restes pris dans le groupe comprenant halogène, alkyle en C₁-C₄, alkoxy en C₁-C₄, (alkoxy en C₁-C₄)-carbonyle, CN et OH, ou cycloalcényle en C₃-C₆ ou
R² et R³ conjointement avec l'atome d'azote forment un hétérocycle à 3 à 6 chaînons, qui est saturé ou insaturé, pouvant contenir, en plus de l'atome d'azote, 1 ou 2 hétéroatomes pris dans le groupe comprenant N, O et S et est non substitué ou substitué par un ou plusieurs restes pris dans le groupe comprenant halogène, alkyle en C₁-C₃, alkoxy en C₁-C₃, oxo et (alkoxy en C₁-C₃)carbonyle, et
R⁴ représente un atome d'hydrogène, un groupe alkyle en C₁-C₄, haloalkyle en C₁-C₄, alkoxy en C₁-C₄ ou halogène,
R⁵ représente un atome d'hydrogène ou un groupe méthyle,
R* représente un atome d'hydrogène ou un groupe alkyle en C₁-C₄,
X et Y indépendamment l'un de l'autre représentent un groupe alkyle en C₁-C₄, alkoxy en C₁-C₄, chacun des deux derniers restes cités est non substitué ou substitué par un ou plusieurs atomes d'halogène, ou représente (alkyl en C₁-C₄)-thio, halogène ou mono- ou di(alkyl en C₁-C₂)-amino et
W représente un atome d'oxygène.

5. Composés ou leurs sels selon l'une des revendications 1 à 3, **caractérisés en ce que**
R¹ représente un groupe alkyle en C₁-C₃, allyle ou propargyle,
R² et R³ indépendamment l'un de l'autre représentent un atome d'hydrogène, un groupe alkyle en C₁-C₄, alcényle en C₁-C₃, alcynyle en C₁-C₃, cycloalkyle en C₁-C₃ ou cycloalcényle en C₃-C₆,
R⁴ représente un atome d'hydrogène, un groupe alkyle en C₁-C₃ ou halogène,
R* représente un groupe alkyle en C₁-C₃,
X représente un groupe alkyle en C₁-C₂, alkoxy en C₁-C₂, (alkyl en C₁-C₂)thio, haloalkyle en C₁-C₂ ou haloalkoxy en C₁-C₂ et
Y représente un groupe alkyle en C₁-C₂, alkoxy en C₁-C₂, halogène, NHCH₃ ou N(CH₃)₂.

6. Procédé pour la préparation de composés de formule (I) ou leurs sels, tels que définis dans les revendications 1 à 5, **caractérisé en ce qu'**on fait réagir
a) un composé de formule (II) 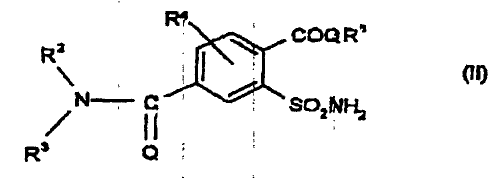 sur un carbamate hétérocyclique de formule (III) 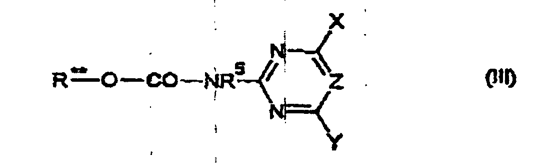 où R** représente un groupe aryle éventuellement substitué ou un reste aliphatique, ou
b) on fait réagir un sulfonylcarbamate de formule (IV) 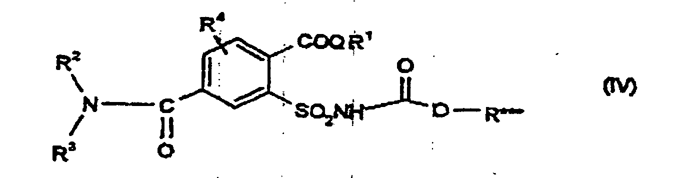 où R*** représente un groupe phényle éventuellement substitué ou un groupe alkyle en C₁-C₄ sur un aminohétérocycle de formule (V) 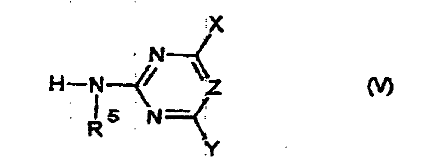 ou
c) on fait réagir un sulfonylisocyanate de formule (VI) 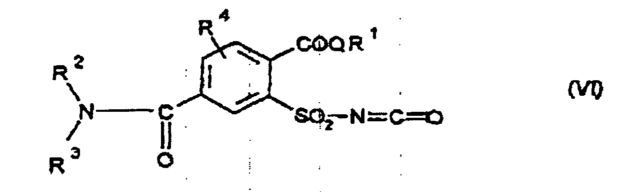 sur un aminohétérocycle de formule (V), ou
d) on fait réagir un sulfonamide de formule (II) sur un (thio)isocyanate de formule (VII) 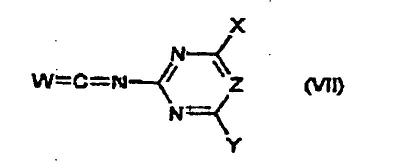 en présence d'une base, ou
e) on fait réagir un aminohétérocycle de formule (V) d'abord sous catalyse par une base sur un ester de l'acide carbonique, par exemple un carbonate de diphényle, et on fait réagir l'intermédiaire dans une réaction en un pot sur un sulfonamide de formule (II) (voir variante a),
dans les formules (II) à (VII), les restes ou les groupes R¹ à R⁵, W, X, Y et Z, sont définis comme à la formule (I) et on obtient dans les variantes de procédé a) à c) et e) d'abord les composés (I) avec W = O.

7. Herbicides ou agents régulateurs de croissance, **caractérisés en ce qu'**il renferment au moins un composés de formule (I) ou son sel selon l'une des revendications 1 à 5, et les adjuvants de formulation usuels dans le domaine phytosanitaire.

8. Procédé pour la lutte contre les plantes adventices ou pour la régulation de la croissance de plantes, **caractérisé en ce qu'**on applique une quantité efficace d'au moins un composé de formule (I) ou son sel selon l'une des revendications 1 à 5, aux plantes adventices ou aux plantes, à leurs semence ou à la surface cultivable.

9. Utilisation des composés de formule (I) ou leurs sels selon l'une des revendications 1 à 5, en tant qu'herbicides ou régulateurs de croissances de plantes.

10. Utilisation selon la revendication 9, **caractérisée en ce qu'**on utilise les composés de formule (I) ou leurs sels pour la lutte contre les plantes adventices ou pour la régulation de la croissance dans des cultures de plantes utiles ou décoratives.

11. Utilisation selon la revendication 10, **caractérisée en ce que** les plantes de culture sont des plantes transgéniques.

12. Composés de formule (II)* 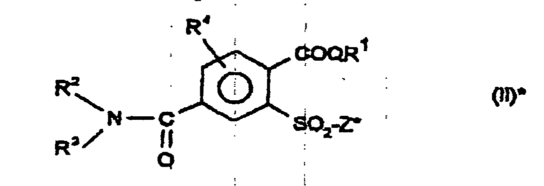 où Z* = NH₂, NHCOOR***, NCO, NH-tert.-butyle ou Cl, et R¹ à R⁴, R*** et Q sont définis comme à la formule (I) ou formule (IV) selon la revendication 6.

13. Composés de formule (IX)' 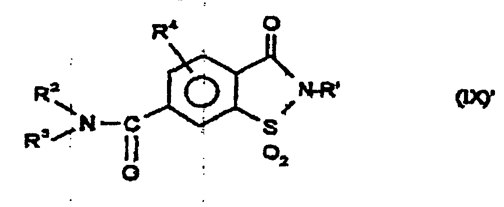 où R² à R⁴ sont définis comme à la formule (I) selon la revendication A, et R' représente un groupe alkyle.
